(19) Europäisches Patentamt
European Patent Office
Office européen des brevets

(11) EP 1 132 472 A1

(12) EUROPEAN PATENT APPLICATION
published in accordance with Art. 158(3) EPC

(43) Date of publication:
12.09.2001 Bulletin 2001/37

(21) Application number: 99972257.2

(22) Date of filing: 11.11.1999

(51) Int Cl.[7]: **C12N 15/12**, C07K 14/47, C07K 16/18, C12N 1/21, A01K 67/027, A61K 31/7088, A61K 35/76, A61K 38/02, A61K 45/00, A61P 25/16, A61P 25/28, A61P 25/08, A61P 25/14, A61P 43/00, A61P 25/00, A61K 48/00, A61K 39/395

(86) International application number:
PCT/JP99/06275

(87) International publication number:
WO 00/29570 (25.05.2000 Gazette 2000/21)

(84) Designated Contracting States:
AT BE CH CY DE DK ES FI FR GB GR IE IT LI LU MC NL PT SE
Designated Extension States:
AL LT LV MK RO SI

(30) Priority: 13.11.1998 JP 32319998
07.12.1998 JP 34692598

(71) Applicant: Takeda Chemical Industries, Ltd.
Osaka 541-0045 (JP)

(72) Inventor: SUGINO, Hiroshi
Tokushima-shi, Tokushima 770-8041 (JP)

(74) Representative:
Helbing, Jörg, Dr. Dipl.-Chem. et al
Patentanwälte
von Kreisler-Selting-Werner,
Postfach 10 22 41
50462 Köln (DE)

# (54) NOVEL PROTEIN AND UTILIZATION THEREOF

(57) The protein of the present invention, a partial peptide thereof or a salt thereof, expressed specifically in the brain, having PDZ domains and/or WW domains and having affinity particularly for activin receptors and/or activin intracellular information transmission molecules, is useful for determining a binding protein having an ability to bind to said protein and for screening a compound inhibiting or promoting a binding of the protein etc. of the present invention to the binding protein.

EP 1 132 472 A1

## Description

Technical Field

The present invention relates to a novel protein having a specific amino acid sequence, having PDZ domains and/or WW domains, and being expressed specifically in the brain, a DNA comprising a DNA region encoding said protein, a process for producing the protein, and use of the protein and the DNA.

Background Art

Heretofore, a large number of physiologically active substances have been isolated and identified, and their functions are being elucidated. Some of them are known to exhibit various activities in a wide variety of organs or cells. The various activities in a wide variety of organs or cells appear generally via receptors to which the physiologically active substances bind, but there are many cases where whether the combination of the physiologically active substances binding to the receptors is identical in all organs and cells or specific to each organ or cell is not elucidated.

Some physiologically active proteins have PDZ domains and WW domains. These domains are protein-binding domain modules found relatively recently. Accordingly, the in vivo distribution, functions and regulatory mechanism of proteins having PDZ domains and WW domains are still not elucidated in many cases.

The proteins having PDZ domains are considered to play an important role in formation of a backing structure of cell membrane, in formation of a network of cytoskeleton, and in formation of an intracellular signal transmission network developed in an intracellular cortex. In the nervous system, these proteins participate in forming complexes of neurotransmitter receptors, ion channels, etc., thus making them essential for assembly of protein clusters in synapse sites. Further, PDZ proteins whose expression is regulated depending on synaptic plasticity were recently found, and there is the possibility that the PDZ proteins participate in altering the morphology of synapses accompanying plasticity through rearrangement of receptors, and they are considered to participate in construction of a nervous network at a development stage and in in vivo high-order mechanism in the brain. The PDZ domain is similar in certain respects to known peptide-binding domains, but has distinctive features.

The PDZ domain is a domain preserved widely in microorganisms, higher plants and animals, and in many cases, the PDZ domain recognizes a C-terminal short amino acid sequence of its target proteins, and these target proteins are often membrane-penetrating receptors or channels. The PDZ domain is often found in a form repeated about 2- to 6-times in the same protein, and unlike other domain modules, some of the PDZ domains form homodimers. These are features important for participating in formation of a protein-crosslinked network and microdomains in intercellular adhesion, such as a cellular surface structure and tight junction of synapses etc.

On the other hand, the WW domain is also a domain consisting of about 40 amino acids preserved well in yeasts and mammals, and recognizes and binds to a relatively short amino acid sequence called PY motif rich in proline. As the protein having the WW domains, there are ubiquitin protein ligase, dystrophin, formine-binding protein, etc. On such protein, 1 to 4 WW domains occur in a form linked in series. A majority of those proteins having WW domains have domains participating in interaction with other proteins and enzymatically active domains such as in proteiphostase [phonetic transcription], ras GAP, etc., and it is suggested that the WW domains have very diverse biological functions.

Activin is a regulatory factor promoting secretion of follicle-stimulating hormone (FSH) from the anterior pituitary. Heretofore, it has been considered that the regulation of secretion of gonadotrophic hormone from the pituitary is based on steroid hormone produced in the gonad, and since activin and its antagonist i.e. inhibin were found, this regulation attracts attention as a new hormone secretion-regulating mechanism in a hypothalamo-hypophysial-germ system. As analysis of the physiological activities of activin proceeds, this system was found to have diverse physiological activities including not only secretory regulation of FSH but also an activity of inducting or inhibiting differentiation of cells in a blood-corpuscle system or in reproductive cells, an activity of maintaining the viability of nerve cells, etc., but many features of this mechanism are not elucidated in detail.

As a means of elucidating the physiological activities of a protein having PDZ domains and/or WW domains expressed particularly in the brain, receptors (e.g., activin receptors) having an ability to bind to said protein, and intracellular information transmission molecules (e.g., Smad) as well as of elucidating detailed molecular mechanisms etc. for neurotrophic factor-like activities and for inhibiting differentiation of cells in nervous-system tissues in an activin-activin receptor system, the object of the present invention is to provide a method for isolating said novel protein, a method for detecting the same, a DNA comprising a gene for said novel protein, a process for producing a protein encoded by the gene for said novel protein, and use of the DNA and the protein.

Disclosure of Invention

To solve the problem described above, the present inventors made extensive study, and as a result, they screened a binding protein from a mouse brain cDNA by means of a yeast two-hybrid method in which an intracellular region in a mouse activin IIA-N receptor protein shown in SEQ ID NO: 1 was used as a bait, thus acquiring a cDNA clone which could be confirmed to bind to activin IIA-N receptors even in COS7 cells. Further, they isolated and analyzed a cDNA clone containing a full-length gene encoded by said clone, and found that said gene is a gene containing a region encoding 5 PDZ domains and 2 WW domains. Said protein encoded by this gene is a protein factor having a plurality of domains for protein-protein interaction, and this protein binds, via PDZ domains out of these domains, to 1) intracellular regions in activin receptors, resulting in inhibiting activin from transmitting information to cells, but not to 2) intracellular regions of receptors for other cytokines, thus exerting no influence on their transmission of information to cells. Further, the present inventors found that this protein binds via other WW domains to activin intracellular transmission molecules thereby inhibiting activin from transmitting information to cells.

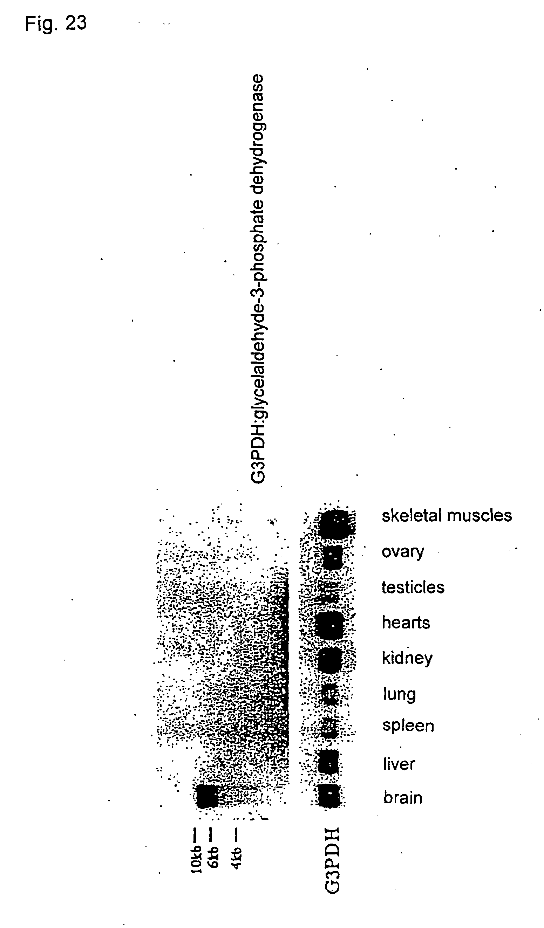

Further, the present inventors extracted poly(A) $^{+}$RNA from a wide variety of mouse organs, and used a DNA shown in SEQ ID NO: 2, 3, or 4 as a probe to examine expression of the novel protein of the present invention by Northern hybridization, and as a result, they found that high-level expression thereof occurs particularly in the brain, as shown in Fig. 23. From this, the activin-activin receptor information transmission system to which said novel protein was added is suggested to govern regulation of multiplication and differentiation of cerebral cells and found to be applicable to diagnosis, treatment, etc. of diseases in the cerebral and nerve system.

As further examination on the basis of these findings, the present inventors arrived at the present invention.

That is, the present invention provides:

1. A protein which has an amino acid sequence identical to or substantially identical to an amino acid sequence represented by SEQ ID NO: 5, or a salt thereof,
2. A protein which has an amino acid sequence identical to or substantially identical to an amino acid sequence represented by SEQ ID NO: 6, or a salt thereof,
3. The protein according to item 1 or 2, which has PDZ domains and WW domains and is expressed specifically in the brain and has an ability to bind to activin receptors and/or activin intracellular information transmission molecules,
4. The protein according to item 3, wherein the activin intracellular information transmission molecule is Smad3,
5. The protein according to item 1 or 2, which has 5 PDZ domains and 2 WW domains and is expressed specifically in the brain and has an ability to bind to activin receptors and Smad3,
6. A partial peptide of the protein according to item 1, a partial peptide of the protein according to item 2, or a salt thereof,
7. A recombinant DNA comprising a DNA which comprises a nucleotide sequence encoding the protein according to item 1 or the protein according to item 2,
8. The DNA according to item 7, which has a nucleotide sequence represented by SEQ ID NO: 7, a nucleotide sequence represented by SEQ ID NO: 8 or a nucleotide sequence hybridizing therewith under high stringent conditions.
9. A recombinant DNA which comprises a DNA having a nucleotide sequence encoding the partial peptide according to item 6.
10. A recombinant vector which comprises the DNA according to item 7,
11. A transformant comprising the recombinant vector according to item 10,
12. A method for producing the protein according to item 1, the protein according to item 2 or a salt thereof, which comprises culturing the transformant according to item 11 to produce and accumulate the protein according to item 1 or the protein according to item 2 is formed and accumulated, and then recovering the product,
13. An antibody against the protein according to item 1, the protein according to item 2, the partial peptide according to item 6 or a salt thereof,
14. A method for quantifying the protein according to item 1, the protein according to item 2, the partial peptide according to item 6 or a salt thereof, which comprises allowing a test solution containing the protein according to item 1, the protein according to item 2, the partial peptide according to item 6 or a salt thereof and the labeled protein according to item 1, the labeled protein according to item 2, the labeled partial peptide according to item 6 or a labeled salt thereof to react competitively with the antibody according to item 13,
15. A method for determining a binding protein to the protein according to item 1, the protein according to item 2, the partial peptide according to item 6 or a salt thereof, which comprises using the protein according to item 1, the protein according to item 2, the partial peptide according to item 6 or a salt thereof,
16. The method according to item 15, which comprises introducing (1) an expression vector which fuses the protein according to item 1, the protein according to item 2 or the partial peptide according to item 6 with a DNA-binding region of a transcriptional factor and (2) a fusion library between a gene encoding a test protein and a transcription-activating region, into a host cell having a reporter gene maintaining a region binding to the transcriptional factor on a promoter, and measuring a change in the amount of the expressed reporter gene which is increased by a binding of the protein according to item 1, the protein according to item 2 or the partial peptide according to item 6 to the test compound,

17. A protein or a salt thereof which binds to the protein according to item 1, the protein according to item 2, the partial peptide according to item 6 or a salt thereof, which is obtained by the method according to item 15,

18. A method for screening a compound or a salt thereof inhibiting or promoting a binding of the protein according to item 1, the protein according to item 2, the partial peptide according to item 6 or a salt thereof to the protein according to item 17 or a salt thereof, activin receptors or activin intracellular information transmission molecules, which comprises using the protein according to item 1, the protein according to item 2, the partial peptide according to item 6 or a salt thereof,

19. A method for screening a compound or a salt thereof inhibiting or promoting a binding of the protein according to item 1, the protein according to item 2, the partial peptide according to item 6 or a salt thereof to activin receptors or activin intracellular information transmission molecules, which comprises comparing the case where the labeled protein according to item 1, the labeled protein according to item 2, the labeled partial peptide according to item 6 or a labeled salt thereof is contacted with activin receptors or activin intracellular information transmission molecules, with the case where the labeled protein according to item 1, the labeled protein according to item 2, the labeled partial peptide according to item 6 or a labeled salt thereof and a test compound are contacted with activin receptors or activin intracellular information transmission molecules, by measuring the amount of the labeled protein according to item 1, the labeled protein according to item 2, the labeled partial peptide according to item 6 or a labeled salt thereof bound to the activin receptors or activin intracellular information transmission molecules in both the cases,

20. A method for screening a compound or a salt thereof inhibiting or promoting a binding of the protein according to item 1, the protein according to item 2, the partial peptide according to item 6 or a salt thereof to the protein according to item 17 or a salt thereof, which comprises comparing the case where the labeled protein according to item 1, the labeled protein according to item 2, the labeled partial peptide according to item 6 or a labeled salt thereof is contacted with the protein according to item 17 or a salt thereof, with the case where the labeled protein according to item 1, the labeled protein according to item 2, [the labeled partial peptide according to item 6] or a labeled salt thereof and a test compound are contacted with the protein according to item 17 or a salt thereof, by measuring the amount of the labeled protein according to item 1, the labeled protein according to item 2, the labeled partial peptide according to item 6 or a labeled salt thereof bound to the protein according to item 17 or a salt thereof in both the cases,

21. A method for screening a compound or a salt thereof inhibiting or promoting a binding of the protein according to item 1, the protein according to item 2, the partial peptide according to item 6 or a salt thereof to the protein according to item 17 or a salt thereof, activin receptors or activin intracellular information transmission molecules, which comprises comparing the case where the protein according to item 1, the protein according to item 2, the partial peptide according to item 6 or a salt thereof is introduced into cells expressing the protein according to item 17 or a salt thereof, activin receptors or activin intracellular information transmission molecules, with the case where the protein according to item 1, the protein according to item 2, the partial peptide according to item 6 or a salt thereof and a test compound are introduced into cells expressing the protein according to item 17 or a salt thereof, activin receptors or activin intracellular information transmission molecules, by measuring the amount of the protein according to item 1, the protein according to item 2, the partial peptide according to item 6 or a salt thereof bound to the protein according to item 17 or a salt thereof, activin receptors or activin intracellular information transmission molecules in the cells in both the cases,

22. A method for screening a compound or a salt thereof inhibiting or promoting a binding of the labeled protein according to item 1, the labeled protein according to item 2, the labeled partial peptide according to item 6 or a labeled salt thereof to the protein according to item 17 or a salt thereof, activin receptors or activin intracellular information transmission molecules, which comprises comparing the case where the labeled protein according to item 1, the labeled protein according to item 2, the labeled partial peptide according to item 6 or a labeled salt thereof is contacted with a membrane fraction of cells expressing the protein according to item 17 or a salt thereof, activin receptors or activin intracellular information transmission molecules, with the case where the labeled protein according to item 1, the labeled protein according to item 2, the labeled partial peptide according to item 6 or a labeled salt thereof and a test compound are contacted with a membrane fraction of cells expressing the protein according to item 17 or a salt thereof, activin receptors or activin intracellular information transmission molecules, by measuring the amount of the protein according to item 1, the protein according to item 2, the partial peptide according to item 6 or a salt thereof bound to the membrane fraction of the cells in both the cases,

23. A method for screening a compound or a salt thereof inhibiting or promoting a binding of the protein according to item 1, the protein according to item 2, the partial peptide according to item 6 or a salt thereof to the protein according to item 17 or a salt thereof, activin receptors or activin intracellular information transmission molecules, which comprises comparing the case where the protein according to item 1, the protein according to item 2, [the partial peptide according to item 6] or a salt thereof is introduced into cells expressing the protein according to item 17 or a salt thereof, activin receptors or activin intracellular information transmission molecules, with the case where the protein according to item 1, the protein according to item 2, the partial peptide according to item 6 or a salt thereof and a test compound are introduced into cells expressing the protein according to item 17 or a salt thereof, activin receptors or activin intracellular information transmission molecules, by measuring the cell-stimulating activity via the protein according to item 17 or a salt thereof, activin receptors or activin intracellular information transmission molecules in both the cases,

24. The method for determining a protein according to item 16 or the screening method according to any one of items 18 to 23, which comprises using the two-hybrid method,

25. A kit for screening a compound or a salt thereof inhibiting or promoting a binding of the protein according to item 1, the protein according to item 2, the partial peptide according to item 6 or a salt thereof to the protein according to item 17 or a salt thereof, activin receptors or activin intracellular information transmission molecules, which comprises the protein according to item 1, the protein according to item 2, the partial peptide according to item 6 or a salt thereof,

26. A compound or a salt thereof which inhibits or promotes a binding of the protein according to item 1, the protein according to item 2, the partial peptide according to item 6 or a salt thereof to the protein according to item 17 or a salt thereof, activin receptors or activin intracellular information transmission molecules, which is obtained by the screening method according to any one of items 18 to 23 or by the screening kit according to item 25,

27. A pharmaceutical composition comprising the protein according to item 17, the compound according to item 26 or a salt thereof,

28. The pharmaceutical composition according to item 27, which is an agent for preventing or treating Alzheimer's disease, Parkinson's disease, epilepsy or Huntington's chorea,

29. A method for preventing and treating abnormalities in nerve cells or cerebral diseases correlated with the protein according to item 17 or a salt thereof, activin receptors or activin intracellular information transmission molecules, which administering an effective amount of the protein according to item 17, the compound according to item 26 or a salt thereof to mammals, and

30. Use of the protein according to item 17, the compound according to item 26 or a salt thereof for preparing an agent for preventing and treating abnormalities in nerve cells or cerebral diseases correlated with the protein according to item 17 or a salt thereof, activin receptors or activin intracellular information transmission molecules.

Further, the present invention provides:

31. The protein according to item 1 or a salt thereof, wherein the protein comprises an amino acid sequence represented by SEQ ID NO: 5, an amino acid sequence in which 1 or 2 or more (preferably 2 to 10) amino acid sequence in an amino acid sequence represented by SEQ ID NO: 5 excluding an amino acid sequence represented by SEQ ID NO: 6 are deleted, an amino acid sequence in which 1 or 2 or more (preferably 2 to 10) amino acid sequence are added or inserted to an amino acid sequence represented by SEQ ID NO: 5 excluding an amino acid sequence represented by NO: 6, or an amino acid sequence in which 1 or 2 or more (preferably 2 to 10) amino acids in an amino acid sequence represented by SEQ ID NO: 5 excluding an amino acid sequence represented by NO: 6 are substituted with other amino acids,

32. The two-hybrid method according to item 24, wherein yeasts are used,

33. A compound obtained by the screening method according to any one of items 19 to 23 or a salt thereof,

34. A pharmaceutical composition comprising the compound according to item 33 or a salt thereof,

35. Non-human mammals having the extraneous DNA according to item 7 or a mutated DNA thereof,

36. The non-human mammals according to item 35 wherein the non-human mammals are rodents,

37. The non-human mammals according to item 35 wherein the rodents are mice or rats, and

38. A recombinant vector comprising the DNA according to item 7 or a mutated DNA thereof and capable of expression in mammals.

Brief Description of Drawings

Fig. 1 shows the entire nucleotide sequence of cDNA encoding the novel protein of the present invention, as well as an amino acid sequence (SEQ ID NO: 5) encoded thereby.

Fig. 2 shows the entire nucleotide sequence of cDNA encoding the novel protein of the present invention, as well as an amino acid sequence (SEQ ID NO: 5) encoded thereby. Fig. 2 is a continuation of Fig. 1.

Fig. 3 shows the entire nucleotide sequence of cDNA encoding the novel protein of the present invention, as well as an amino acid sequence (SEQ ID NO: 5) encoded thereby. Fig. 3 is a continuation of Fig. 2.
Fig. 4 shows the entire nucleotide sequence of cDNA encoding the novel protein of the present invention, as well as an amino acid sequence (SEQ ID NO: 5) encoded thereby. Fig. 4 is a continuation of Fig. 3.
Fig. 5 shows the entire nucleotide sequence of cDNA encoding the novel protein of the present invention, as well as an amino acid sequence (SEQ ID NO: 5) encoded thereby. Fig. 5 is a continuation of Fig. 4.
Fig. 6 shows the entire nucleotide sequence of cDNA encoding the novel protein of the present invention, as well as an amino acid sequence (SEQ ID NO: 5) encoded thereby. Fig. 6 is a continuation of Fig. 5.
Fig. 7 shows the entire nucleotide sequence of cDNA encoding the novel protein of the present invention, as well as an amino acid sequence (SEQ ID NO: 5) encoded thereby. Fig. 7 is a continuation of Fig. 6.
Fig. 8 shows the entire nucleotide sequence of cDNA encoding the novel protein of the present invention, as well as an amino acid sequence (SEQ ID NO: 5) encoded thereby. Fig. 8 is a continuation of Fig. 7.
Fig. 9 shows the entire nucleotide sequence of cDNA encoding the novel protein of the present invention, as well as an amino acid sequence (SEQ ID NO: 5) encoded thereby. Fig. 9 is a continuation of Fig. 8.
Fig. 10 shows the entire nucleotide sequence of cDNA encoding the novel protein of the present invention, as well as an amino acid sequence (SEQ ID NO: 5) encoded thereby. Fig. 10 is a continuation of Fig. 9.
Fig. 11 shows the entire nucleotide sequence of cDNA encoding the novel protein of the present invention, as well as an amino acid sequence (SEQ ID NO: 5) encoded thereby. Fig. 11 is a continuation of Fig. 10.
Fig. 12 shows the entire nucleotide sequence of cDNA encoding the novel protein of the present invention, as well as an amino acid sequence (SEQ ID NO: 6) encoded thereby.
Fig. 13 shows the entire nucleotide sequence of cDNA encoding the novel protein of the present invention, as well as an amino acid sequence (SEQ ID NO: 6) encoded thereby. Fig. 13 is a continuation of Fig. 12.
Fig. 14 shows the entire nucleotide sequence of cDNA encoding the novel protein of the present invention, as well as an amino acid sequence (SEQ ID NO: 6) encoded thereby. Fig. 14 is a continuation of Fig. 13.
Fig. 15 shows the entire nucleotide sequence of cDNA encoding the novel protein of the present invention, as well as an amino acid sequence (SEQ ID NO: 6) encoded thereby. Fig. 15 is a continuation of Fig. 14.
Fig. 16 shows the entire nucleotide sequence of cDNA encoding the novel protein of the present invention, as well as an amino acid sequence (SEQ ID NO: 6) encoded thereby. Fig. 16 is a continuation of Fig. 15.
Fig. 17 shows the entire nucleotide sequence of cDNA encoding the novel protein of the present invention, as well as an amino acid sequence (SEQ ID NO: 6) encoded thereby. Fig. 17 is a continuation of Fig. 16.
Fig. 18 shows the entire nucleotide sequence of cDNA encoding the novel protein of the present invention, as well as an amino acid sequence (SEQ ID NO: 6) encoded thereby. Fig. 18 is a continuation of Fig. 17.
Fig. 19 shows the entire nucleotide sequence of cDNA encoding the novel protein of the present invention, as well as an amino acid sequence (SEQ ID NO: 6) encoded thereby. Fig. 19 is a continuation of Fig. 18.
Fig. 20 shows the entire nucleotide sequence of cDNA encoding the novel protein of the present invention, as well as an amino acid sequence (SEQ ID NO: 6) encoded thereby. Fig. 20 is a continuation of Fig. 19.
Fig. 21 shows the entire nucleotide sequence of cDNA encoding the novel protein of the present invention, as well as an amino acid sequence (SEQ ID NO: 6) encoded thereby. Fig. 21 is a continuation of Fig. 20.
Fig. 22 shows the entire nucleotide sequence of cDNA encoding the novel protein of the present invention, as well as an amino acid sequence (SEQ ID NO: 6) encoded thereby. Fig. 22 is a continuation of Fig. 21.
Fig. 23 shows the analysis result of expression by Northern hybridization.
Fig. 24 shows the result of examination of the specific interaction of the novel protein of the present invention with Smad3. ARIP1 on the abscissa indicates the novel protein of the present invention. The interaction on the ordinate shows the strength of luciferase activity.

## Best Mode for Carrying Out the Invention

The protein comprising an amino acid sequence substantially identical to the amino acid sequence represented by SEQ ID NO: 5 or NO: 6 of the present invention may be a protein derived from cells (e.g., hepatic cells, spleen cells, nerve cells, glia cells, pancreatic β-cells, bone marrow cells, mesangial cells, Langerhans' cells, epidermal cells, epithelial cells, endothelial cells, fibroblasts, fibrous cells, muscular cells, fat cells, immune cells (e.g., macrophage, T cell, B cell, natural killer cell, mast cell, neutrophils, basophils, eosinophils, monocyte), megakaryocyte, synovial membrane cells, soft-bone cells, bone cells, osteoblast, osteoclast, mammary gland cells or interstitial cells, or their precursor cells, stem cells or cancer cells) or any tissues having such cells, for example, the brain and each site of brain (e.g., olfactory bulb, tonsil nuclei, cerebral basal bulb, hippocampus, thalamus, hypothalamus, cerebral cortex, medulla bulb, cerebellum), spinal cord, pituitary, stomach, pancreas, kidney, liver, gonads, thyroid glands, galls, bone marrow, adrenals, skin, muscles, lungs, digestive tracts (e.g., large and small intestines), blood vessels, heart, thymus, spleen, salivary glands, peripheral blood, prostate, testicles, ovary, placenta, uterus, bone, joints, skeletal muscles, or cells in the hemocyte system or their cultured cell strain (particularly in the brain), from humans or warm-blooded animals (e.g., guinea pig, rat, mouse, chicken, rabbit, pig, sheep, cow, monkey, etc.), as well as a recombinant protein or a synthetic protein.

As the protein comprising an amino acid sequence substantially identical to an amino acid sequence represented by SEQ ID NO: 5 or NO: 6, an amino acid sequence having about 50 % or more, preferably about 70 % or more, more preferably about 90 % or more, and most preferably about 95 % or more homology with an amino acid sequence represented by SEQ ID NO: 5 or NO: 6, etc., are exemplified.

The protein of the present invention comprising an amino acid sequence substantially identical to an amino acid sequence represented by SEQ ID NO: 5 or NO: 6 is preferably, for example, a protein having an amino acid sequence substantially identical to the amino acid sequence represented by SEQ ID NO: 5 or NO: 6 and having substantially identical physiological properties with those of a protein having the amino acid sequence represented by SEQ ID NO: 5 or NO: 6.

As the substantially identical physiological properties, for example, qualitative elements such as receptor affinity, signal transmission ability, specificity of distribution of expression in organs, etc. are exemplified. The terms "substantially identical" mean that these physiological activities are biologically or physiologically homogenous. Therefore, although it is preferable that the activities such as the strength of receptor affinity are equal (e.g., about 0.1- to 20-fold, preferably about 0.5- to 2-fold), quantitative elements such as a degree of these activities and the molecular weight of the protein may be different.

Measurement of the receptor affinity can be carried out according to the per se known method, for example, by the screening method described later.

In addition, as the protein of the present invention, for example, a mutein such as a protein comprising an amino acid sequence in which 1 or 2 or more (preferably about 1 to 30, more preferably about 1 to 10, further preferably a few) amino acids are deleted, an amino acid sequence in which 1 or 2 or more (preferably about 1 to 30, more preferably about 1 to 10, further preferably a few) amino acids are added or inserted to an amino acid sequence represented by SEQ ID NO: 5 or NO: 6, or an amino acid sequence in which 1 or 2 or more (preferably about 1 to 30, more preferably about 1 to 10, further preferably a few) amino acids are substituted with other amino acids, or a protein containing a combination of these amino acid sequences, etc. are exemplified.

In proteins in the present specification, a left end is an N-terminal (amino terminal) and a right end is a C-terminal (carboxyl terminal) according to the convention of the peptide display. Although the proteins of the present invention including a protein comprising an amino acid sequence represented by SEQ ID NO: 5 or NO: 6 have usually carboxyl group (-COOH) or carboxylate ($-COO^-$) as a C-terminal, they may have an amide ($-CONH_2$) or an ester (-COOR) as a C-terminal.

As R in ester, for example $C_{1-6}$ alkyl group such as methyl, ethyl, n-propyl, isopropyl and n-butyl, $C_{3-8}$ cycloalkyl group such as cyclopentyl and cyclohexyl, $C_{6-12}$ aryl group such as phenyl and $\alpha$-naphthyl, and $C_{6-12}$ aryl-$C_{1-2}$ alkyl group such as benzyl, phenetyl and $\alpha$-naphthylmethyl, as well as a pivaloyloxymethyl group used generally in an oral ester are used.

When the protein of the present invention has carboxyl group (or carboxylate) at a position other than a C-terminal, proteins in which carboxyl group is amidated or esterified are also included in the protein of the present invention. As an ester used in this case, for example, an ester at a C-terminal described above is used.

Further, the protein of the present invention includes proteins in which an amino group of a methionine residue of a N-terminal amino acid residue is protected with a protecting group (e.g., $C_{1-6}$ acyl group such as formyl group and acetyl group) in the aforementioned proteins, proteins in which a N-terminal glutamic acid residue formed by cutting in the living body is converted into pyroglutamine, proteins in which for example -OH, COOH, $NH_2$, -SH, etc. on a side chain of an. intramolecular amino acid is protected with a suitable protecting group (e.g., $C_{1-6}$ acyl group such as formyl group and acetyl group), and conjugated proteins such as glycoprotein having sugar chains bound thereto.

As an embodiment of the protein of the present invention, for example, a protein comprising an amino acid sequence represented by SEQ ID NO: 5 or NO: 6, etc. are used.

The protein of the present invention has PDZ domains and/or WW domains, preferably 5 PDZ domains and/or 2 WW domains, and is expressed specifically in the brain. Then, the protein of the present invention binds via the PDZ domains to an activin receptor isolated as a serine/threonine kinase-type receptor, or via the WW domains to an activin intracellular information transmission molecule.

The activin receptor may be of any subtype of activin receptor, but particularly activin receptor IIA-N etc. are preferably used.

As the activin intracellular information transmission molecule, for example, Smad such as Smad1, 2, 3, 4, 5, 6, 7, etc. is exemplified, and the protein of the present invention strongly binds particularly to Smad3.

As the protein of the present invention, in particular, a protein having 5 PDZ domains and 2 WW domains, being expressed specifically in the brain and having an ability to bind to activin receptors and/or Smad3 is preferably used.

As a partial peptide of the protein of the present invention, any peptides may be used as long as they are partial peptides of the protein of the present invention described above, having the physiological activities possessed by the protein of the present invention, for example activities such as receptor affinity and an ability to transmit signal information, and specificity of distribution of expression thereof in organs. For example, a peptide having 100 or more, preferably 250 or more, more preferably 350 or more, further preferably 500 or more and most preferably 800 or more amino acids from the amino acid sequence constituting the protein of the present invention and having receptor affinity and an ability to transmit signal information, etc., are used.

Further, in the partial peptide of the present invention, 1 or 2 or more (preferably about 1 to 10, more preferably a few) amino acids in the amino acid sequence thereof may be deleted, 1 or 2 or more (preferably about 1 to 20, more preferably about 1 to 10, further preferably a few) amino acids may be added to the amino acid sequence thereof, or 1 or 2 or more (preferably about 1 to 10, more preferably a few, further preferably about 1 to 5) amino acids in the amino acid sequence thereof may be substituted with other amino acids.

Although the partial peptide of the present invention has usually carboxyl group (-COOH) or carboxylate ($-COO^-$) as a C-terminal, it may have an amide ($-CONH_2$) or an ester (-COOR) as a C-terminal, as with the protein of the present invention described above.

Further, the partial peptide of the present invention, similar to the protein of the present invention described above, includes peptides in which an amino group of a methionine residue as a N-terminal amino acid residue is protected with a protecting group, peptides in which a N-terminal glutamyl group formed by cutting in the living body is converted into pyroglutamine, peptides in which a substituent group on a side chain of an intramolecular amino acid is protected with a suitable protecting group, and conjugated peptides such as glycopeptides having sugar chains bound thereto.

The partial peptide of the present invention preferably has an ability to bind to activin receptors and/or the above activin intracellular information transmission molecules (particularly Smad3).

As a salt of the protein of the present invention or of a partial peptide thereof, physiologically acceptable acid addition salts are particularly preferable. As such salts, for example, salts with inorganic acids (e.g., hydrochloric acid, phosphoric acid, hydrobromic acid, sulfuric acid) or salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid), etc., are used. Further, salts with inorganic bases (e.g., salts with alkali metals such as sodium and potassium, alkaline earth metals such as calcium and magnesium, or aluminum, ammonium, etc.), salts with organic bases (e.g., trimethylamine, triethylamine, pyridine, picoline, 2,6-lutidine, ethanolamine, diethanolamine, triethanolamine, cyclohexyl amine, dicyclohexyl amine, N,N'-dibenzylethylene diamine, etc.), etc., are also used.

The protein of the present invention or a salt thereof can be produced from cells or tissues of a human being or a warm blood mammal described above by the method of purifying proteins known per se, or can be prepared by culturing a transformant containing a DNA encoding a protein described below. Alternatively, it can also be prepared according to a method of synthesizing a peptide described below.

For production from human or mammalian tissues or cells, the human or mammalian tissues or cells are homogenized and then extracted with e.g. an acid, and the extract can be subjected to purification and isolation by a combination of chromatographic techniques such as reverse-phase chromatography, ion-exchange chromatography, etc.

For synthesis of the protein of the present invention or a partial peptide thereof or a salt thereof or amide derivatives thereof, usually commercially available resin for protein synthesis can be used. Such resin includes, for example, chloromethyl resin, hydroxymethyl resin, benzhydryl amine resin, aminomethyl resin, 4-benzyloxybenzyl alcohol resin, 4-methylbenzhydryl amine resin, PAM resin, 4-hydroxymethyl phenylacetamidemethyl resin, polyacrylamide resin,
4-(2',4' -dimethoxyphenylhydroxymethyl)phenoxy resin,
4-(2',4'-dimethoxyphenyl-Fmoc aminoethyl)phenoxy resin, etc. On the resin described above, each amino acid with the α-amino group and side-chain functional group properly protected is condensed sequentially in accordance with the sequence of the desired protein by the per se known condensation methods. At the end of the reaction, the protein is cleaved off from the resin while various protecting groups are removed, and the product is subjected to a reaction of forming intramolecular disulfide bonds in a highly dilute solution to give the desired protein or an amide thereof.

A wide variety of activating reagents usable for protein synthesis can be used for condensation of the protected amino acids described above, and carbodiimides are particularly preferable. Examples of such carbodiimides include DCC, N,N'-diisopropylcarbodiimide, N-ethyl-N'-(3-dimethylaminoprolyl)carbodiimide, etc. For activation by these reagents, the protected amino acids along with racemization inhibitors (e.g., HOBt, HOOBt) can be added to the resin directly or after the protected amino acids were previously activated as symmetric acid anhydrides or HOBt esters or HOOBt esters.

The solvent used for activation of each protected amino acid or for condensation thereof with the resin can be selected as necessary from those solvents known to be usable in protein condensation reaction. Examples of such solvent include acid amides such as N,N-dimethylformamide, N,N-dimethylacetamide and N-methylpyrrolidone, halogenated hydrocarbons such as methylene chloride and chloroform, alcohols such as trifluoroethanol, sulfoxides such as dimethyl sulfoxide, ethers such as pyridine, dioxane and tetrahydrofuran, nitriles such as acetonitrile and propionitrile, esters such as methyl acetate and ethyl acetate, or a suitable mixture thereof. The reaction temperature is usually selected as necessary within the range known to be usable in the reaction of forming protein bonds, and usually the reaction temperature is selected within the range of about -20 °C to 50 °C. The activated amino acid derivatives are used usually in excess (1.5- to 4-fold). When their condensation is insufficient, their sufficient condensation is achieved as a result of a test using ninhydrin reaction by repeatedly carrying out the condensation reaction without conducting elimination of the protecting groups. When their sufficient condensation is not achieved even by repeatedly carrying out the reaction, the unreacted amino acids are acetylated with acetic anhydride or acetyl imidazole.

The protecting groups for amino groups in the starting materials include, for example, Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosfinothioyl, Fmoc, etc.

The carboxyl group can be protected by, for example, alkyl esterification (e.g., straight-chain, branched or cyclic alkyl esterification such as methyl, ethyl, propyl, butyl, tert-butyl, cyclopentyl, cyclohexyl, cycloheptyl, cycloctyl or 2-adamantyl esterification), aralkyl esterification (e.g., benzyl esterification, 4-nitrobenzyl esterification, 4-methoxybenzyl esterification, 4-chlorobenzyl esterification, benzhydryl esterification), phenacyl esterification, benzyloxycarbonylhydrazidation, tert-butoxycarbonylhydrazidation, tritylhydrazidation, etc.

The hydroxyl group in serine can be protected by, for example, esterification or etherification. A suitable group used in this esterification includes, for example, lower alkanoyl groups such as acetyl group, alloyl groups such as benzoyl group, and carbonic acid-derived groups such as benzyloxycarbonyl group and ethoxycarbonyl group. A suitable group for etherification includes, for example, a benzyl group, tetrahydropyranyl group, t-butyl group, etc.

The protecting group used for the phenolic hydroxyl group in tyrosine includes, for example, Bzl, $Cl_2$-Bzl, 2-nitrobenzyl, Br-Z, tert-butyl, etc.

The protecting group used for imidazole in histidine includes, for example, Tos, 4-methoxy-2,3,6-trimethylbenzenesulfonyl, DNP, benzyloxymethyl, Bum, Boc, Trt, Fmoc, etc.

Activated carboxyl groups in the starting materials include, for example, the corresponding acid anhydrides, azides and active esters (i.e. esters with alcohols such as pentachlorophenol, 2,4,5-trichlorophenol, 2,4-dinitrophenol, cyanomethyl alcohol, p-nitrophenol, HONB, N-hydroxysuccinamide, N-hydroxyphthalimide and HOBt). The activated amino groups in the starting materials include, for example, the corresponding phosphoric acid amides.

Examples of methods for removing (leaving) the protecting groups include catalytic reduction in a hydrogen stream in the presence of a catalyst such as Pd-black or PC-carbon, acid treatment with anhydrous hydrogen fluoride, methane sulfonic acid, trifluoromethane sulfonic acid, trifluoroacetic acid or a mixed solution thereof, base treatment using diisopropylethylamine, triethylamine, piperidine or piperazine, and reduction using sodium in liquid ammonia. The leaving reaction by the acid treatment described above is carried out generally at a temperature of about -20 °C to 40 °C, and it is effective in the acid treatment to add a cation scavenger such as anisole, phenol, thioanisole, m-cresol, p-cresol, dimethylsulfide, 1,4-butanedithiol and 1,2-ethanedithiol. A 2,4-dinitrophenyl group used as a protecting group for imidazole in histidine can also be removed by treatment with thiophenol, while a formyl group used as a protecting group for indole in tryptophan can be removed not only by deprotection by acid treatment in the presence of 1,2-ethanedithiol or 1,4-butanedithiol, but also by alkali treatment using dilute sodium hydroxide solution or dilute ammonia.

Protection and protecting groups for functional groups which should not participate in the reaction of the starting materials, elimination of the protecting groups, and activation of functional groups participating in the reaction can be selected as necessary from known groups or known means.

Another method of obtaining an amide derivative of the protein or a partial peptide thereof includes, for example, protecting the α-carboxyl group of a C-terminal amino acid by amidation, then extending a peptide (protein) chain at the side of the amino group until it attains desired chain length, and thereafter producing a protein (partial peptide) of said peptide chain from which only the protecting group for the N-terminal α-amino group was removed and a protein (partial peptide) of said peptide chain from which only the protecting group for the C-terminal carboxyl group was removed, followed by condensation both the proteins in the mixed solvent as described above. The details of the condensation reaction are the same as described above. The protected protein obtained by condensation is purified, and every protecting group is removed by the method descried above, whereby the desired crude protein (partial peptide) can be obtained. This crude protein (partial peptide) is purified by a wide variety of known purification techniques, and by lyophilizing its major fraction, the desired amide derivative of the protein (partial peptide) can be obtained.

To obtain an ester derivative of the protein or a partial peptide thereof, for example the α-carboxyl group of a C-terminal amino acid is condensed with desired alcohol to form an amino acid ester from which the desired ester derivative of the protein (partial peptide) can be obtained in the same manner as for the amide derivative of the protein (partial peptide).

The partial peptide of the present invention or a salt thereof can be produced according to a per se known peptide synthesis method or by cleaving the protein of the present invention with a suitable peptidase. For example, the peptide synthesis method may be the solid- or liquid-phase synthesis method. That is, the desired peptide can be obtained by condensation of a partial peptide or amino acids capable of constituting the partial peptide of the present invention with the remainder, followed by elimination of protecting groups if any from the product. As the known condensation method and the elimination of the protecting groups, mention is made of e.g. the methods described in (1) to (5) below:

(1) M. Bodanszky and M. A. Ondetti, Peptide Synthesis, Interscience Publisher, New York (1966);
(2) Schroeder and Luebke, The Peptide, Academic Press, New York (1965);
(3) Nobuo Izumiya et al., Basis and Experiments in Peptide Synthesis (in Japanese), Maruzen Co., Ltd. (1975);
(4) Haruaki Yajima and Shunpei Sakakibara, Biochemical Experimental Course 1, Protein Chemistry IV, 205 (1977); and
(5) Haruaki Yajima (supervisor), Development of Medicines, a second series, vol. 14, Peptide Synthesis, Hirokawashoten.

After the reaction, the protein of the present invention or a partial peptide thereof can be isolated and purified by a combination of conventional purification techniques such as solvent extraction, distillation, column chromatography, liquid chromatography and recrystallization. If said protein or a partial peptide thereof is obtained in a free form by these methods, the product can be converted into a suitable salt by a known method, or if the product is obtained in a salt form, it can be converted into a free form by a known method.

As the DNA encoding the protein of the present invention, any DNAs may be used as long as they comprise a nucleotide sequence encoding the protein of the present invention described above. Further, the DNA may be any of a genomic DNA, a genomic DNA library, a cDNA derived form the aforementioned cells or tissues, a cDNA isolated by the per se known method from a cDNA library derived from the aforementioned cells or tissues, and a synthetic DNA.

A vector used for a library may be any of bacteriophage, plasmid, cosmid and phagemide. In addition, amplification can be performed directly by Reverse Transcriptase Polymerase Chain Reaction (abbreviated hereinafter to RT-PCR method) using total RNA or mRNA fraction prepared from the aforementioned cells or tissues.

As the DNA encoding the protein of the present invention, for example, (1) a DNA comprising a nucleotide sequence represented by SEQ ID NO: 7 or NO: 8 or (2) a DNA comprising a nucleotide sequence hybridizing under high stringent conditions with a nucleotide sequence represented by SEQ ID NO: 7 or NO: 8 and encoding a protein having substantially homogenous physiological activities (for example activities such as receptor affinity and signal transmission ability, and specificity of distribution of expression thereof in organs) with those of a protein comprising an amino acid sequence represented by SEQ ID NO: 5 or NO: 6 may be used.

As the DNA which can hybridize, under high stringent conditions, with a nucleotide sequence represented by SEQ ID NO: 7 or NO: 8, for example, a DNA comprising a nucleotide sequence having about 70 % or more, preferably about 80 % or more, more preferably about 90 % or more and most preferably about 95 % or more homology with a nucleotide sequence represented by SEQ ID NO: 7 or NO: 8, etc. are used.

Hybridization can be carried out according to a per se known method or its analogous method, for example a method described in Molecular Cloning, 2nd ed. (J. Sambrook et al., Cold Spring Harbor Lab. Press, 1989). If a commercial library is used, hybridization can be carried out according to the manufacture's instructions. Preferably, hybridization can be conducted under high stringent conditions.

The high stringent conditions refer to those conditions under which the concentration of sodium is about 19 to 40 mM, preferably about 19 to 20 mM, and the temperature is about 50 to 70 °C, preferably about 60 to 65 °C. In particular, the case where the concentration of sodium is about 19 mM and the temperature is about 65 °C is the most preferable.

More specifically, as a DNA encoding a protein comprising an amino acid sequence represented by SEQ ID NO: 5 or NO: 6, a DNA comprising a nucleotide sequence represented by SEQ ID NO: 7 or NO: 8, etc. are used.

As a DNA encoding the partial protein of the present invention, any DNAs may be used as long as they comprise a nucleotide sequence encoding the partial protein of the present invention described above. Further, said DNA may be any of a genomic DNA, a genomic DNA library, a cDNA derived form the aforementioned cells or tissues, a cDNA isolated by the per se known method from a cDNA library derived from the aforementioned cells or tissues, and a synthetic DNA.

As a DNA encoding the partial peptide of the present invention, for example, (1) a DNA comprising a nucleotide sequence represented by SEQ ID NO: 7 or NO: 8 or (2) a DNA comprising a nucleotide sequence hybridizing under high stringent conditions with a nucleotide sequence represented by SEQ ID NO: 7 or NO: 8 and encoding a protein having substantially homogenous physiological activities with those of a protein comprising an amino acid sequence represented by SEQ ID NO: 5 or NO: 6, etc. are used.

As the hybridization method and high stringent conditions, those described above are used.

As a means of cloning the DNA encoding the protein of the present invention or a partial peptide thereof (hereinafter referred to collectively as the protein of the present invention), it is possible to use amplification by the PCR method using synthetic DNA primers having a nucleotide sequence encoding a partial sequence of the protein of the present invention as well as isolation by hybridizing, with a labeled DNA fragment or synthetic DNA encoding a part or the whole of the protein of the present invention, a DNA integrated in a suitable vector or a genomic DNA, a genomic DNA library, a cDNA derived form the aforementioned cells or tissues, a cDNA isolated by the per se known method from a cDNA library derived from the aforementioned cells or tissues. Hybridization can be carried out according to a method described in, for example, Molecular Cloning, 2nd ed. (J. Sambrook et al., Cold Spring Harbor Lab. Press, 1989). If a commercial library is used, hybridization can be carried out according to the manufacture's instructions.

Conversion of DNA into a nucleotide sequence can be carried out by a per se known method, for example by the Gupped duplex method or Kunkel method or its analogous method by using a known kit such as Mutant™-G (Takara Shuzo Co., Ltd.) or Mutant™-K (Takara Shuzo Co., Ltd.).

A DNA encoding a cloned protein can be used as it is depending on the object, or if desired, by digesting it with a restriction enzyme or adding a linker thereto. The DNA may have ATG as a translation initiation codon at the 5'-terminal thereof and TAA, TGA or TAG as a translation termination codon at the 3'-terminal thereof. These translation initiation and termination codons can also be added to the DNA via a suitable synthetic DNA adaptor.

An expression vector for the protein of the present invention can be produced for example by (A) cutting the desired DNA fragment off from the DNA encoding the protein of the present invention and then (B) ligating said DNA fragment to a region downstream from a promoter in a suitable expression vector.

The vector used includes E. coli-derived plasmid (e.g., pBR322, pBR325, pUC12, pUC13), Bacillus subtilis-derived plasmid (e.g., pUB110, pTP5, pC194), yeast-derived plasmid (e.g., pSH19, pSH15), bacteriophage such as λ-phage, and animal viruses such as retrovirus, vaccinia virus and Baculovirus, as well as pA1-11, pXT1, pRc/CMV, pRc/RSV, pcDNAI/Neo, etc.

The promoter used in the present invention may be any suitable promoter compatible with a host used for expression of the gene. For example, when animal cells are used as the host, mention is made of SV40-derived promoter, retrovirus promoter, metallothionein promoter, heat shock promoter, cytomegalovirus promoter, SRα promoter, etc. Among these, cytomegalovirus promoter, SRα promoter, etc. are preferably used. It is preferable to use trp promoter, lac promoter, recA promoter, $\lambda P_L$ promoter, lpp promoter, etc. for microorganisms of the genus Escherichia as the host, SPO1 promoter, SPO2 promoter, penP promoter, etc. for microorganisms of the genus Bacillus as the host, and PH05 promoter, PGK promoter, GAP promoter, ADH promoter, etc. for yeasts as the host. When insect cells are used as the host, polyhedron promoter, P10 promoter, etc. are preferable.

The expression vector may contain an enhancer, a splicing signal, a poly A-added signal, a selective marker, an SV40 origin of replication (also referred to hereinafter as SV40 ori), etc. in addition to the promoter described above. The selective marker includes, for example, dihydrofolate reductase (also referred to hereinafter as dhfr) gene [methotrexate (MTX) resistance], ampicillin resistance gene (also referred to hereinafter as $Amp^r$) and neomycin resistance gene (G418 resistance, also referred to hereinafter as $Neo^r$). In particular, if the dhfr gene is used as a selective marker for CHO ($dhfr^-$) cells, a transformant carrying the desired gene can also be selected in a thymidine-free medium.

A signal sequence compatible with the host is added as necessary to a nucleotide sequence for the N-terminal of the protein of the present invention. An alkali phosphatase signal sequence, Omp A signal sequence, etc. can be utilized for microorganisms of the genus Escherichia used as the host; an α-amylase signal sequence, subtilisin signal sequence, etc. for microorganisms of the genus Bacillus as the host; a mating factor α signal sequence, invertase signal sequence, etc. for yeasts as the host; and an insulin signal sequence, α-interferon signal sequence, antibody molecule signal sequence, etc. for animal cells as the host.

The thus constructed vector containing the DNA encoding the protein of the present invention can be used to produce transformants.

The microorganisms of the genus Escherichia, the microorganisms of the genus Bacillus, yeasts, insect cells, insects, animal cells, etc. are used as the host.

The microorganisms of the genus Escherichia used include, for example, Escherichia coli K12 DH1 (Proc. Natl. Acad. Sci. USA, 60, 160 (1968)), JM103 (Nucleic Acids Research, 9, 309 (1981)), JA221 (Journal of Molecular Biology, 120, 517 (1978)), HB101 (Journal of Molecular Biology, 41, 459 (1969)), C600 (Genetics, 39, 440 (1954)), etc.

The microorganisms of the genus Bacillus used include, for example, Bacillus subtilis MI114 (Gene, 24, 255 (1983)), 207-221 (Journal of Biochemistry, 95, 87 (1984)), etc.

The yeasts used include, for example, Saccharomyces cerevisiae AH22, $AH22R^-$, NA87-11A, DKD-5D, 20B-12, Schizosaccharomyces pombe NCYC1913, NCYC2036, Saccharomyces picjia pastoris, etc.

The insect cells used when the virus is AcNPV include, for example, cells (Spodoptera frugiperda cells; Sf cells) from an established cell line derived from caterpillars of Spodoptera frugiperda, MG1 cells derived from the midgut in Trichoplusia ni, High Five™ cells derived from eggs of Trichoplusia ni, cells derived from Mamestra brassicae and cells derived from Estigmena acrea. When the virus is BmNPV, cells (Bombyx mori N cells; BmN cells) from an established cell line derived from silkworms, etc., are used. The Sf cells used include, for example, Sf9 cells (ATCC CRL1711), Sf21 cells (Vaughn, J. L. et al., In Vivo, 13, 213-217 (1977)), etc.

The insects used include, for example, silkworm caterpillars (Maeda et al., Nature, 315, 592 (1985)).

The animals cells used include, for example, simian cell COS-7, Vero, Chinese hamster cell CHO, DHFR gene-defect Chinese hamster cell CHO ($dhfr^-$ CHO cells), mouse L cells, mouse AtT-20, mouse myeloma cells, rat GH3, human FL cells, etc.

The microorganisms of the genus Escherichia can be transformed according to a method described in e.g. Proc. Natl. Acad. Sci. USA, 69, 2110 (1972) or Gene, 17, 107 (1982).

The microorganisms of the genus Bacillus can be transformed according to a method described in e.g. Molecular & General Genetics, 168, 111 (1979), etc.

The yeasts can be transformed according to a method described in e.g. Methods in Enzymology, 194, 182-187 (1991), Proc. Natl. Acad. Sci. USA, 75, 1929 (1978), etc.

The insect cells or insects can be transformed according to a method described in e.g. Bio/Technology, 6, 47-55 (1988), etc.

The animal cells can be transformed according to a method described in e.g. "Saibo Kogaku Bessatsu 8, Shin-Saibo Kogaku Jikken Protocol (Cell Technology, Extra Number 8, New Experimental Protocol in Cell Technology) ", 263-267 (1995) (published by Shujunsha), Virology, 52, 456 (1973), etc.

Thereby, a transformant transformed with an expression vector comprising a DNA encoding the protein can be obtained.

When transformants derived from the microorganisms of the genus Escherichia or Bacillus as the host are cultured, the medium used for their culture is preferably a liquid medium containing a carbon source, a nitrogen source, inorganic matter, etc. necessary for growth of the transformants. The carbon source includes, for example, glucose, dextrin, soluble starch, sucrose, etc.; the nitrogen source includes, for example,. inorganic or organic materials such as ammonium salts, nitrates, corn steep liquor, peptone, casein, meat extract, soybean cake and potato extract; and the inorganic matter includes, for example, calcium chloride, sodium dihydrogen phosphate, magnesium chloride, etc. In addition, yeast extracts, vitamins, growth promoters, etc. may be added. The pH value of the medium is desirably about 5 to 8.

For example, the medium for culturing the microorganisms of the genus Escherichia is preferably M9 medium containing glucose and casamino acid (Miller, Journal of Experiments in Molecular Genetics, 431-433, Cold Spring Harbor Laboratory, New York, 1972). To permit the promoter to work efficiently, a chemical such as 3β-indolylacrylic acid can be added as necessary.

The transformants from the microorganisms of the genus Escherichia as the host are cultured usually at about 15 to 43 °C for about 3 to 24 hours during which the medium may be aerated or stirred as necessary.

The transformants from the microorganisms of the genus Bacillus as the host are cultured usually at about 30 to 40 °C for about 6 to 24 hours during which the medium may be aerated or stirred as necessary.

The medium used for culturing the transformants from yeasts as the host includes, for example, Burkholder minimum medium (Bostian, K. L. et al., Proc. Natl. Acad. Sci. USA, 77, 4505 (1980)) and SD medium containing 0.5 % casamino acid (Bitter, G. A. et al., Proc. Natl. Acad. Sci. USA, 81, 5330 (1984)). The pH value of the medium is adjusted preferably to about 5 to 8. The transformants are cultured usually at about 20 to 35 °C for about 24 to 72 hours during which the medium may be aerated or stirred as necessary.

The medium used for culturing the transformants from insect cells or insects as the host includes, for example, a medium prepared by adding inactivated additives such as 10 % bovine serum as necessary to Grace's insect medium (Grace, T. C. C., Nature, 195, 788 (1962)). The pH value of the medium is adjusted preferably to about 6.2 to 6.4. The transformants are cultured usually at about 27 °C for about 3 to 5 days during which the medium may be aerated or stirred as necessary.

The medium used for culturing the transformants from animal cells as the host includes, for example, MEM medium containing about 5 to 20 % fetal bovine serum (Science, 122, 501 (1952)), DMEM medium (Virology, 8, 396 (1959)), RPMI 1640 medium (Journal of the American Medical Association, 199, 519 (1967)), 199 medium (Proceeding of the Society for the Biological Medicine, 73, 1 (1950)), etc. The pH value is preferably about 6 to 8. The transformants are cultured usually at about 30 to 40 °C for about 15 to 60 hours during which the medium may be aerated or stirred as necessary.

As described above, the protein of the present invention can be formed in the culture medium or in the transformants.

Separation and purification of the protein of the present invention from the above culture can be performed for example by the following method.

To extract the protein of the present invention from the cultured microorganisms or cells, the cultured microorganisms or cells are collected in a usual manner, suspended in a suitable buffer, disrupted by sonication, lysozyme and/or freezing and thawing, and centrifuged or filtered to give a crude extract of the protein. The buffer may contain protein denaturants such as urea and guanidine hydrochloride and surfactants such as Triton X-100™. When the protein is secreted into the culture liquid, the culture supernatant is collected by separating the supernatant from the cultured microorganisms or cells by a per se known method.

The culture supernatant thus obtained, or the protein contained in the extract, can be purified by a suitable combination of separation and purification techniques known per se. These known separation and purification techniques make use of a method of utilizing solubility, such as salting-out and solvent precipitation, a method of mainly utilizing a difference in molecular weight, such as dialysis, ultrafiltration, gel filtration, and SDS-polyacrylamide gel electrophoresis, a method of utilizing a difference in electric charge, such as ion-exchange chromatography, a method of utilizing specific affinity, such as affinity chromatography, a method of utilizing a difference in hydrophobicity, such as reverse-phase high performance liquid chromatography, a method of utilizing a difference in isoelectric point, such as isoelectric focusing.

If the protein thus obtained is in a free form, it can be converted into a salt by a per se known method or its analogous method, while if the resulting protein is obtained in the form of a salt, it can be converted into a free protein or another salt by a per se known method or its analogous method.

Before or after purification, the protein produced by the transformants can be arbitrarily modified or its partial polypeptide can be removed by allowing a suitable protein-modifying enzyme to act on the protein. For example, trypsin, chymotrypsin, arginyl endopeptidase, protein kinase or glycosidase is used as the protein-modifying enzyme.

The thus formed protein of the present invention or a salt thereof can be measured for its activity by an experiment of binding to a labeled ligand, enzyme immunoassays using specific antibody, etc.

The antibody against the protein of the present invention, a partial peptide thereof or a salt thereof may be a polyclonal or monoclonal antibody capable of recognizing the protein of the present invention, a partial peptide thereof or a salt thereof.

The antibody against the protein of the present invention, a partial peptide thereof or a salt thereof (referred to collectively as the protein of the present invention) can be produced by a known process for producing antibody or antiserum by using the protein of the present invention as the antigen.

[Preparation of a monoclonal antibody]

(a) Preparation of monoclonal antibody-producing cells

The protein of the present invention is administered alone or together with a carrier and a diluent into mammals at a site where the antibody can be produced by administration. To enhance the ability of the animals upon administration to produce the antibody, complete Freund's adjuvant or incomplete Freund's adjuvant may be administered. Administration is conducted usually once every 2 to 6 weeks and about 2 to 10 times in total. The mammals used include, for example, monkey, rabbit, dog, guinea pig, mouse, rat, sheep, goat and chicken, among which mouse and rat are preferably used.

For production of the monoclonal antibody-producing cells, those animals having antibody titer are selected from the warm-blooded animals (e.g. mice) immunized with the antigen, and on the second to fifth day after the final immunization, their spleens or lymph nodes are collected, and the antibody-producing cells contained therein are fused with myeloma cells, whereby monoclonal antibody-producing hybridoma can be produced. The antibody titer in antiserum can be measured for example by reacting the antiserum with the labeled protein described below and then measuring the activity of the labeling agent bound to the antibody. Fusion can be carried out by a known method such as the method of Keller and Millstein (Nature, 256, 495 (1975)). The fusion promoter includes polyethylene glycol (PEG) and Sendai virus, and PEG is preferably used.

The myeloma cells include, for example, NS-1, P3U1, SP2/0 and AP-1 among which P3U1 is preferably used. The ratio of the antibody-producing cells (spleen cells) to the myeloma cells used is from about 1 : 1 to about 20 : 1, and cell fusion can be effected efficiently by incubating the cells for about 1 to 10 minutes at 20 to 40 °C, preferably 30 to 37 °C, in the presence of PEG (preferably PEG 1000 to PEG 6000) at a concentration of about 10 to 80 %.

The anti-protein antibody-producing hybridoma can be screened by various methods, for example by adding a culture supernatant of the hybridoma to a solid phase (e.g., a microplate) having the antibody to the protein, etc., adsorbed thereon directly or along with a carrier and then adding a radioactive substance- or enzyme-labeled anti-immunoglobulin antibody (which is e.g. an anti-mouse immunoglobulin antibody when mouse cells are subjected to cell fusion) or protein A to detect the monoclonal antibody bound to the solid phase or by adding a culture supernatant of the hybridoma to a solid phase having an anti-immunoglobulin antibody or protein A adsorbed thereon and then adding the protein labeled with a radioactive substance or with an enzyme to detect the anti-protein monoclonal antibody bound to the solid phase.

The anti-protein monoclonal antibody can be screened according to a per se known method or its analogous method. Screening can be carried out usually in an animal cell culture medium to which HAT (hypoxanthine, aminopterin, thymidine) was added. The screening and breeding medium may be any medium in which the hybridoma can grow. Examples of such medium include PRMI 1640 medium containing 1 to 20 % (preferably 10 to 20 %) FBS, GIT medium containing 1 to 10 % FBS (Wako Pure Chemical Industries, Ltd.), and a serum-free medium for hybridoma culture (SFM-101, Nissui Pharmaceutical, Co., Ltd.). The culture temperature is usually 20 to 40 °C, preferably about 37 °C. The culture time is usually 5 days to 3 weeks, preferably 1 to 2 weeks. Culture can be conducted usually in 5 % $CO_2$ gas. The antibody titer in a culture supernatant of the hybridoma can be measured in the same manner as in the measurement of the anti-protein antibody titer in antiserum as described above.

(b) Purification of the monoclonal antibody

Separation and purification of the anti-protein monoclonal antibody can be performed according to the per se known method, for example, a method of separating and purifying an immunoglobulin, such as a salting-out method, an alcohol precipitation method, an isoelectric precipitation method, an electrophoresis method, an absorbing-desorbing method with an ion-exchanger (e.g. DEAE), an ultracentrifugation method, a gel filtration method], a specific purifying method for obtaining an antibody by collecting it exclusively on antigen-bound solid phase or on an active adsorbent such as protein A or protein G and then dissociating a bonding thereof to obtain the antibody.

[Preparation of a polyclonal antibody]

The polyclonal antibody of the present invention can be produced by the per se known method or its analogous method. For example, the polyclonal antibody can be produced by making an immune antigen (protein antigen)-carrier protein conjugate, then immunizing a mammal therewith in the same manner as in the method of producing the above monoclonal antibody, collecting a material comprising an antibody to the protein of the present invention from the immunized animal, and separating and purifying the antibody.

For the immune antigen-carrier protein conjugate used for immunizing mammals, the type of the carrier protein and the mixing ratio of the carrier to the hapten are not particularly limited insofar as the desired antibody can be efficiently produced by immunization with the antigen crosslinked via the hapten with the carrier, and for example, the conjugate is produced by coupling the hapten with bovine serum albumin, bovine cyloglobulin, hemocyanin or the like in a ratio of from 1 to about 0.1 to 20, preferably from 1 to about 1 to 5.

The hapten can be coupled with the carrier by use of various condensation agents such as glutaraldehyde, carbodiimide, maleimide-activated ester, and activated ester reagents containing thiol group and dithiopyridyl group.

The resulting condensation product is administered alone or together with a carrier and a diluent into warm-blooded animals at a site where the antibody can be produced. To enhance the ability of the animals upon administration to produce the antibody, complete Freund's adjuvant or incomplete Freund's adjuvant may be administered. Administration is conducted usually once every 2 to 6 weeks and about 3 to 10 times in total.

The polyclonal antibody can be collected form blood, ascites, etc. preferably blood in the warm-blooded animals immunized by the method described above.

The polyclonal antibody titer in antiserum can be measured in the same manner as in the measurement of the antibody titer in antiserum as described above. Separation and purification of the polyclonal antibody can be carried out by a method of separating and purifying immunoglobulin, which is similar to the separation and purification of the monoclonal antibody as described above.

As the antisense DNA having a nucleotide sequence substantially complementary to a DNA or mRNA encoding the protein of the present invention or a partial peptide thereof, any antisense DNAs may be used as long as they are oligonucleotides or derivatives thereof comprising a nucleotide sequence substantially complementary to the whole or a part of the nucleotide sequence of a DNA or mRNA encoding the protein of the present invention or a partial peptide thereof and having an ability to suppress expression of said protein or a partial peptide thereof.

As the nucleotide sequence substantially complementary to the DNA or mRNA, for example, a nucleotide sequence having about 70 % or more, preferably about 80 % or more, more preferably about 90 % or more, and most preferably about 95 % or more homology with the whole or a part of a nucleotide sequence (that is, a complementary chain of said DNA or mRNA) complementary to said DNA or mRNA, etc., are exemplified. Particularly, antisense DNAs having about 70 % or more, preferably about 80 % or more, more preferably about 90 % or more, and further preferably about 95 % or more homology with the partial nucleotide sequence which codes, in the entire nucleotide sequence of the complementary chain of the DNA or mRNA of the present invention, for an N-terminal region of the protein of the present invention (e.g., a nucleotide sequence in the vicinity of the initiation codon) are preferable. These antisense DNAs can be produced using e.g. a known DNA synthesizer.

The protein of the present invention, a partial peptide thereof or a salt thereof (also referred to hereinafter as the protein of the present invention), the DNA encoding the protein of the present invention or a partial peptide thereof (also referred to hereinafter as the DNA of the present invention), the antibody against the protein of the present invention (also referred to hereinafter as the antibody of the present invention) and the antisense DNA can be used in (1) a method of determining a binding protein to the protein of the present invention, (2) construction of a system for expressing a recombinant protein, (3) development of an assay system using the two-hybrid method and screening of candidates for pharmaceutical compounds, (4) practice of a drug design based on comparison with a structurally analogous ligand receptor and as (5) a reagent in formation of probes, PCR primers in genetic diagnosis etc., and further can be used as (6) a chemical for gene therapy etc.

In particular, by acquisition of a binding protein to the protein of the present invention by using the two-hybrid method and further by a binding assay system using the protein of the present invention and an activin receptor, acquired other receptors or an activin intracellular information transmission molecule, a promoter or an inhibitor in an information transmission system specific to warm-blooded animals. such as human beings can be screened, and the promoter or the inhibitor can be used as an agent for preventing or treating various diseases, as described in detail below.

(1) A method of determining a binding protein to the protein of the present invention

The protein of the present invention is useful as a reagent for screening or determining a binding protein to the protein of the present invention.

That is, the present invention provides a method of determining a binding protein to the protein of the present invention, which comprises screening a binding protein interacting with the protein of the present invention.

Specifically, the method of determining a binding protein according to the present invention is a method of determining a protein or a salt thereof interacting, with the protein of the present invention, which comprises introducing (1) a vector having the protein of the present invention fused with a DNA binding region of a transcriptional factor on a host cell expression vector and (2) a fusion library of a test protein with a transcription-activating region, into a host cell having a reporter gene maintaining said binding region of the transcriptional factor on a promoter, and measuring a change in the amount of the expressed reporter gene which is increased by binding of the two kinds of proteins.

The method of determining a binding protein according to the present invention comprises use of the two-hybrid method of detecting the binding protein by converting the interaction of the protein of the present invention with a test protein into the expression of a specific reporter gene.

The method of determining a binding protein according to the present invention is described below in more detail.

As the DNA encoding the protein of the present invention used in the method of determining a binding protein, any DNAs can be used insofar as they comprise a nucleotide sequence encoding a protein containing an amino acid sequence substantially identical to an amino acid sequence represented by SEQ ID NO: 5 or NO: 6 in the present invention or a nucleotide sequence encoding a partial peptide thereof. Further, the DNA may be a genomic DNA from warm-blooded animals (e.g., humans), a genomic DNA library from warm blooded animals (e.g., humans), a cDNA derived from tissues and cells in warm blooded animals (e.g., humans), a DNA isolated by a per se known method from a cDNA library derived from tissues and cells in warm-blooded animals (e.g., humans), a synthetic or semisynthetic DNA. The vector used in the library may be any of bacteriophage, plasmid, cosmid and phagemide. Alternatively, the DNA can be isolated directly by the RT-PCR method using an mRNA fraction prepared from tissues and cells, or can also be produced by chemically producing partial nucleotide sequences respectively and ligating them.

As the test protein library screened, commercial cDNA libraries (e.g. MATCHMAKER cDNA produced by Clontech) derived from various animals, etc. are used.

As the vector expressing a fusion protein of the DNA-binding region with a test protein, pAS2-1, pGBT9, pKAD-09, pSD09, pEG202, pBTM116, etc. are used.

As the vector expressing a fusion protein of an activating region of a transcriptional factor with the present protein, pGAD424, pACT2, pKT10Gal-VP, pJG4-5, pVP16, etc. are used.

As the transcriptional factor, GAL4, LexA, SRF, etc. are used.

As the reporter gene, b-galactosidase gene (LacZ), histidine gene (HIS3), luciferase gene, chloramphenicol acetyl transferase gene, etc. are used.

As the host cells, yeast (Saccharomyces cerevisiae), Escherichia coli, etc. are used, among which CG-1945, Y190, Y187, HF7c, SFY526, L40, EGY48, HIS/L1, 62L yeast strains, etc. are used.

Specifically, in the method of determining a binding protein to the present protein, first a cDNA library expressed in the form of a fusion protein by ligating a plasmid bound to the same frame as that of a DNA fragment containing a nucleotide sequence encoding the present protein and a DNA fragment containing a nucleotide sequence encoding a DNA-binding region on a plasmid (e.g., pAS2-1), to a DNA fragment containing a nucleotide sequence encoding a transcription-activating region of a transcriptional factor (e.g., GAL4) on a plasmid (e.g., pACT2) can be formed according to a method described in, for example, Molecular Cloning, 2nd ed. (J. Sambrook et al., Cold Spring Harbor Lab. Press, 1989).

To introduce the two plasmids described above into host cells (e.g. Saccharomyces cerevisiae Y190), both the plasmids may be transformed simultaneously into the cells, or one plasmid may be first introduced followed by introducing the other plasmid, and for example, transformation can be carried out in a method described in, for example, Methods in Enzymology, 194, 182-187 (1991) or Proc. Natl. Acad. Sci. USA, 75, 1929 (1978).

Expression of a reporter gene in the transformant can be detected by conversion into the amount, emission, etc. of a coloring or fluorescent material formed by the reporter enzyme. More specifically, when the host cells are yeasts, expression of the reporter gene (e.g., β-galactosidase activity) can be detected by blue/white-coloring screening by use of the replica plate method or the filter method, preferably the filter method.

The colonies colored (e.g. blue) by the filter method are treated according to a method described in, for example, A Practical Approach (Bartel, P. L. et al., Oxford University Press, Oxford; 153-179, 1993a), whereby the positive clone can be separated as a single colony.

Recovery of the plasmid DNA from the single transformant thus separated is performed according to a method described in, for example, Gene, 57, 267 (1987), Bio Techniques, 14, 552 (1993), etc., and by determining the nucleotide sequence of said DNA, a receptor for the protein of the present invention can be determined.

Further, the binding protein to the protein of the present invention can also be determined by using a commercial kit, for example MATCHMAKER GAL4 Two-Hybrid Systems (Clontech) according to the manufacture's instructions.

(2) Agent for preventing or treating deficiency of the protein of the present invention

If the binding protein to the protein of the present invention is revealed in the method described in (1) above, the expression site of said binding protein and the action possessed by the protein of the present invention via said binding protein, etc., could be revealed. On the basis of these findings, the DNA encoding the protein of the present invention can be used as an agent for preventing or treating diseases occurring via said binding protein. Further, the protein of the present invention exhibits the activity of binding to activin receptors or activin intracellular information transmission molecules, so it can be used as an agent for preventing or treating diseases occurring via the activin receptors or activin intracellular information transmission molecules.

For example, the gene for the protein of the present invention is expressed specifically in the brain, and therefore, when there are those patients with nerve cell abnormalities or cerebral diseases correlated with said binding protein, activin receptors or activin intracellular information transmission molecules, the action of the protein of the present invention in cerebral cells in said patients can be sufficiently demonstrated by (A) administering the DNA encoding the protein of the present invention into the patients, or (B) inserting the DNA encoding the protein of the present invention into cerebral cells etc. to express the present protein, followed by transplanting said cells in the patients. Accordingly, the DNA encoding the protein of the present invention can be used as a safe and low-toxic preventive or therapeutic agent for diseases occurring via the binding protein to the protein of the present invention or via activin receptors or activin intracellular information transmission molecules

To use the DNA of the present invention as the above therapeutic agent, said DNA can be administered in a usual manner into a human being or a mammal directly or after insertion thereof into a suitable vector such as retrovirus vector, adenovirus vector and adenovirus-associated virus vector. For example, the DNA of the present invention can be used orally as tablets coated with sugar as necessary, as capsules, elixirs and microcapsules, or parenterally as an aseptic solution with water or with other pharmaceutically acceptable solutions or as an injection such as suspension. For example, the DNA of the present invention can be formed by blending with physiologically acceptable carriers, flavors, excipients, vehicles, preservatives, stabilizers and binders, into a unit dosage form required for the generally recognized preparation implementation. An amount of an active ingredient in these pharmaceutical preparations is such that a suitable volume in an indicated range can be obtained.

The additives which can be admixed with the tablets, capsules, etc. include, for example, binders such as gelatin, corn starch, tragacanth, gum arabic, excipients such as crystalline cellulose, swelling agents such as corn starch, gelatin and alginic acid, lubricants such as magnesium stearate, sweeteners such as sucrose, lactose and saccharine, and flavors such as peppermint, akamono oil and cherry. When one capsule is in a unit form, liquid carriers such as fats and oils can be contained in the materials described above. The aseptic composition for injection can be formulated according to conventional pharmaceutical manufacturing by dissolving or suspending the active material and naturally occurring vegetable oils such as sesame oil and coconut oil in vehicles such as injection water. The aqueous solution for injection includes physiological saline or an isotonic solution containing glucose and other supplementary agents (e.g., D-sorbitol, D-mannitol, sodium chloride, etc.), and may be used in combination with suitable solubilizers such as alcohols (e.g., ethanol etc.), polyalcohols (e.g., propylene glycol, polyethylene glycol, etc.) and nonionic surfactants (e.g., Polysorbate 80™, HCO-50, etc.). The oily solution includes sesame oil, soybean oil, etc.,

and may be used in combination with solubilizer such as benzyl benzoate, benzyl alcohol, etc.

Further, the above preventing or treating agent may be blended, for example, with the buffer (e.g., phosphate buffer, sodium acetate buffer, etc.), soothing agents (e.g., benzalkonium chloride, procaine hydrochloride, etc.), stabilizers (e.g., human serum albumin, polyethylene glycol, etc.), preservatives (e.g., benzyl alcohol, phenol, etc.), antioxidants, etc. Usually, the prepared injection is introduced into suitable ampoules. Since the thus obtained preparation is safe and low toxic, it can be administered, for example, to a mammal (e.g., rat, rabbit, sheep, pig, cow, cat, dog, monkey, human being, etc.). A dose of the DNA is different depending on symptom etc., and in the case of oral administration, the dose is usually about 0.1 to 100 mg, preferably about 1.0 to 50 mg and more preferably about 1.0 to 20 mg per day in an adult (60 kg). When parenterally administered, one time dose is different depending upon an administration subject, a subject organ, symptom and an administration method and, for example, in the form of an injection, it is advantageous to administer about 0.01 to 30 mg, preferably about 0.1 to 20 mg and more preferably about 0.1 to 10 mg per day through intravenous injection in an adult (60 kg). In the case of other animals, an amount calculated per 60 kg can be administered.

(3) A method of screening a compound inhibiting or promoting a binding of the protein of the present invention to its binding protein

By use of the protein of the present invention or a salt thereof, or by use of a protein competitive binding assay system using an expression system constructed for a recombinant binding protein, a compound (e.g. peptides, proteins, non-peptide compounds, synthetic compounds, fermentation products, etc.) or a salt thereof inhibiting or promoting a binding of the protein of the present invention to its binding protein can be screened. Further, by use of an expression system (two-hybrid method) for a reporter gene by interaction of the two kinds of proteins in a transformant into which a DNA encoding the protein of the present invention was introduced, a compound (e.g. peptides, proteins, non-peptide compounds, synthetic compounds, fermentation products, etc.) or a salt thereof for inhibiting or promoting a binding of the protein of the present invention to its binding protein can also be screened.

Such compounds include a compound (so-called an agonist) having a cell-stimulating activity (e.g., the activity of promoting or inhibiting growth promotion and phosphorylation of intracellular proteins) via the binding protein and a compound (so-called an antagonist) not having said cell-stimulating activity.

As the protein of the present invention, the protein determined in (1) above, activin receptors and the aforementioned activin intracellular information transmission molecules (particularly, Smad such as Smad3), etc., are used. Hereinafter, these are also referred to as a binding protein to the protein of the present invention.

That is, the present invention provides:

A method of screening a compound or a salt thereof inhibiting or promoting a binding of the protein of the present invention to a binding protein to the protein of the present invention, which comprises comparing:

1) (i) the case where the protein of the present invention or a salt thereof is contacted with a binding protein to the protein of the present invention with (ii) the case where the protein of the present invention or a salt thereof and a test compound are contacted with a binding protein to the protein of the present invention, or
2) (i) a transformant into which a DNA encoding the protein of the present invention and a binding protein to the protein of the present invention with (ii) said transformant brought into contact with a test compound.

The screening method of the present invention comprises comparing:

1) (i) the case where the protein of the present invention or a salt thereof is contacted with a binding protein to the protein of the present invention with (ii) the case where the protein of the present invention or a salt thereof and a test compound are contacted with a binding protein to the protein of the present invention, for example by measuring the amount of the protein of the present invention or a salt thereof bound to a binding protein to the protein of the present invention, the cell-stimulating activity thereof, etc., or
2) (i) a transformant into which a DNA encoding the protein of the present invention and a binding protein to the protein of the present invention with (ii) said transformant brought into contact with a test compound, for example by measuring the degree of coloration indicative of the degree of expression of a reporter gene.

More specifically, the present invention provides:.

(1) a method of screening a compound or a salt thereof inhibiting or promoting a binding of the protein of the present invention or a salt thereof to a binding protein to the protein of the present invention, which comprises comparing the case where the labeled protein of the present invention or a salt thereof is contacted with a binding protein to the present invention with the case where the labeled protein of the present invention or a salt thereof and a test compound are contacted with a binding protein to the protein of the present invention, by measuring the amount of the labeled protein or a salt thereof bound to the binding protein in both the cases,

(2) a method of screening a compound or a salt thereof inhibiting or promoting a binding of the protein of the present invention or a salt thereof to a binding protein to the protein of the present invention in the case where the binding protein to the present invention is a membrane-bound protein (e.g., membrane penetration receptor, channel, etc.), which comprises comparing the case where the labeled protein of the present invention or a salt thereof is contacted with cells containing the binding protein to the protein of the present invention or with a membrane fraction of said cells, with the case where the labeled protein of the present invention or a salt thereof and a test compound are contacted with cells containing the binding protein to the protein of the present invention or with a membrane fraction of said cells, by measuring the amount of the labeled protein of the present invention or a salt thereof bound to said cells or said membrane fraction in both the cases,

(3) a method of screening a compound or a salt thereof inhibiting or promoting a binding of the protein of the present invention or a salt thereof to a binding protein to the protein of the present invention, which comprises comparing the case where the labeled protein of the present invention or a salt thereof is contacted with the binding protein expressed in cells by culturing a transformant containing a DNA encoding the binding protein to the protein of the present invention, with the case where the labeled protein of the present invention or a salt thereof and a test compound are contacted with the binding protein expressed in cells by culturing a transformant containing a DNA encoding the binding protein to the protein of the present invention, by measuring the amount of the labeled protein of the present invention or a salt thereof bound to the binding protein in both the cases, and

(4) a method of screening a compound or a salt thereof inhibiting or promoting a binding of the protein of the present invention or a salt thereof to a binding protein to the protein of the present invention, which comprises comparing host cells into which a plasmid expressing a gene for the protein of the present invention and a plasmid expressing a gene for a binding protein to the protein of the present invention were introduced, with said host cells brought into contact with a test compound, by measuring the expression of a gene group whose expression is regulated by interaction between the protein of the present invention and the binding protein to the protein of the present invention, or physiological reaction (e.g., section of FSH, etc., when said binding protein is an activin receptor) in both the host cells.

Hereinafter, the screening method of the present invention is specifically described.

First, as the binding protein used in the screening method of the present invention, any proteins may be used as long as they contain said binding protein or a salt thereof, but extracts from organs in mammals are preferable. However, since organs derived particularly from human beings are hardly obtainable, the binding protein or a salt thereof expressed in a large amount by genetic recombination technology is suitable for screening.

In order to prepare the binding protein, the aforementioned method is used, but preparation can be performed by expressing a DNA encoding said protein in a mammal cell or an insect cell. As a DNA fragment encoding a protein part of interest, a complementary DNA is usually used but is not necessarily limited to it. For example, a gene fragment or a synthetic DNA may be used. In order to introduce a DNA fragment encoding the binding protein into a host animal cell and express it effectively, it is preferable that the DNA fragment is integrated in downstream of polyhedron promoter from nuclear polyhedrosis virus (NPV) belonging to Baculovirus whose host is an insect, SV40-derived promoter, promoter of retrovirus, metallothionein promoter, human heat shock promoter, cytomegalovirus promoter and SRα promoter. Investigation of an amount and quality of the expressed receptor can be conducted by the per se known method. The investigation can be conducted by, for example, a method described in a reference [Nambi, P. et al., J. Biol. Chem., vol. 267, pp. 19555-19559, 1992].

Therefore, in the screening method of the present invention, a material comprising the binding protein or a salt thereof or a salt thereof may be the binding protein or a salt thereof purified according to the per se known method, or a cell comprising said protein may be used, or when said protein is a membrane-bound protein, a cell membrane fraction containing the same may be used.

In the screening method of the present invention, when a cell comprising the binding protein is used, the cell may be fixed with glutaraldehyde or formalin. The fixing method can be conducted according to the per se known method.

A cell comprising the binding protein refers to a host cell which expressed the binding protein and, as the host cell, Escherichia coli, Bacillus subtilis, yeast, insect cell and animal cell are exemplified.

A cell membrane fraction refers to a fraction in which a cell membrane obtained by the per se known method after disruption of a cell is contained in a large amount. As a method for disrupting a cell, there are a method of squeezing a cell with a Potter-Elvehjem type homogenizer, disruption with a Waring blender or Polytron (manufactured by Kinematica), disruption by sonication, and disruption by jetting a cell through a thin nozzle while pressurizing with a French press. For fractionating a cell membrane, a fractionating method by centrifugation force such as a fractionating centrifugation method and density gradient centrifugation method is mainly used. For example, a disrupted cell solution is centrifuged at a low speed (500 to 3000 rpm) in a short time (usually about 1 minute to 10 minutes), the supernatant is centrifuged at a higher speed (15000 to 30000 rpm) usually for 30 minutes to 2 hours, and the resulting precipitate is used as a membrane fraction. An expressed binding protein and membrane components such as cell-derived phospholipid and membrane protein are contained in a large amount in the membrane fraction.

An amount of a binding protein in a cell comprising the binding protein or its membrane fraction is preferably $10^2$ to $10^8$ molecules, suitably $10^5$ to $10^7$ molecules per cell. In addition, as an expressed amount is larger, the activity of binding to the protein of the present invention per a cellular extract or a membrane fraction (specific activity) is raised, and not only high sensitive screening system can be constructed but also a large amount of samples can be measured at the same lot.

In order to conduct the above methods (1) to (3) for screening a compound inhibiting binding of the protein of the present invention to a binding protein to the protein of the present invention, a suitable binding protein fraction and the labeled protein of the present invention are necessary. As the binding protein fraction, a natural binding protein fraction or a recombinant binding protein fraction having the equal activity thereto are desirable. Here, equal activity denotes an equal activity of binding to the protein of the present invention, etc.

As the labeled protein of the present invention, the labeled protein of the present invention, a labeled compound analogous to the protein of the present invention, etc. are used. For example, the protein of the present invention labeled with [$^3$H], [$^{125}$I], [$^{14}$C], [$^{135}$S] or the like can be utilized.

Specifically, in order to conduct screening of a compound inhibiting binding of the protein of the present invention to a binding protein to the protein of the present invention, first, an authentic binding protein is prepared by suspending a cell comprising the binding protein or a membrane fraction of the cell in a buffer suitable for screening. Any buffers which do not inhibit binding of the protein of the present invention to a binding protein to the protein of the present invention, for example phosphate buffer and Tris-HCl buffer, pH 4 to 10 (desirably pH 6 to 8, may be used. In addition, in order to decrease non-specific binding, surfactants such as CHAPS, Tween-80™ (Kao-Atlas Company), digitonin, and deoxycholate may be added to the buffer. Further, in order to suppress degradation of the binding protein and the protein of the present invention by a protease, a protease inhibitor such as PMSF, leupeptin, E-64 (manufactured by Peptide Laboratory) and pepstain may be added. The protein of the present invention labeled with a predetermined amount of (5000 to 500000 cpm) of a radioactive substance is added to 0.01 ml to 10 ml of the binding protein solution, and simultaneously $10^{-4}$ to $10^{-10}$ M test compound is allowed to be coexist. In order to know an amount of non-specific binding (NSB), a reaction tube to which the unlabeled protein of the present invention has been added in large excess is also prepared. The reaction is conducted at about 0 to 50 °C, preferably about 4 to 37 °C, for about 20 minutes to 24 hours, desirably about 30 minutes to 3 hours. After the reaction, the reaction solution is filtered with a glass fiber filtering paper and washed with a suitable amount of the same buffer, and the radioactivity remaining in the glass fiber filtering paper is measured by a scintillation counter or a γ-counter. Assuming that the count (B0 - NSB) obtained by subtracting the amount of non-specific binding (NSB) from the count (B0) in the absence of the antagonist is 100 %, a test compound by which the amount of specific binding (B - NSB) becomes e.g. 50 % or less can be selected as a candidate for a substance having an antagonistic inhibitory ability, while a test compound by which the amount of specific binding (B - NSB) becomes e.g. 150 % or more can be selected as a candidate for a compound having a binding-promoting ability.

To practice the method in (4) above for screening a compound inhibiting or promoting a binding of the protein to a binding protein to the protein of the present invention, expression of a gene group whose expression is regulated by interaction between the protein of the present invention and a binding protein to the protein of the present invention, or physiological reactions (e.g., secretion of FSH, etc. when the binding protein is an activin receptor) in host cells to which a plasmid. expressing a gene for the protein of the present invention and a plasmid expressing a gene for a binding protein to the protein of the present invention were introduced can be measured by a known method. Specifically, when the host cell is yeast, a transformant transformed by introducing, to a yeast cell, a plasmid expressing a fusion protein between the protein of the present invention and a DNA-binding region of a transcription-regulating factor and a plasmid expressing a fusion protein between a binding protein to the protein of the present invention and an activating region of a transcription-regulating factor is created by the same method as described above. For screening, this transformant is cultured at 30 °C for 2 to 4 days on an agar medium containing $10^{-4}$ to $10^{-10}$ M test compound. As the agar medium, a tryptophan/leucine/histidine-deficient SD medium, a tryptophan/leucine-deficient SD medium, etc. are used. Thereafter, the filter method or the like is used in order to detect expression of the reporter gene as coloration of colonies by β-galactosidase activity. For this detection, a Whatman #5 filter or VWR grade 410 filter moistened with buffer Z/X-gal (Clontech) is placed on a filter to which the transformant adheres, and incubated at 30 °C for 30 minutes to 8 hours, and then the coloration of the colonies on the filter is compared with the coloration of a control transformant not containing a test compound. A test compound added to the culture medium of colonies showing darker coloration than that of the control can be selected as a candidate for a compound having an ability to promote binding, while a test compound added to the culture medium of colonies showing lighter coloration than that of the control can be selected as a candidate as a compound having an ability to inhibit binding. Further, expression of the reporter gene can also be quantified as β-galactosidase activity by measuring the amount of o-nitrophenol formed by decomposition of the substrate. First, the transformant is cultured under shaking at 30 °C for 8 to 24 hours in a liquid medium containing $10^{-4}$ to $10^{-10}$ M test compound. As the liquid medium, a tryptophan/leucine/histidine -deficient SD medium, a tryptophan/leucine-deficient SD medium, etc. are used. Preferably, after overnight culture, an aliquot of the culture liquid is inoculated into YPD medium and cultured under shaking at 30 °C for 3 to 5 hours during which the $OD_{600}$ of 0.5 is increased to 1.0. An aliquot of this culture liquid is centrifuged, and the resulting precipitate is added to a mixture of buffer Z/β-mercaptoethanol and then reacted by adding an o-nitrophenyl galactoside solution (Sigma). The reaction is carried out at 0 to 50 °C for 3 minutes to 24 hours, desirably at 30 °C for 30 minutes to 15 hours, and the absorbance ($OD_{420}$) of the thus yellowed supernatant at 420 nm is measured. From the absorbance ($OD_{420}$) thus obtained, the β-galactosidase activity is calculated as Miller unit by use of the equation below.

In the case where upon adding a test compound to the medium, the β-galactosidase activity is increased by about 10 % or more, preferably about 20 % or more, more preferably about 30 % or more and most preferably about 50 % or more, said test compound can be selected as a candidate for a compound having an ability to promote binding between the protein of the present invention and a binding protein to the present invention. On the other hand, in the case where upon adding a test compound to the medium, the β-galactosidase activity is decreased by about 10 % or more, preferably about 20 % or more, more preferably 30 % or more and most preferably about 50 % or more, said test compound can be selected as a candidate for a compound having an ability to inhibit binding between the protein of the present invention and a binding protein to the present invention.

To perform screening by measuring the expression activity of the reporter gene, a DNA encoding the binding protein to the protein of the present invention is necessary. As the DNA encoding the binding protein to the protein of the present invention, a DNA comprising said DNA, a DNA hybridizing therewith under high stringent conditions, etc. are desirable.

As the test compound, for example, peptides, proteins, non-peptide compounds, synthetic compounds, fermentation products, cellular extracts, plant extracts, animal tissue extracts, etc. are exemplified, and these compounds may be novel compounds or known compounds.

A kit for screening a compound or a salt thereof inhibiting or promoting a binding of the protein of the present invention to a binding protein to the protein of the present invention comprises the protein of the present invention or a salt thereof, a partial peptide thereof or a salt thereof, cells containing the binding protein to the protein of the present invention, a fraction extracted from cells containing the binding protein to the protein of the present invention, or a transformant into which a plasmid expressing a fusion protein between the protein of the present invention and a DNA-binding region of a transcription-regulating factor and a plasmid expressing a fusion protein between the binding protein to the protein of the present invention and an activating region of a transcription-regulating factor were introduced.

As the screening kit of the present invention, for example the following is exemplified.

1. Reagents for screening

(1) Transformant

Yeast Y190 strain transformed by introducing a plasmid expressing a fusion protein between the protein of the present invention and a DNA-binding region of a transcription-regulating factor and a plasmid expressing a fusion protein between an activin IIA-N receptor protein and an activating region of a transcription-regulating factor.

(2) Liquid culture medium

Tryptophan/leucine/histidine-deficient SD medium: An aqueous solution of a yeast nitrogen source (Difco) is sterilized in an autoclave, and then a drop-out solution consisting of L-isoleucine, L-valine, L-adenine hemisulfate, L-arginine hydrochloride, L-lysine hydrochloride, L-methionine, L-phenyl alanine, L-threonine, L-tyrosine and L-uracil, which was sterilized in an autoclave and stored at 4 °C, is added thereto. Further, 40 % dextrose/stock solution (Sigma) sterilized by filtration with a filter is added thereto at a concentration of 2 %, which may be stored at 4 °C or prepared when the medium is used.

YPD medium: Difco peptone and an aqueous solution of a yeast extract are sterilized in an autoclave, and 40 % dextrose sterilized by filtration with a filter is added thereto at a final concentration of 2 %, which may be stored at 4 °C or prepared when the medium is used.

(3) Buffer

Z buffer: A buffer containing $Na_2HPO_4 \cdot 7H_2O$, $NaH_2PO_4 \cdot H_2O$, KCl, $MgSO_4 \cdot 7H_2O$ is adjusted to pH 7 or thereabout and sterilized in an autoclave, which may be stored at 4 °C or prepared when the buffer is used.

Buffer Z/β-mercaptoethanol: A mixture of 100 parts of buffer Z and 0.27 part of β-mercaptoethanol.

(4) Substrate for β-mercaptoethanol

An o-nitrophenyl galactoside solution (Sigma) is dissolved in buffer Z and adjusted to a concentration of 4 mg/ml, and this substrate is prepared when it is used.

(5) Reaction termination solution

1 M sodium carbonate solution stored at 4 °C is used, and this solution may be prepared when it is used.

2. Measurement method

(1) A yeast transformant is cultured under shaking at 30 °C overnight in 1 ml tryptophan/leucine-deficient SD medium containing 5 μl of $10^{-3}$ to $10^{-10}$ M test compound solution.
(2) 0.4 ml of the culture liquid is added to 1.6 ml YPD medium and cultured under shaking at 30 °C for 3 to 5 hours during which the $OD_{600}$ of 0.5 is increased to 1.0.
(3) 0.3 ml of the culture liquid is centrifuged at 14000 rpm for 30 seconds, and the resulting precipitates are suspended in 0.3 ml buffer Z and centrifuged again, and the resulting precipitates are suspended in 0.1 ml buffer Z.
(4) After the suspension is frozen once in liquid nitrogen, it is melted at 37 °C for 30 seconds to 1 minute. 0.7 ml mixture of buffer Z/β-mercaptoethanol and 0.16 ml o-nitrophenyl galactoside solution are added thereto, and the mixture is incubated at 30 °C until the solution turns yellow, and thereafter, 0.4 ml of 1 M sodium carbonate solution is added thereto.
(5) The mixture is centrifuged at 14000 rpm for 10 minutes, and the absorbance of its supernatant at 420 nm is measured, and the β-galactosidase activity is calculated as Miller unit by use of Equation 1 below.

[Equation 1]

$$\beta\text{-Galactosidase activity} = 1000 \times OD_{420}/(t \times V \times OD_{600})$$

$OD_{420}$: Absorbance at 420 nm
t: Reaction time (min)
V: The volume of a suspension of the transformant in buffer Z, used in the reaction x the degree of dilution

A compound or a salt thereof obtained by use of the screening method or the screening kit according to the present invention is either a compound inhibiting binding between the present protein (particularly the protein which has 5 PDZ domains and/or 2 WW domains, is expressed particularly in the brain, and binds to activin receptors and/or activin intracellular information transmission molecules) and a binding protein to the protein of the present invention, or a compound promoting said binding (hereinafter, a promoter).

The compound inhibiting binding of the protein of the present invention and a binding protein to the protein of the present invention includes (1) a compound or a salt thereof (so-called an agonist) which binds to a binding protein to the protein of the present invention thereby inhibiting binding between the protein of the present invention and the binding protein to the protein of the present invention and which in itself has a cell-stimulating activity via the binding protein, (2) a compound or a salt thereof (so-called an antagonist) which binds to a binding protein to the protein of the present invention thereby inhibiting binding between the protein of the present invention and the binding protein to the protein of the present invention but which in itself does not have a cell-stimulating activity via the binding protein, and (3) a compound (referred to hereinafter as an inhibitor) or a salt thereof which inhibits binding between the protein of the present invention and a binding protein to the present invention without binding to the binding protein to the protein of the present invention.

Whether the binding between the compound or a salt thereof obtained by using the screening method or the screening kit according to the present invention and the binding protein to the protein of the present invention is present or not can be confirmed according to the above method of measuring the binding activity.

The cell-stimulating activity via the binding protein can be measured according to the per se known method or its analogous method.

As the compound obtained by using the screening method or the screening kit according to the present invention, peptides, proteins, non-peptide compounds, synthetic compounds, fermentation products, etc. are exemplified, and these compounds may be novel compounds or known compounds.

The agonist and the promoter have the same action as the physiological activity possessed by the protein of the present invention or have the action of enhancing its physiological activity so that depending on the activity of said protein, they are useful in a safe and low-toxic pharmaceutical composition, particularly as an agent for preventing or treating nerve cell abnormalities or cerebral diseases (for example, Alzheimer's disease, Parkinson's disease, epilepsy, Huntington's chorea, etc.) correlated with said binding protein or activin receptors.

On the other hand, since the antagonist or the inhibitor can inhibit the physiological activity possessed by the protein of the present invention, it can be used in a safe and low-toxic pharmaceutical composition inhibiting the activity of said protein, particularly as an agent for preventing or treating nerve cell abnormalities or cerebral diseases (for example, Alzheimer's disease, Parkinson's disease, epilepsy, Huntington's chorea, etc.) correlated with said binding protein or activin receptors.

When the compound or a salt thereof obtained by using the screening method or the screening kit according to the present invention is used as the aforementioned pharmaceutical composition, a conventional means can be used. For example, said compound or a salt thereof can be used orally as tablets coated with sugar as necessary, as capsules, elixirs and microcapsules, or parenterally as an aseptic solution with water or with other pharmaceutically acceptable solutions or as an injection such as suspension. For example, said compound or a salt thereof can be formed by blending with physiologically acceptable carriers, flavors, excipients, vehicles, preservatives, stabilizers and binders, into a unit dosage form required for the generally recognized preparation implementation. An amount of the active ingredient in these pharmaceutical preparations is such that a suitable volume in an indicated range can be obtained.

The additives which can be admixed with the tablets, capsules, etc. include, for example, binders such as gelatin, corn starch, tragacanth, gum arabic, excipients such as crystalline cellulose, swelling agents such as corn starch, gelatin and alginic acid, lubricants such as magnesium stearate, sweeteners such as sucrose, lactose and saccharine, and flavors such as peppermint, akamono oil and cherry. When one capsule is in a unit form, liquid carriers such as fats and oils can be contained in the materials described above. The aseptic composition for injection can be formulated according to conventional pharmaceutical manufacturing by dissolving or suspending the active material and naturally occurring vegetable oils such as sesame oil and coconut oil in vehicles such as injection water.

The aqueous solution for injection includes, for example, physiological saline or an isotonic solution containing glucose and other supplementary agents (e.g., D-sorbitol, D-mannitol, sodium chloride, etc.), and may be used in combination with suitable solubilizers such as alcohols (e.g., ethanol etc.), polyalcohols (e.g., propylene glycol, polyethylene glycol, etc.) and nonionic surfactants (e.g., Polysorbate 80™, HCO-50, etc.).

The oily solution includes, for example, sesame oil, soybean oil, etc., and may be used in combination with solubilizer such as benzyl benzoate, benzyl alcohol, etc. Further, the above preventing or treating agent may be blended, for example, with the buffer (e.g., phosphate buffer, sodium acetate buffer, etc.), soothing agents (e.g., benzalkonium chloride, procaine hydrochloride, etc.), stabilizers (e.g., human serum albumin, polyethylene glycol, etc.), preservatives (e.g., benzyl alcohol, phenol, etc.), antioxidants, etc. Usually, the prepared injection is introduced into suitable ampoules.

Since the thus obtained preparation is safe and low toxic, it can be administered, for example, to a mammal (e.g., rat, rabbit, sheep, pig, cow, cat, dog, monkey, human being, etc.).

A dose of said compound or a salt thereof is different depending on symptom etc., and in the case of oral administration, the dose is usually about 0.1 to 100 mg, preferably about 1.0 to 50 mg and more preferably about 1.0 to 20 mg per day in an adult (60 kg). When parenterally administered, one time dose is different depending upon an administration subject, a subject organ, symptom and an administration method and, for example, in the form of an injection, it is advantageous to administer about 0.01 to 30 mg, preferably about 0.1 to 20 mg and more preferably about 0.1 to 10 mg per day through intravenous injection in an adult (60 kg). In the case of animals other than human beings, an amount calculated per 60 kg can be administered. (4) Quantification of the protein of the present invention or a partial peptide or a salt thereof

The antibody against the protein of the present invention can specifically recognize the protein etc. of the present invention so that it can be used for example in quantification of the protein etc. of the present invention in a test sample, particularly in quantification thereof by sandwich immunoassays.

That is, the present invention provides:

(i) a method of quantifying the protein etc. of the present invention in a test solution, which comprises allowing a test solution and the labeled protein etc. of the present invention to react competitively with an antibody reacting with the protein etc. of the present invention and determining the ratio of the labeled protein etc. of the present invention bound to said antibody; and

(ii) a method of quantifying the protein etc. of the present invention in a test solution, which comprises allowing a test solution to react with the antibody of the present invention insolubilized on a carrier and the labeled antibody of the present invention simultaneously or successively and then measuring the activity of the labeling agent on the insolubilizing carrier, wherein one of the two antibodies is an antibody recognizing an N-terminal region or a C-terminal region of the protein etc. of the present invention, and the other antibody is an antibody reacting with an amino acid sequence of SEQ ID NO: 5 or NO: 6.

The monoclonal antibody recognizing the protein etc. of the present invention (hereinafter, also referred to as the anti-protein antibody) can be used not only for quantifying the protein etc. of the present invention but also for detection thereof by tissue staining etc. For these purposes, the antibody molecule itself may be used, or an $F(ab')_2$, Fab' or Fab fraction of the antibody molecule may be used.

The method of quantifying the protein etc. of the present invention by means of the antibody of the present invention is not particularly limited as long as the method comprises detecting the amount of the antibody, the antigen or an antigen-antibody conjugate, corresponding to the amount of the antigen (e.g., the amount of the protein of the present invention) in a test solution, by chemical or physical means and calculating it on the basis of a standard curve prepared using standard solutions containing known amounts of the antigen. For example, nephelometry, the competitive method, the immunometric method and the sandwich method are preferably used, but the sandwich method described later is particularly preferably used in respect of sensitivity and specificity.

The labeling agent used in the measurement method using a labeling substance includes, for example, a radioisotope, enzyme, fluorescent material and luminescent material. The radioisotope includes, for example, [$^{125}I$], [$^{131}I$], [$^{3}H$], [$^{14}C$], etc.; the enzyme described above is preferably a stable enzyme with high specific activity, which includes, for example, β-galactosidase, β-glucosidase, alkali phosphatase, peroxidase and malate dehydrogenase; the fluorescent material includes, for example, fluorescamine and fluorescein isocyanate; the luminescent material includes luminol, luminol derivatives, luciferin and lucigenin. Further, a biotin-avidin system can also be used for binding the labeling agent to the antibody or antigen.

The antigen or antibody may be insolubilized by physical adsorption or via chemical bonding used conventionally for insolubilization or immobilization of proteins, enzymes, etc. The carrier includes insoluble polysaccharides such as agarose, dextran and cellulose, synthetic resin such as polystyrene, polyacrylamide and silicon, and glass, etc.

In the sandwich method, a test sample is allowed. to react with the insolubilized anti-protein antibody (primary reaction), then the labeled anti-protein antibody is allowed to react therewith (secondary reaction), and the activity of the labeling agent on the insolubilizing carrier is measured, whereby the protein of the present invention in the test sample can be quantitatively determined. The primary and secondary reactions may be carried out in the reverse order, simultaneously, or separately after a predetermined time. The labeling agent and the method of insolubilization may be in accordance with those described above. The antibody used as the solid-phase antibody or as the labeling antibody in immunoassays by the sandwich method may not necessarily be one kind of antibody and may use a mixture of two or more kinds of antibodies for the purpose of improving measurement sensitivity etc.

In the method of measuring the protein etc. of the present invention by the sandwich method according to the present invention, the anti-protein antibodies used in the primary and secondary reactions are preferably those antibodies to which the protein etc. of the present invention are bound at different sites. That is, when the antibody used in, for example, the secondary reaction recognizes a C-terminal region of the protein etc. of the present invention, the other antibody used in the primary reaction recognizes preferably an amino acid sequence represented by SEQ ID NO: 5 or NO: 6.

The antibody against the protein etc. of the present invention can be used not only in the sandwich method but also in other measurement systems such as competitive method, immunometric method and nephelometry.

In the competitive method, an antigen in a test sample and its labeled antigen are allowed to react competitively with the antibody, then the unreacted labeled antigen (F) and the labeled antibody (B) bound to the antibody are separated from each other (B/F separation), and the amount of either labeled B or F is determined to quantify the amount of the antigen in the sample. This reaction method employs either a liquid-phase method in which a soluble antibody is used as the antibody and polyethylene glycol and a second antibody against the above antibody are used in B/F separation, or a solid-phase method in which a solid-phase (i.e. immobilized) antibody is used as the first antibody, or a soluble antibody is used as the first antibody while a solid-phase antibody is used as the second antibody.

In the immunometric method, the antigen in a test solution and the solid-phase antigen are allowed to react competitively with a predetermined amount of the labeled antibody, followed by separating the solid phase from the liquid phase, or alternatively the antigen in a test solution is allowed to react with an excess of the labeled antibody, and then the solid-phase antigen is added thereto to permit the unreacted labeled antibody to be bound to the solid phase, followed by separating the solid phase from the liquid phase. Then, the amount of the label in either phase is measured to quantify the amount of the antigen in the test solution.

Further, in nephelometry, the amount of insoluble precipitates in gel or solution, formed by antigen-antibody reaction, is measured. Laser nephelometry using laser scattering can be applied preferably to the case where precipitates are obtained in a small amount because of a very small amount of antigen in a test solution.

For application of these immunoassays to the quantification method of the present invention, it is not necessary to establish special conditions, procedures etc. A measurement system for the protein etc. of the present invention can be established in an ordinary manner by those skilled in the art by modifying the conventional conditions and procedures. The details of these general technical means can be referred to in general remarks, books, etc. [for example, reference can be made of "Radioimmunoassays" edited by Hirhoshi Irie (published by Kodansha in 1974), "Radioimmunoassays" (2nd edition) edited by Hiroshi Irie (published by Kodansha in 1979), "Enzyme Immunoassays" edited by Eiji Ishikawa et al. (published by Igakushoin in 1978), "Enzyme Immunoassays" (2nd edition) edited by Eiji Ishikawa et al. (published by Igakushoin in 1982), "Enzyme Immunoassays" (3rd edition) edited by Eiji Ishikawa et al. (published by Igakushoin in 1987), Methods in Enzymology, Vol. 70 (Immunochemical Techniques (Part A), Methods in Enzymology, Vol. 73 (Immunochemical Techniques (Part B)), Methods in Enzymology, Vol. 74 (Immunochemical Techniques (Part C)), Methods in Enzymology, Vol. 84 (Immunochemical Techniques (Selected Immunoassays (Part D)), and Methods in Enzymology, Vol. 92 (Immunochemical Techniques (Monoclonal Antibodies and General Immunoassay Methods (Part E)), Methods in Enzymology, Vol. 121 (Immunochemical Techniques (Hybridoma Technology and Monoclonal Antibodies (Part I)) (which are published by Academic Press Ltd.).

By using the antibody of the present invention in the manner as described above, the protein etc. of the present invention can be quantified with good sensitivity.

Further, by quantifying the concentration of the protein etc. of the present invention by means of the antibody against the protein etc. of the present invention, for example, diagnosis of those diseases correlated with the protein etc. of the present invention can be performed.

Further, the antibody of the present invention can be used to detect the protein etc. of the present invention present in humor and tissues. In addition, the antibody of the present invention can be used for preparation of an antibody column used in purification of the protein etc. of the present invention, for detection of the protein etc. of the present invention in each fraction during purification, and for analysis of the behavior of the protein etc. of the present invention in cells examined.

(5) Genetic diagnostic agent

Since the DNA of the present invention can be used for example as a probe to detect abnormalities in a gene encoding the protein of the present invention or a partial peptide thereof in a human being or a mammal (e.g., rat, mouse, guinea pig, rabbit, bird, sheep, pig, cow, horse, cat, dog, monkey, chimpanzee, etc.), the DNA of the present invention is useful as a genetic diagnostic agent for e.g. mutation of said DNA or abnormal accumulation or abnormal reduction of the mRNA, etc.

This genetic diagnosis using the DNA of the present invention can be carried out by techniques known per se, for example Northern hybridization and the PCR-SSCP method (Genomics, Vol. 5, pp. 874-879 (1989) and Proceedings of the National Academy of Sciences of the United States of America, Vol. 86, pp. 2766-2770 (1989)).

(6) DNA containing an antisense DNA

An antisense DNA capable of binding complimentarily to the DNA or mRNA encoding the protein etc. of the present invention to suppress transcription or translation of the DNA or mRNA can suppress abnormal expression of a gene encoding the protein etc. of the present invention. Therefore, the antisense DNA can be used as e.g. a therapeutic or preventive agent for diseases attributable to abnormal expression of a gene encoding the protein etc. of the present invention.

When the antisense DNA is used as the above therapeutic or preventive agent, it can be produced in the same manner as for the aforementioned pharmaceutical preparation containing the DNA of the present invention. For example, said antisense DNA can be administered into a human being or a mammal in a usual manner directly or after insertion thereof into a suitable vector such as retrovirus vector, adenovirus vector and adenovirus-associated virus vector. The antisense DNA is formed alone or along with physiologically acceptable carriers such as a supplementary agent for promoting ingestion, into a pharmaceutical preparation which can be administered via a gene gun or a catheter such as hydrogel catheter.

Further, said antisense DNA can also be used as a diagnostic oligonucleotide probe for examining the presence or expression of the DNA of the present invention in tissues or cells.

(7) Creation of DNA-transferred animals

The present invention provides non-human mammals having an extraneous DNA encoding the protein etc. of the present invention (referred to hereinafter as the extraneous DNA of the present invention) or a mutated DNA thereof (also referred to hereinafter as the extraneous mutated DNA of the present invention).

That is, the present invention provides:

(i) non-human mammals having the extraneous DNA of the present invention or a mutated DNA thereof;
(ii) the non-human mammals according to item (i), wherein the non-human mammals are rodents;
(iii) the non-human mammals according to item (ii), wherein the rodents are mice;
(iv) the non-human mammals according to item (ii), wherein the rodents are rats; and
(v) a recombinant vector comprising the extraneous DNA of the present invention or a mutated DNA thereof and capable of expression in mammals.

The non-human mammals having the extraneous DNA of the present invention or its mutated DNA (hereinafter, referred to as the DNA-transferred animals of the present invention) can be produced by transferring the intended DNA to embryonic cells such as unfertilized cells, fertilized cells, sperms and their initial cells, preferably at the stage of embryonic development in the development of non-human mammals (more preferably at the stage of single cells or fertilized egg cells and generally before the 8-cell stage) by the calcium phosphate method, electric pulse method, lipofection method, aggregation method, microinjection method, particle gun method or DEAE-dextran method. By the method of transferring the DNA, the intended extraneous DNA of the present invention can be transferred to somatic cells, organs in a living body, tissues and cells, and utilized in cell culture and tissue culture, and these cells can also be fused with embryonic cells by the cell fusion per se known method in order to produce the DNA-transferred animals of the present invention.

The non-human mammals used include, for example, rat, mouse, guinea pig, hamster, rabbit, sheep, pig, cow, cat, dog, etc. In particular, rodents, particularly mice (for example, C57BL/6 strain and DBA2 strain as pure strain and B6C3F$_1$ strain, BDF$_1$ strain, B6D2F$_1$ strain, BALB/c strain and ICR strain as crossed strain) and rats (for example, Wister, SD, etc.) are preferable in formation of a morbid animal model system because of their easy breeding with a relatively short period of development and biological cycle.

With respect to the recombinant vector capable of expression in mammals, the "mammals" include human beings in addition to the non-human mammals described above.

The extraneous DNA of the present invention is not the DNA of the present invention inherent in non-human mammals but the DNA encoding the protein of the present invention, which was once isolated and extracted from mammals.

The mutated DNA of the present invention includes a DNA derived from the original DNA of the present invention by mutating the nucleotide sequence thereof, specifically by adding or deleting some bases or by substituting some bases with other bases, and the mutated DNA also includes an abnormal DNA.

The abnormal DNA refers to a gene expressing the abnormal protein of the present invention, and for example, a DNA expressing a protein suppressing the functions of the normal protein of the present invention is used.

The extraneous DNA of the present invention may be derived from animals of either the same species as or a different species from that of the intended animals. For transferring the DNA of the present invention to the intended animals, the DNA is generally advantageously used as a DNA construct having the DNA bound to a region downstream from a promoter capable of expressing the DNA in animal cells. For example, in the case where the DNA of the present invention is to be transferred, a DNA construct (e.g. a vector) having the DNA of the present invention bound to a region downstream from a promoter permitting expression of a DNA having high homology with the DNA of the present invention and derived from various mammals (e.g., rabbit, dog, cat, guinea pig, hamster, rat, mouse, etc.) carrying the DNA of the present invention is microinjected into fertilized eggs of the intended animals, such as mouse fertilized eggs, whereby DNA-transferred animals highly expressing the DNA of the present invention can be produced.

The expression vector carrying said construct includes plasmids derived from E. coli, Bacillus subtilis or yeast, bacteriophage such as λ-phage, retrovirus such as MALONEY [phonetic transcription] leukemia virus, and animal viruses such as vaccinia virus and Baculovirus. In particular, the plasmids derived from E. coli or yeast are preferably used.

The promoter for regulating expression of the DNA includes, for example, promoters derived from viruses (e. g., simian virus, cytomegalovirus, MALONEY [phonetic] leukemia virus, JC virus, breast cancer virus, poliovirus, etc.), promoters derived from various mammals (human being, rabbit, dog, cat, guinea pig, hamster, rat, mouse, etc.), for example, promoters for albumin, insulin II, UROPLAKIN (phonetic) II, elastase, erythropoietin, endoserine, muscular creatine kinase, glia fibrous acidic protein, glutathione S-transferase, platelet-derived growth factor β, keratin K1, K10 and K14, collagen I and II type, cyclic AMP-dependent kinase βI subunit, dystrophin, tartaric acid-resistant alkali phosphatase, atrial natriuretic factor, endothelial receptor tyrosine kinase (abbreviated generally to Tie 2), sodium potassium adenosine triphosphatase (Na,K-ATPase), neurofilament light chain, metallothionein I and IIA, metalloproteinase 1 tissue inhibitor, MHC class I antigen (H-2L), H-ras, renin, dopamine β-hydroxylase, thyroid peroxidase, polypeptide chain-extending factor 1α (EF-1α), β-actin, α- and β-myosin heavy chains, myosin light chains 1 and 2, myelin basic protein, thyroglobulin, Thy-1, immunoglobulin, H chain variable region (VNP), serum amyloid P component, myoglobin, troponin, smooth muscular α-actin, preproenkephalin A and vasopressin, among which promoters such as cytomegalovirus promoter, human polypeptide-extending factor 1α (EF-1α) promoter, human and chicken β actin promoters can be preferably used because of their ability to express the DNA in the whole body.

Preferably, the vector has a sequence (generally called a terminator) for terminating transcription of the intended mRNA in the DNA-transferred mammals, and for example the sequence of each DNA derived from viruses or mammals, preferably SV40 terminator from simian virus, can be used.

Further, a splicing signal for each DNA, an enhancer region, a part of introns from eucaryotic DNA, etc. can also be linked depending on the object to a 5'-upstream region from the promoter, to a region between the promoter and the translating region, or to a 3'-downstream region from the translating region.

The translating region for the normal protein of the present invention can be obtained in the per se known method as the whole or a part of genomic DNA derived from the liver, kidney, thyroid cells, and fibroblasts derived from various mammals (e.g., rabbit, dog, cat, guinea pig, hamster, rat, mouse, human being) and genomic DNA from a variety of commercial genomic DNA libraries or by use of a complementary DNA as the starting material prepared in the per se known method from RNA derived from the liver, kidney, thyroid cells, and fibroblasts. Further, the extraneous abnormal DNA can be obtained from the above cells or tissues with those diseases attributable to a mutation in the protein of the present invention. In addition, a mutated translating region can be produced by a point mutagenesis method of mutating the translating region for the normal protein obtained from the above cells or tissues.

The translating region can be constructed as a DNA construct capable of expression in the DNA-transferred animals by conventional DNA engineering means for linking it to a downstream region from the promoter or if desired to an upstream region from the transcription termination site.

The extraneous DNA of the present invention is transferred to cells at the stage of fertilized egg cells so as to secure the presence of the DNA in all embryonic cells and somatic cells of the intended mammals. The presence of the extraneous DNA of the present invention in the embryonic cells in the produced animals after transfer of the DNA means that the extraneous DNA of the present invention is maintained in all embryonic cells and somatic cells in the offspring of the produced animals. The offspring of the produced animals, which has inherited the extraneous DNA of the present invention, has the extraneous DNA of the present invention in all embryonic cells and somatic cells thereof.

After it is confirmed that the non-human mammals having the extraneous normal DNA of the present invention transferred to them maintain the DNA stably through crossbreeding, they can be bred generation after generation as animals carrying said DNA under normal breeding environments.

The extraneous DNA of the present invention is transferred to cells at the stage of fertilized egg cells so as to secure the presence of the DNA in excess in all embryonic cells and somatic cells in the intended mammals. The presence of the extraneous DNA of the present invention in excess in the embryonic cells in the produced animals after transfer of the DNA means that the extraneous DNA of the present invention is maintained in excess in all embryonic cells and somatic cells in the offspring of the produced animals. The offspring of the produced animals, which has inherited the extraneous DNA of the present invention, has the extraneous DNA of the present invention in excess in all embryonic cells and somatic cells thereof. Homozygote animals having the introduced DNA in both homogenous chromosomes are obtained, and these male and female animals are crossbred to produce the offspring having said DNA in excess and capable of breeding generation after generation.

The non-human mammals having the normal DNA of the present invention can be used as a morbid animal model since the normal DNA of the present invention is highly expressed in these animals to promote the functions of endogenous normal DNA, resulting in the onset of functional exasperation by the protein of the present invention. For example, the normal DNA-transferred animals of the present invention can be used to elucidate the mechanism of the morbid functional exasperation caused by the protein of the present invention or those diseases correlated with the protein of the present invention or to examine the method of treating these diseases.

Further, the mammals having the extraneous normal DNA of the present invention transferred to them can also be used in a test for screening a therapeutic agent against those diseases correlated with the protein of the present invention because these animals have the symptom of an increase in the protein of the present invention.

On the other hand, the non-human mammals having the extraneous abnormal DNA of the present invention transferred to them are confirmed to maintain the extraneous DNA stably through crossbreeding, and they can be then bred generation after generation as animals carrying said DNA under normal breeding environments. Further, the desired extraneous DNA can be used as a starting material by integrating it in the plasmid described above. The DNA construct having the promoter can be produced in usual DNA engineering means. The abnormal DNA of the present invention is transferred to cells at the stage of fertilized egg cells so as to secure the presence of the DNA in all embryonic cells and somatic cells of the intended mammals. The presence of the abnormal DNA of the present invention in the embryonic cells in the produced animals after transfer of the DNA means that the abnormal DNA of the present invention is maintained in all embryonic cells and somatic cells in the offspring of the produced animals. The offspring of the produced animals, which has inherited the DNA, has the abnormal DNA of the present invention in all embryonic cells and somatic cells thereof. Homozygote animals having the introduced DNA in both homogenous chromosomes are obtained, and these male and female animals can be crossbred to produce the offspring having said DNA and capable of breeding generation after generation.

The non-human mammals having the abnormal DNA of the present invention can be used as a morbid animal model since the abnormal DNA of the present invention is highly expressed in these animals to inhibit the functions of endogenous normal DNA, resulting in the onset of functional inactivated refractory conditions [literal translation] of the protein of the present invention. For example, the abnormal DNA-transferred animals of the present invention can be used to elucidate the mechanism of the morbid functional inactivated refractory conditions of the protein of the present invention or to examine the method of treating this disease.

For specific applicability, the abnormal DNA high-expression animals of the present invention serve as a model for elucidation of the functional inhibition (dominant negative action) of the normal protein by the abnormal protein of the present invention in the functional inactivated refractory conditions of the protein of the present invention. Further, the mammals having the extraneous abnormal DNA of the present invention transferred to them can also be used in a test for screening a therapeutic agent against the functional inactivated refractory conditions of the protein of the present invention since these animals have the symptom of an increase in the protein of the present invention.

Other applicability of the two DNA-transfected animals of the present invention includes:

1. Use thereof as a cellular source for tissue culture;
2. Analysis of the relationship with proteins specifically expressed or activated by the protein of the present invention by direct analysis of mRNA in tissues in the DNA-transferred animals of the present invention or by analysis of tissues where the protein is expressed in the DNA-transferred animals;
3. Screening of chemicals promoting or inhibiting the functions of cells by using the cells described according to item 1; and
4. Isolation and purification of the mutated protein of the present invention and preparation of an antibody against said protein.

Further, the DNA-transferred animals of the present invention can be used to examine the clinical symptoms of those diseases correlated with the protein of the present invention, including the functional inactivated refractory conditions of the protein of the present invention, or to obtain further detailed pathological findings in each organ in a model with those diseases correlated with the protein of the present invention, and to contribute to development of new therapeutic methods as well as to the study and treatment of secondary diseases resulting from said diseases.

Further, each organ is removed from the DNA-transferred animals of the present invention, cut into thin pieces, and digested with proteinase such as trypsin, whereby the free DNA-transferred cells can be obtained, and these cells can be cultured and these cultured cells can be established as a cell line. By identifying those cells producing the protein of the present invention and by examining their relationship with differentiation or multiplication or the signal transfer mechanism therein, their abnormalities can be examined so that these cells can be used as an useful material for study on the protein of the present invention and in elucidating their action.

The DNA-transferred animals of the present invention can also be used to provide an effective and rapid screening method for therapeutic agents for those diseases correlated with the protein of the present invention by use of the examination method and quantification method described above, so that the therapeutic agents against these diseases including the functional inactive type refractory conditions of the protein of the present invention can be developed. Further, the DNA-transferred animals of the present invention or the extraneous DNA expression vector of the present invention can be used for examination and development of the gene therapeutic method to treat those diseases correlated with the protein of the present invention.

When bases or amino acids are expressed in abbreviations in the present specification and drawings, the following abbreviations in accordance with IUPAC-IUB Commission on Biochemical Nomenclature or based on customary abbreviations in this field are used. If amino acids can occur as optical isomers, L-isomers are referred to unless otherwise specified.

DNA: deoxyribonucleic acid
cDNA: complementary deoxyribonucleic acid
A: adenine
T: thymine

G: guanine
C: cytosine
RNA: ribonucleic acid
mRNA: messenger ribonucleic acid
dATP: deoxyadenosine triphosphate
dTTP: deoxythymidine triphosphate
dGTP: deoxyguanosine triphosphate
dCTP: deoxycytidine triphosphate
ATP: adenosine triphosphate
EDTA: ethylene diamine tetraacetate
SDS: sodium dodecyl sulfate
EIA: enzyme immunoassay
Gly: glycine
Ala: alanine
Val: valine
Leu: leucine
Ile: isoleucine
Ser: serine
Thr: threonine
Cys: cysteine
Met: methionine
Glu: glutamic acid
Asp: aspartic acid
Lys: lysine
Arg: arginine
His: histidine
Phe: phenyl alanine
Tyr: tyrosine
Trp: tryptophan
Pro: proline
Asn: asparagine
Gln: glutamine
pGl: pyroglutamic acid
Me: methyl group
Et: ethyl group
Bu: butyl group
Ph: phenyl group
TC: thiazolidine-4 (R)-carboxamide group

The substituent groups, protecting groups and reagents appearing frequently in the present specification are expressed in the following symbols.

Tos: p-toluene sulfonyl
CHO: formyl
Bzl: benzyl
$Cl_2$-Bzl: 2,6-dichlorobenzyl
Bom: benzyloxymethyl
Z: benzyloxycarbonyl
Cl-Z: 2-chlorobenzyloxycarbonyl
Br-Z: 2-bromobenzyloxycarbonyl
Boc: t-butoxycarbonyl
DNP: dinitrophenol
Trt: trityl
Bum: t-butoxymethyl
Fmoc: N-9-fluorenyl methoxycarbonyl
HOBt: 1-hydroxybenztriazole
HOOBt: 3,4-dihydro-3-hydroxy-4-oxo-1,2,3-benzotriazine
HONB: l-hydroxy-5-norbornane-2,3-dicarbodiimide

DCC: N,N'-dicyclohexyl carbodiimide

The sequence numbers in the Sequence Listing in the present specification show the following sequences:

SEQ ID NO: 1 shows the nucleotide sequence of a DNA fragment encoding an intracellular domain of activin IIA-N receptor protein as bait plasmid used in the two-hybrid method.
SEQ ID NO: 2 shows the nucleotide sequence of a DNA fragment as a probe used in Northern hybridization.
SEQ ID NO: 3 shows the nucleotide sequence of a DNA fragment as a probe used in Northern hybridization.
SEQ ID NO: 4 shows the nucleotide sequence of a DNA fragment as a probe used in Northern hybridization.
SEQ ID NO: 5 shows the amino acid sequence of the protein of the present invention.
SEQ ID NO: 6 shows the amino acid sequence of the protein of the present invention.
SEQ ID NO: 7 shows the nucleotide sequence of a cDNA encoding the protein of the present invention having an amino acid sequence represented by SEQ ID NO: 5.
SEQ ID NO: 8 shows the nucleotide sequence of a cDNA encoding the protein of the present invention having an amino acid sequence represented by SEQ ID NO: 6.

The transformant Escherichia coli DH5α/pBSYN3-6 obtained in Example 1 below has been deposited under FERM BP-6592 from December 2, 1998 with the National Institute of Bioscience and Human-Technology (NIBH), the Agency of Industrial Science of Technology, the Ministry of International Trade and Industry, and under IFO 16221 with Institution Fermentation Organization (IFO) from November 18, 1998.

Hereinafter, the present invention is described in more detail by reference to the Reference Example and the Examples, but is not limited thereto. The method of operating the two-hybrid method using yeasts was conducted according to instructions attached to the commercial kit, and the genetic manipulation using E. coli was conducted according to methods described in Molecular Cloning.

Example 1 Cloning of a cDNA encoding the protein of the present invention

(1) Screening of a protein interacting with activin IIA-N receptor protein by the two-hybrid method

For the following screening of a protein interacting with activin IIA-N receptor protein from a cDNA library, MATCHMAKER™ Two-Hybrid System 2 (Cat. No. K1604-1, Clontech) was used as a kit.

According to the method described above, an Eco RI site and Bam HI site in vector pAS2-1 for DNA ligation were cleaved with suitable restriction enzymes, treated with alkali phosphatase and then purified. Thereafter, a DNA fragment encoding an intracellular domain in the activin IIA-N receptor protein set forth in SEQ ID NO: 1 was ligated thereto, to give a plasmid (pAS-IIA-N) expressing the desired GAL4 DNA-binding domain and activin IIA-N receptor intracellular domain as a fusion protein. As the GAL4 transcription-activating region-fused library plasmid, a commercial mouse brain MATCHMAKER cDNA library (Clontech) was used.

A single colony (diameter of 2 to 3 mm) of yeast strain Y190 was inoculated into 20 ml tryptophan-deficient SD medium and cultured under shaking for 18 hours at 30 °C. 10 ml of this culture was inoculated into 300 ml YPD medium such that its $OD_{600}$ of 0.2 became 0.3, and the yeast was cultured under shaking at 30 °C for 3 hours. The supernatant was transferred to a sterilized centrifuge tube and centrifuged at 1000×g for 5 minutes at room temperature. The supernatant was discarded, and the cells were suspended in 25 ml sterilized water and centrifuged again at 1000×g for 5 minutes at room temperature. The supernatant was discarded, and the cells were suspended in a mixed solution of 1.5 ml TE buffer (0.01 M Tris-HCl, 1 mM EDTA, pH 7.5)/0.1 M lithium acetate (pH 7.5) and used for the subsequent transformation.

1 ml of the cell suspension prepared above was added to 10 μg of the previously prepared plasmid (pAS-IIA-N) and 2 mg herring spermary carrier DNA (Clontech), and well mixed. Further, 6 ml mixed solution of PEG (40 % PEG 4000)/TE buffer/0.1 M. lithium acetate was added thereto and mixed in a vortex mixer. After the mixture was shaken at 30 °C at 200 rpm for 30 minutes, 700 μl DMSO (final concentration, 10 %) was added thereto, and the mixture was stirred gently. Thereafter, the mixture was heated at 42 °C for 15 minutes while being occasionally shaken, and after the container was cooled on ice, the mixture was centrifuged at 1000×g for 5 minutes. The supernatant was discarded, and the cells were suspended in 0.5 ml TE buffer. 100 μl of the resulting cell suspension was spread onto a tryptophan-deficient SD medium and cultured at 30 °C for 4 days to give a strain maintaining said plasmid stably. Subsequently, the GAL4 transcription-activating region-fused library plasmid was also introduced in the same manner into said yeast strain. 0.2 ml of the resulting transformant was plated on a tryptophan/leucine/histidine-deficient SD medium and cultured at 30 °C for 8 days, and $His^+$ colonies were streaked on a tryptophan/leucine/histidine-deficient agar medium.

5 ml mixture of buffer Z ($Na_2HPO_4 \cdot 7H_2O$, $NaH_2PO_4 \cdot H_2O$, KCl, $MgSO_4 \cdot 7H_2O$, pH 7)/X-gal solution (2 % 5-bromo-4-chloro-3-indolyl-β-D-galactoside solution in DMF)/β-mercaptoethanol was placed in a Petri dish, to moisten a sterilized Whatman #5 filter therein. Another filter was placed on the agar plate containing said transformant colonies, and then this filter was removed and frozen in liquid nitrogen with the colonies facing upward. The filter was removed from liquid nitrogen and then thawed at room temperature, and with the colonies facing upward, the filter was placed on the previously moistened filter. A lid was placed on the Petri dish, and the colonies were incubated at 30 °C for 1 hour, and about 100 blue positive colonies were separated therefrom. The resulting respective positive clones were inoculated into 3 ml leucine-deficient SD liquid medium and cultured for 2 days, and the culture liquid was diluted 10000-fold, plated on a leucine-deficient SD plate, and incubated at 30 °C for 3 days. 20 to 30 colonies were picked up with a sterilized picker and replicated onto a tryptophan/leucine-deficient SD plate and a leucine-deficient SD plate. Then, those colonies requiring a tryptophan nutrient were selected from the previous. positive colonies, and their β-galactosidase activity was assayed to select 2 colonies having no activity.

The resulting true positive colonies were inoculated into 2 ml YPD liquid medium and cultured at 30 °C overnight. The culture was centrifuged for 5 minutes at room temperature, and after the supernatant was discarded, 0.2 ml yeast-lyzing solution (2 % Triton X-100, 1 % SDS, 100 mM NaCl, 10 mM Tris-HCl (pH 8.0), 1 mM EDTA) was added to suspend the microorganisms. 0.2 ml phenol/chloroform/isoamyl alcohol. (25 : 24 : 1) and glass beads washed with an acid were added thereto, and the mixture was stirred for 2 minutes with a vortex mixer. After centrifugation at 14000 rpm for 5 minutes at room temperature, the separated supernatant was mixed with a 1/10 volume of 3 M sodium acetate solution (pH 5.2) and a 2.5-fold excess volume of ethanol. The resulting precipitates were washed with 70 % ethanol and dissolved in 20 μl sterilized water. 1 μl of this solution was introduced by electroporation into E. coli HB101, and the plasmid DNA was purified by the usual mini-prep method to give the desired cDNA (YN3).

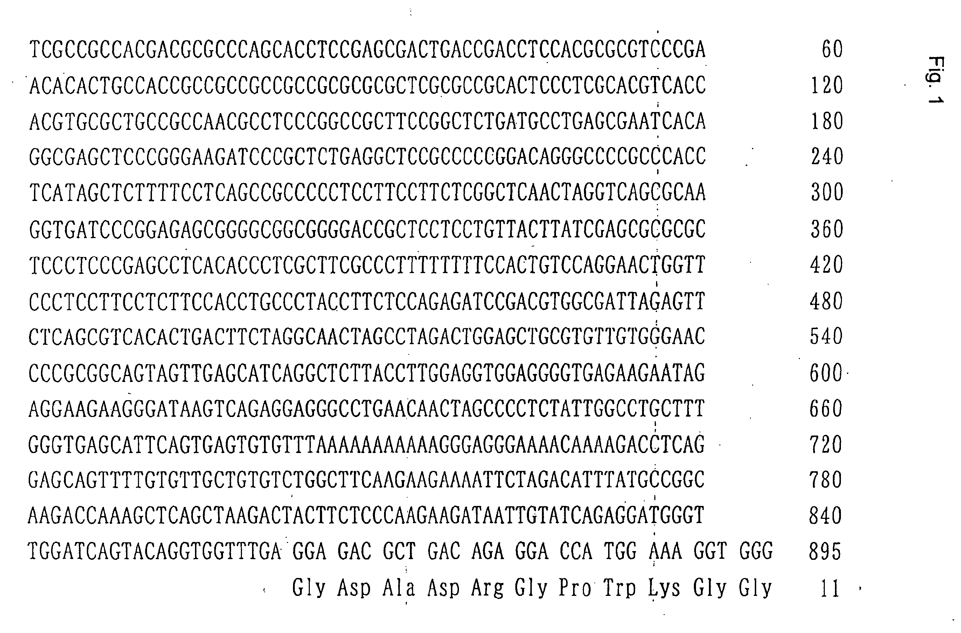

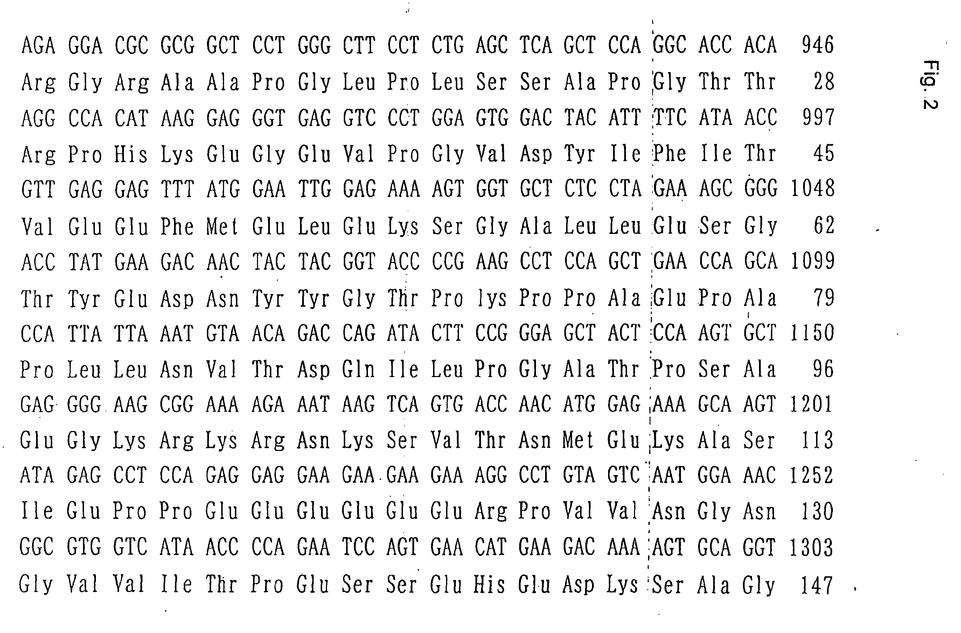

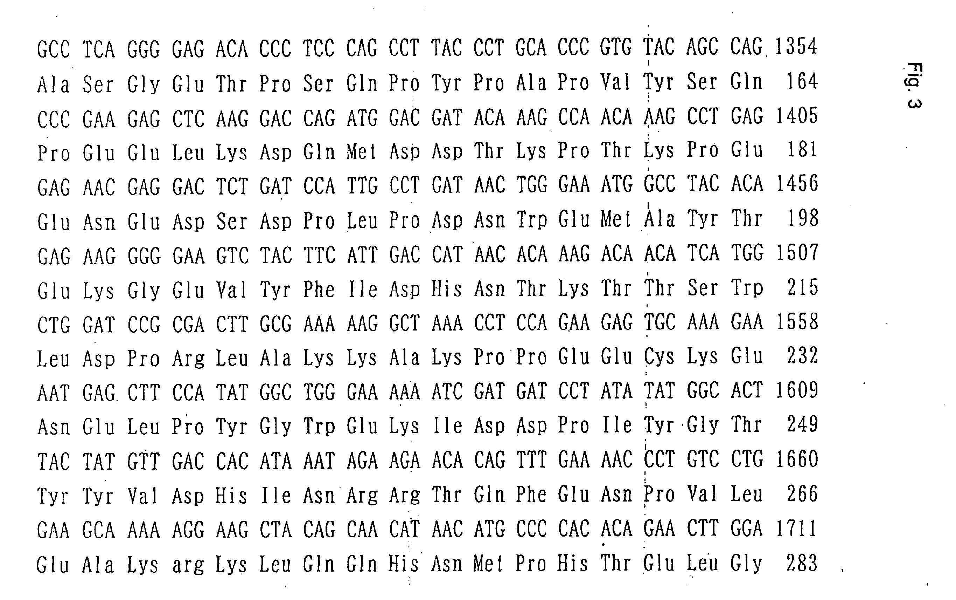

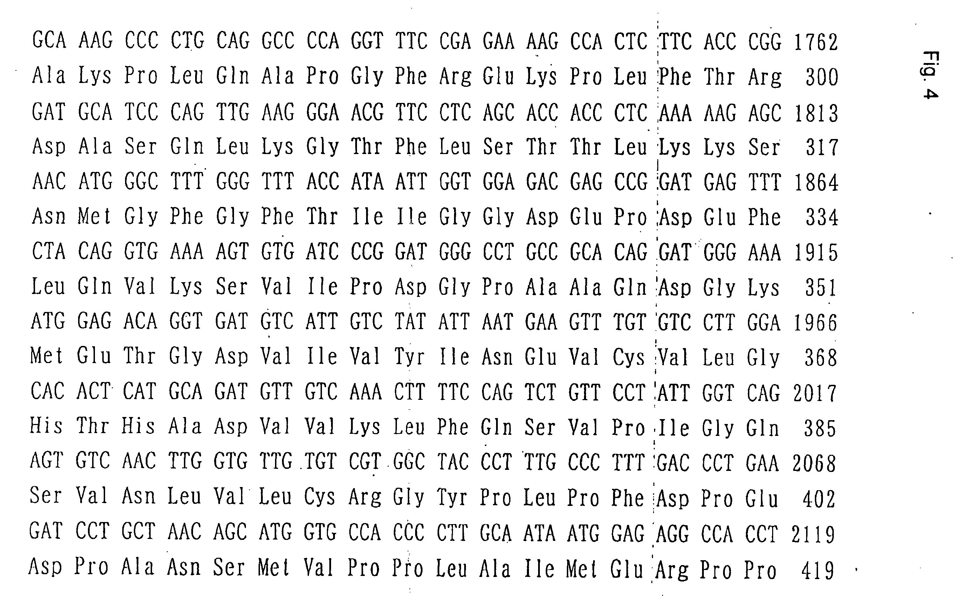

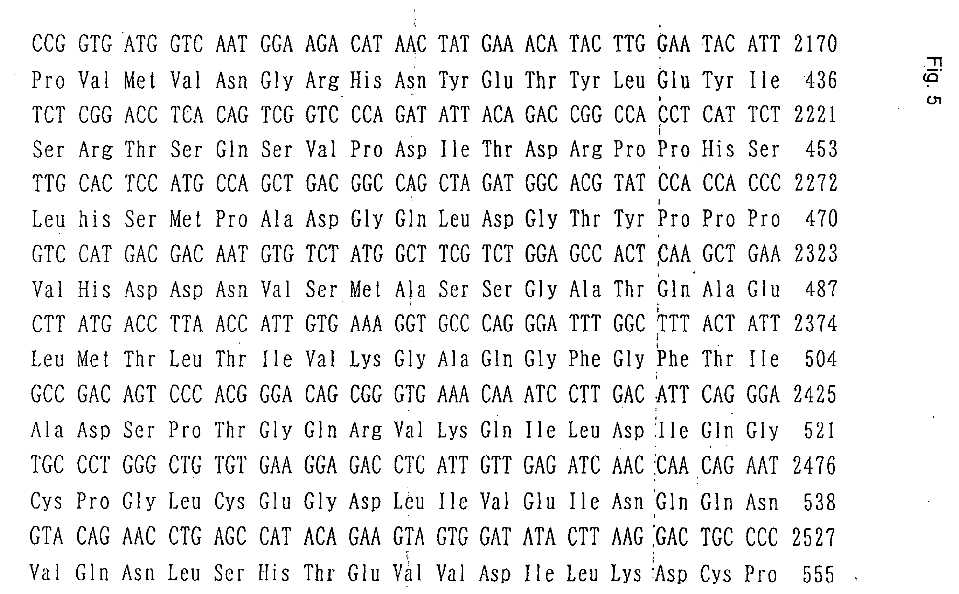

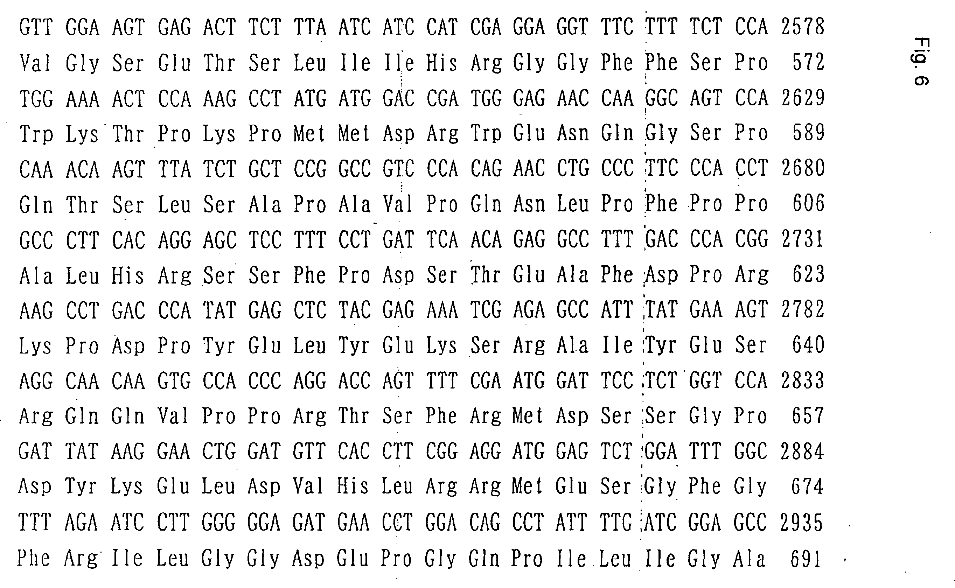

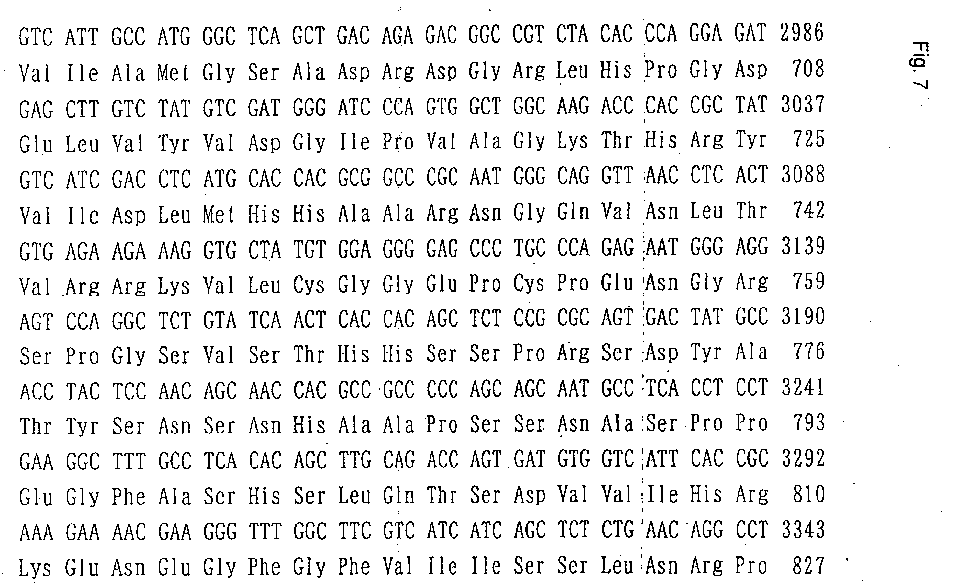

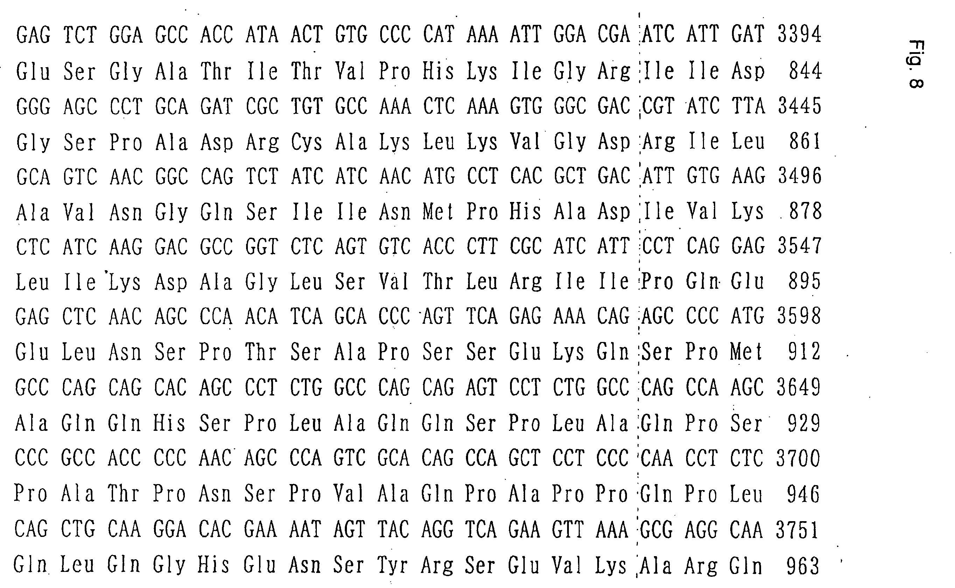

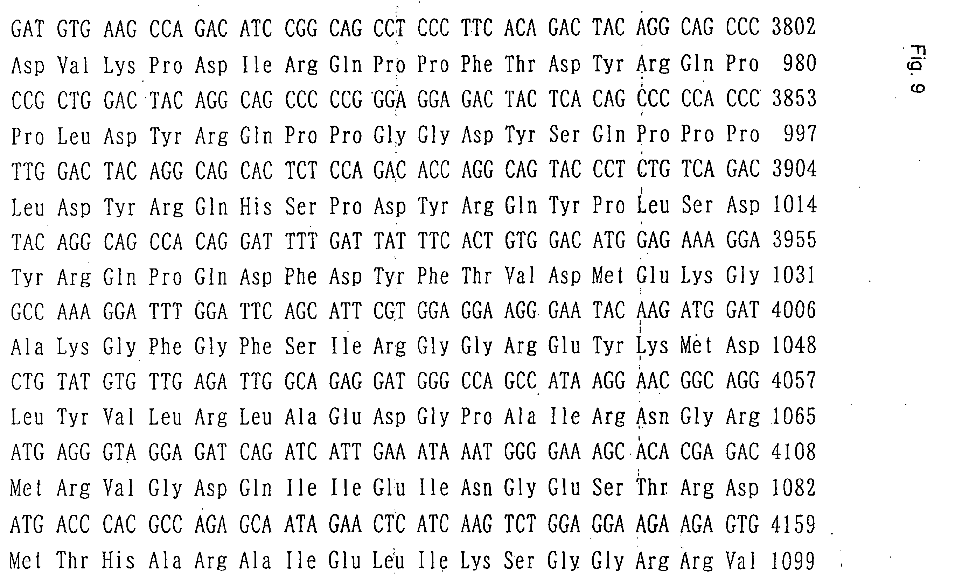

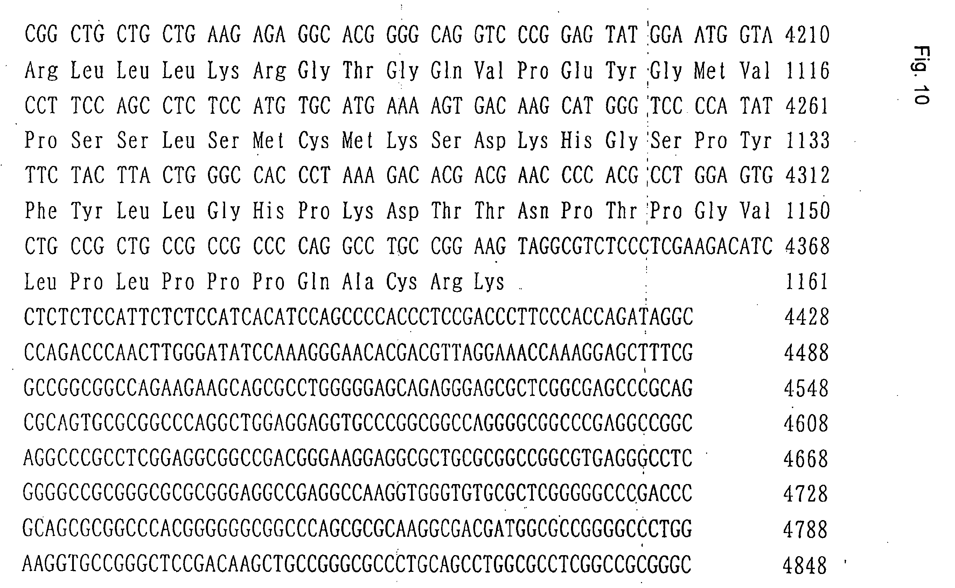

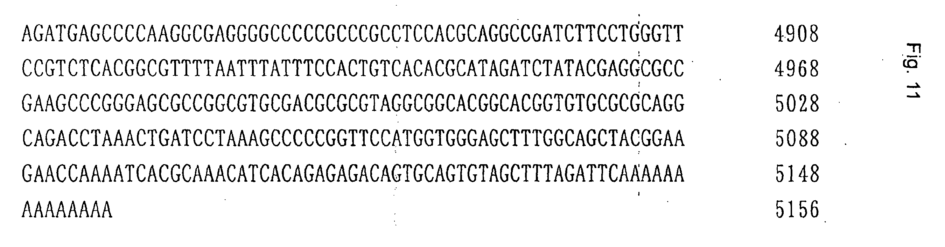

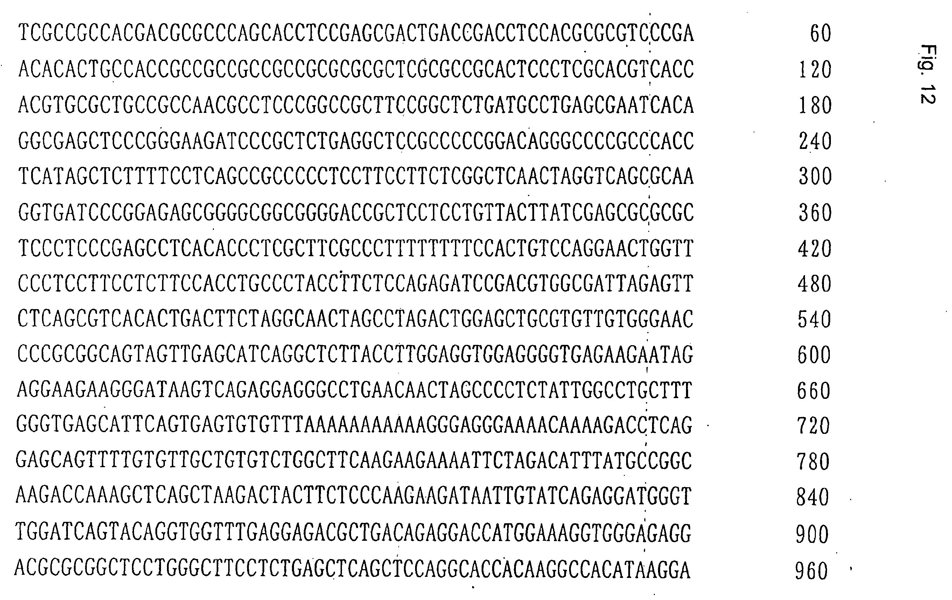

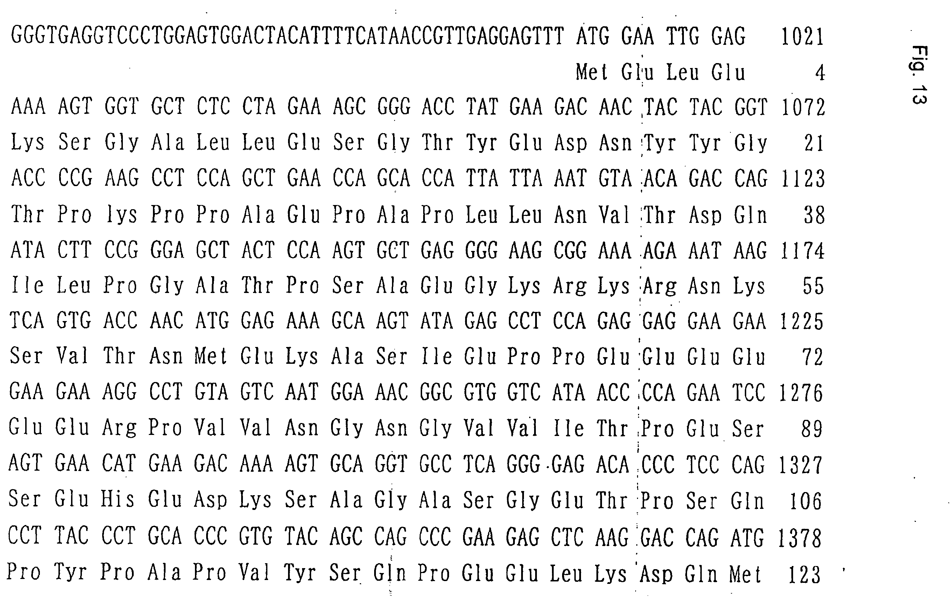

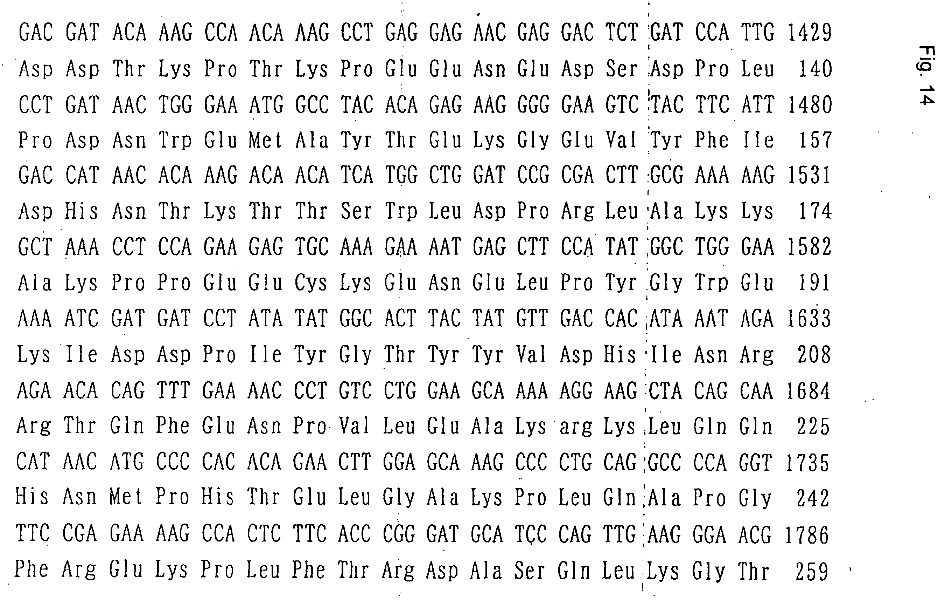

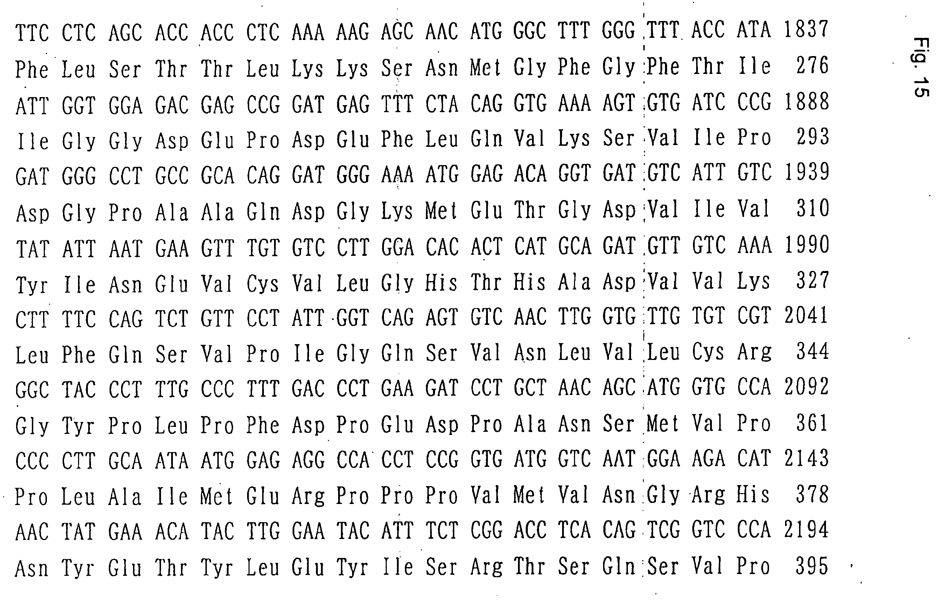

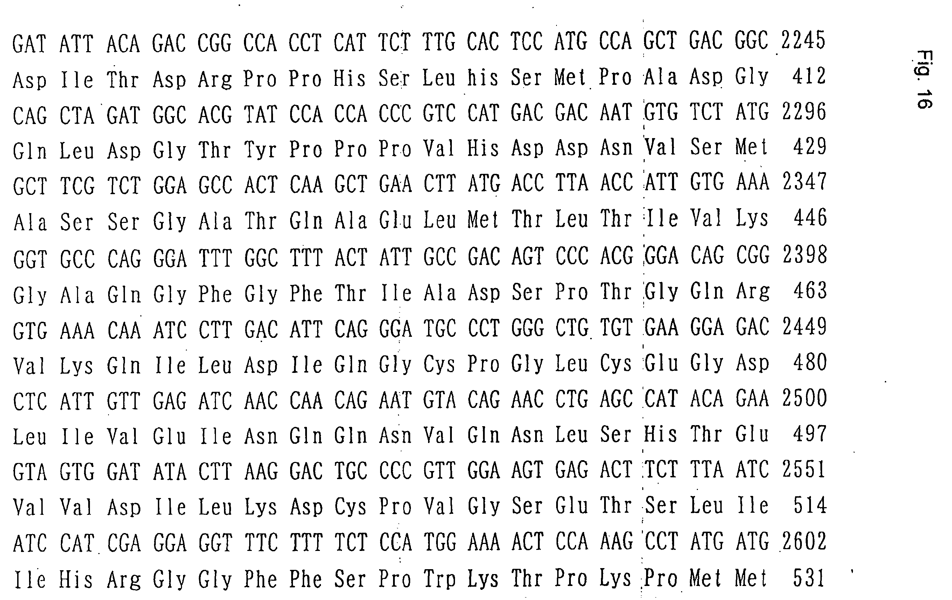

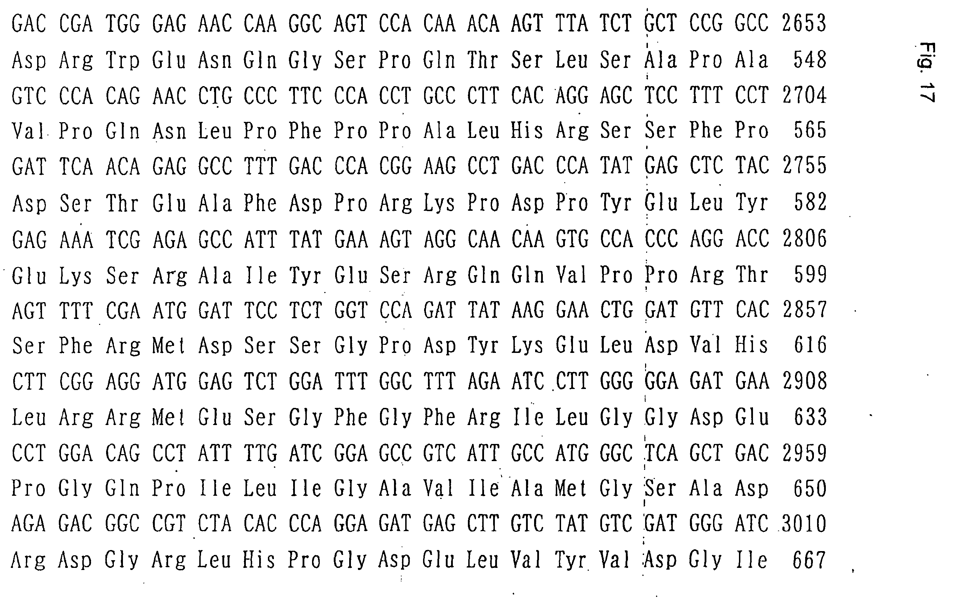

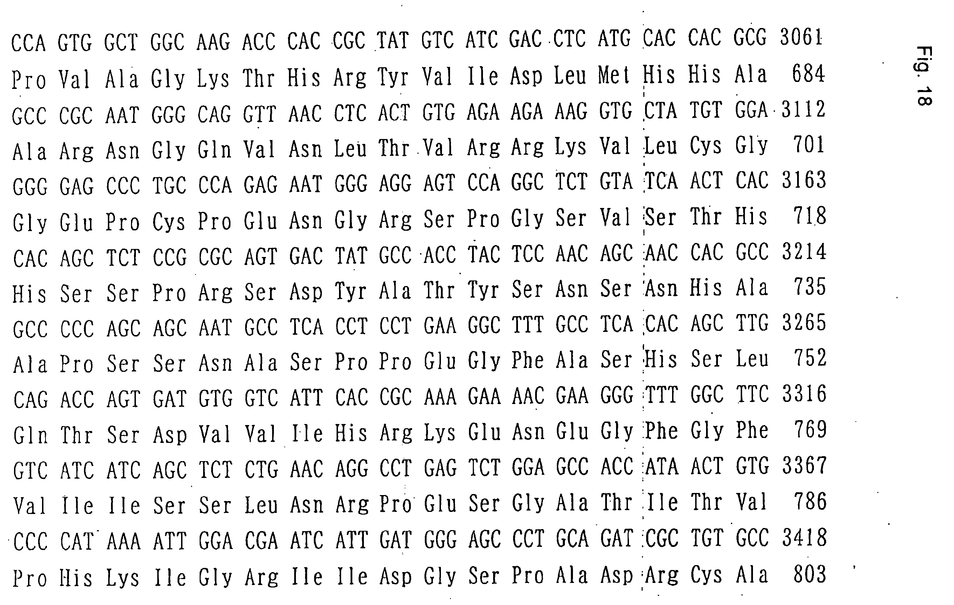

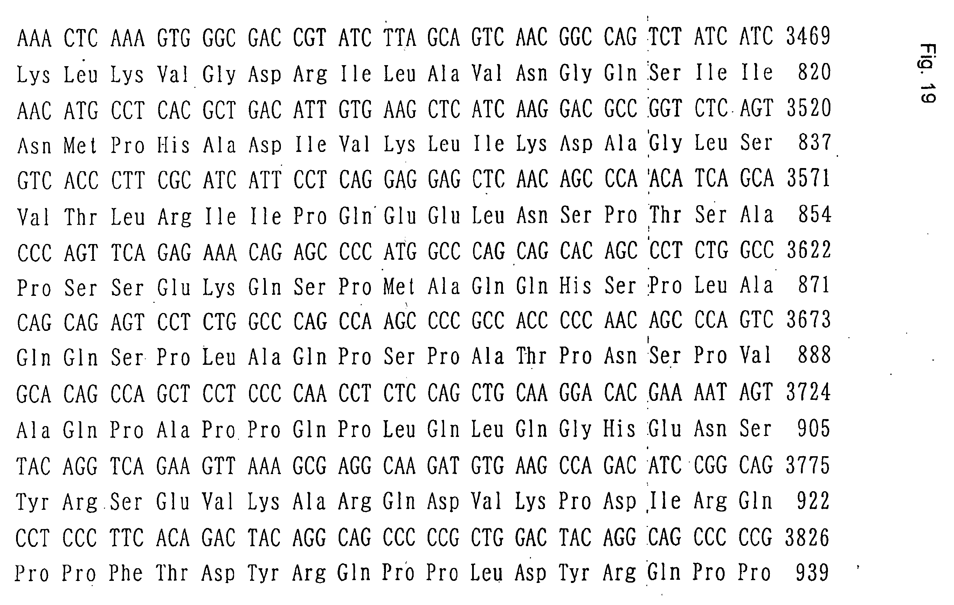

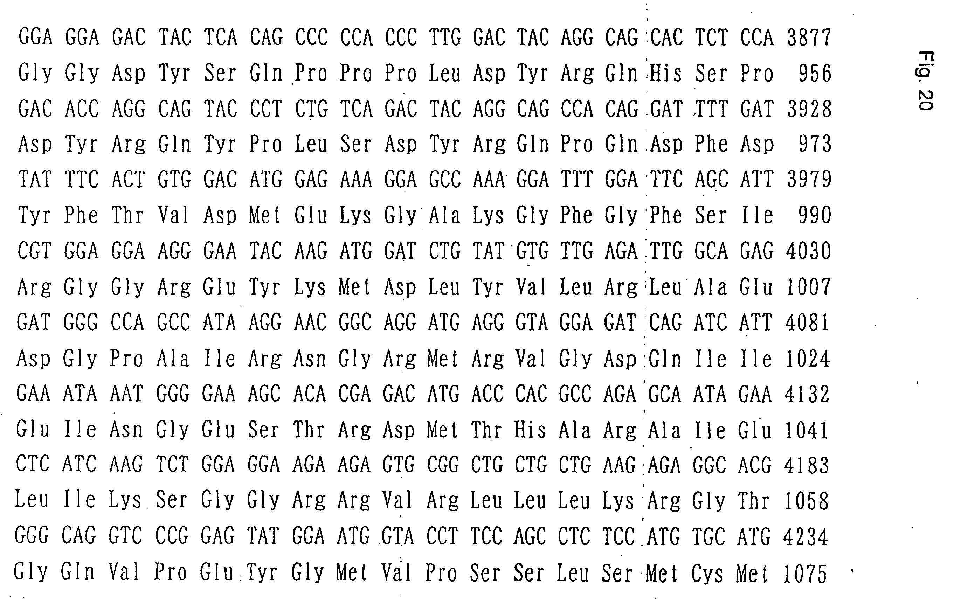

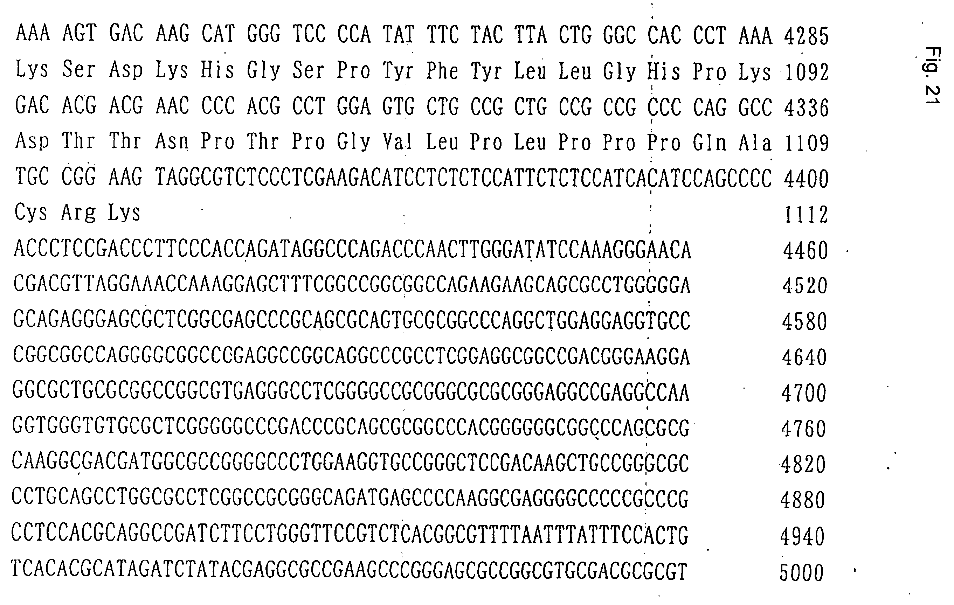

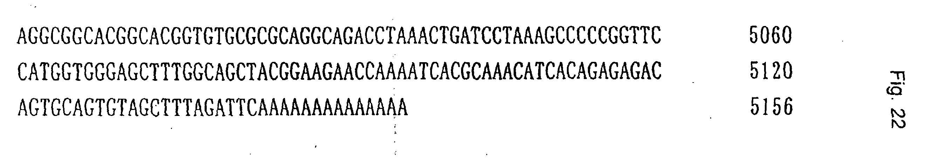

To obtain the full-length cDNA containing a whole coding region of the nucleotide sequence of YN3, the full-length insert in the resulting YN3 was labeled with $^{32}P$ by the random prime method. Using the labeled YN3 was used as the probe, a mouse brain Lambda cDNA library (Lambda ZAPII vector, Stratagene) was screened by conventional plaque hybridization. The full-length insert in the resulting clones was further used as the probe, and the same screening was carried out repeatedly to give those considered as having the maximum full-length insert containing the whole coding region for the protein of the present invention. One of the resulting positive phages was converted into phargimide ((pBluescript SK) (-) vector) according to the method of Stratagene, and its nucleotide sequence was determined. The full-length cDNA (YN3-6) for the protein of the present invention was 5156 bp encoding a polypeptide [Figs. 1-11] consisting of 1161 amino acids represented by SEQ ID NO: 5 or a polypeptide [Figs. 12-22] consisting of 1112 amino acids represented by SEQ ID NO: 6.

Plasmid pBSYN3-6 containing the full-length cDNA (YN3-6) encoding a polypeptide consisting of amino acids represented by SEQ ID NO: 5 or NO: 6 was transformed into E. coli DH5α, to give a transformant (E. coli DH5α/pBSYN3-6).

Example 2 Detection of expression by Northern hybridization using poly(A) $^+$RNA derived from a variety of mouse organs

In the insert of YN3-6, the DNA fragments represented by SEQ ID NOS: 2, 3 and 4 were labeled with digoxigenin by use of a DIG-PCR probe synthesis kit (Boehringer) and used as probes.

Further, the brain, liver, spleen, embryo, kidney, hearts, testicles, ovary and skeletal muscles were excised from Balb/c mice, and total RNA was extracted therefrom with a TRIzol reagent (GIBCO BRL). Thereafter, poly (A) $^+$RNAs were purified by use of PolyAT tract mRNA Isolation System (Promega).

1 μg each of poly(A) $^+$RNAs was subjected to 1 % agarose gel electrophoresis in a formalin-gel method and blotted onto Hybond N (Amersham-Pharmacia) by a vacuum blotting method using a blotting unit (Amersham-Pharmacia). The blotted sample was incubated at 65 °C overnight in a hybridization buffer (5×SSC, 0.1 % N-lauroylsarcosine, 0.02 % SDS, 0.5 % blocking reagent (Boehringer), 100 μg/ml salmon testicle DNA) containing 5 ng/ml of each of the previously prepared probe DNAs. Thereafter, the sample was washed 3 times with 0.1×SSC and 0.1 % SDS at 65 °C for 20 minutes. Further, the sample was maintained in a solution (0.1 M Tris-HCl pH 7.5, 0.15 M NaCl) containing 150 mU/ml alkali phosphatase-labeled anti-digoxigenin antibody (Boehringer), and then washed 3 times with 0.1 M Tris-HCl (pH 7.5), 0.15 M NaCl and 0.1 % Tween 20 for 15 minutes. Finally, the sample was subjected to chemiluminescence using Lumi-Phos 530 (Wako Pure Chemical Industries, Ltd.) as the substrate and detected by exposure onto an X-ray film, and as a result, it was confirmed that a majority of the proteins of the present invention are expressed particularly in the brain [Fig. 23].

Example 3 Interaction between ARIP1 and Smad3

In CHO cells transduced with the full-length Smad3 DNA fused with the GAL4 DNA-binding domain and the protein of the present invention (ARIP1) fused with the VP16-activating domain, the specific interaction of ARP1 with Smad3 was examined in the mammalian two-hybrid system.

A DNA construct for the mammalian two-hybrid screening was prepared as follows. That is, for expression of a fusion protein between the GAL4 DNA-binding domain and Smad3, plasmid pBIND was used, and the full-length cDNA encoding human Smad3 was ligated to the plasmid pBIND to construct pBIND-Smad3. Further, for expression of a fusion protein between the VP16-activating domain and ARIP1, plasmid pACT was used. A cDNA fragment having a partial nucleotide sequence (from the 923- to 4446-positions) of the full-length cDNA encoding ARIP1 constructed by ligating the fragment obtained by PCR to a fragment prepared from plasmid pBS-ARIP1-short having a partial nucleotide sequence (from the 1187- to 4446-positions) of the full-length cDNA encoding ARIP1 was subcloned into pACT, to construct pACT-ARIP1.

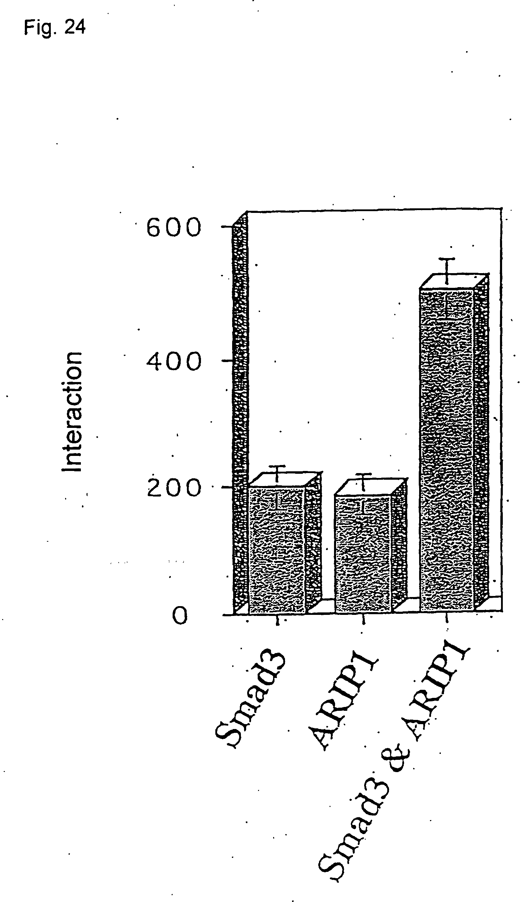

For the mammalian two-hybrid assay, Check Mate Mammalian Two-Hybrid System (Promega) was used as the kit, and the assay was carried out according to its protocol. The plasmids pBIND-Smad3 and pACT-ARIP1 obtained above, cytomegalovirus promoter-derived βgal (CMV-βgal), and reporter plasmid pGSluc inducing a luciferase gene under the control of a GAL4 response promoter were introduced into CHO cells. The strength of the interaction between Smad3 and ARIP1 in the CHO cells was evaluated where the activity of luciferase expressed by the luciferase gene was used as the indicator. The luciferase activity was measured by the same per se known method (Endocrinology, 136, 5493-5503 (1995)) as in measurement of β-galactosidase activity, and as a result, the protein of the present invention (ARIP1), similar to the activin receptor IIA, showed direct interaction with Smad3 in the mammalian cells [Fig. 24].

Industrial Applicability

The protein of the present invention, a partial peptide thereof or a salt thereof (also referred to hereinafter as the protein of the present invention), the DNA encoding the protein of the present invention or a partial peptide thereof (also referred to hereinafter as the DNA of the present invention), the antibody against the protein of the present invention and the antisense DNA can be used as reagents in (1) a method of determining a binding protein to the protein of the present invention, (2) construction of a system for expressing a recombinant protein, (3) development of a binding assay system using said expression system and an assay system using the two-hybrid method and screening of candidates for pharmaceutical compounds, (4) practice of a drug design based on comparison with structurally analogous ligand receptors and (5) formation of probes and PCR primers in gene diagnosis, etc., and further can be used as chemicals in (6) gene therapy, etc. In particular, elucidation of the structure and properties of the protein of the present invention leads to development of unique pharmaceutical preparations acting on these systems.

## Claims

1. A protein which has an amino acid sequence identical to or substantially identical to an amino acid sequence represented by SEQ ID NO: 5, or a salt thereof.

2. A protein which has an amino acid sequence identical to or substantially identical to an amino acid sequence represented by SEQ ID NO: 6, or a salt thereof.

3. The protein according to claim 1 or 2, which has PDZ domains and WW domains and is expressed specifically in the brain and has an ability to bind to activin receptors and/or activin intracellular information transmission molecules.

4. The protein according to claim 3, wherein the activin intracellular information transmission molecule is Smad3.

5. The according to claim 1 or 2, which has 5 PDZ domains and 2 WW domains and is expressed specifically in the brain and has an ability to bind to activin receptors and Smad3.

6. A partial peptide of the protein according to claim 1, a partial peptide of the protein according to claim 2, or a salt thereof.

7. A recombinant DNA which comprises a DNA having a nucleotide sequence encoding the protein according to claim 1 or the protein according to claim 2.

8. The DNA according to claim 7, which has a nucleotide sequence represented by SEQ ID NO: 7, a nucleotide sequence represented by SEQ ID NO: 8 or a nucleotide sequence hybridizing therewith under high stringent conditions.

**9.** A recombinant DNA which comprises a DNA having a nucleotide sequence encoding the partial peptide according to claim 6.

**10.** A recombinant vector which comprises the DNA according to claim 7.

**11.** A transformant comprising the recombinant vector according to claim 10.

**12.** A method for producing the protein according to claim 1, the protein according to claim 2 or a salt thereof, which comprises culturing the transformant according to claim 11 to produce and accumulate the protein according to claim 1 or the protein according to claim 2 is formed and accumulated, and then recovering the product.

**13.** An antibody against the protein according to claim 1, the protein according to claim 2, the partial peptide according to claim 6 or a salt thereof.

**14.** A method for quantifying the protein according to claim 1, the protein according to claim 2, the partial peptide according to claim 6 or a salt thereof, which comprises allowing a test solution containing the protein according to claim 1, the protein according to claim 2, the partial peptide according to claim 6 or a salt thereof and the labeled protein according to claim 1, the labeled protein according to claim 2, the labeled partial peptide according to claim 6 or a labeled salt thereof to react competitively with the antibody according to claim 13.

**15.** A method for determining a binding protein to the protein according to claim 1, the protein according to claim 2, the partial peptide according to claim 6 or a salt thereof, which comprises using the protein according to claim 1, the protein according to claim 2, the partial peptide according to claim 6 or a salt thereof.

**16.** The method according to claim 15, which comprises introducing (1) an expression vector which fuses the protein according to claim 1, the protein according to claim 2 or the partial peptide according to claim 6 with a DNA-binding region of a transcriptional factor and (2) a fusion library between a gene encoding a test protein and a transcription-activating region, into a host cell having a reporter gene maintaining a region binding to the transcriptional factor on a promoter, and measuring a change in the amount of the expressed reporter gene which is increased by a binding of the protein according to claim 1, the protein according to claim 2 or the partial peptide according to claim 6 to the test compound.

**17.** A protein or a salt thereof which binds to the protein according to claim 1, the protein according to claim 2, the partial peptide according to claim 6 or a salt thereof, which is obtained by the method according to claim 15.

**18.** A method for screening a compound or a salt thereof inhibiting or promoting a binding of the protein according to claim 1, the protein according to claim 2, the partial peptide according to claim 6 or a salt thereof to the protein according to claim 17 or a salt thereof, activin receptors or activin intracellular information transmission molecules, which comprises using the protein according to claim 1, the protein according to claim 2, the partial peptide according to claim 6 or a salt thereof.

**19.** A method for screening a compound or a salt thereof inhibiting or promoting a binding of the protein according to claim 1, the protein according to claim 2, the partial peptide according to claim 6 or a salt thereof to activin receptors or activin intracellular information transmission molecules, which comprises comparing the case where the labeled protein according to claim 1, the labeled protein according to claim 2, the labeled partial peptide according to claim 6 or a labeled salt thereof is contacted with activin receptors or activin intracellular information transmission molecules, with the case where the labeled protein according to claim 1, the labeled protein according to claim 2, the labeled partial peptide according to claim 6 or a labeled salt thereof and a test compound are contacted with activin receptors or activin intracellular information transmission molecules, by measuring the amount of the labeled protein according to claim 1, the labeled protein according to claim 2, the labeled partial peptide according to claim 6 or a labeled salt thereof bound to the activin receptors or activin intracellular information transmission molecules in both the cases.

**20.** A method for screening a compound or a salt thereof inhibiting or promoting a binding of the protein according to claim 1, the protein according to claim 2, the partial peptide according to claim 6 or a salt thereof to the protein according to claim 17 or a salt thereof, which comprises comparing the case where the labeled protein according to claim 1, the labeled protein according to claim 2, the labeled partial peptide according to claim 6 or a labeled salt thereof is contacted with the protein according to claim 17 or a salt thereof, with the case where the labeled protein according to claim 1, the labeled protein according to claim 2 or a labeled salt thereof and a test compound are contacted with the protein according to claim 17 or a salt thereof, by measuring the amount of the labeled protein according to claim 1, the labeled protein according to claim 2, the labeled partial peptide according to claim 6 or a labeled salt thereof bound to the protein according to claim 17 or a salt thereof in both the cases.

**21.** A method for screening a compound or a salt thereof inhibiting or promoting a binding of the protein according to claim 1, the protein according to claim 2, the partial peptide according to claim 6 or a salt thereof to the protein according to claim 17 or a salt thereof, activin receptors or activin intracellular information transmission molecules, which comprises comparing the case where the protein according to claim 1, the protein according to claim 2, the partial peptide according to claim 6 or a salt thereof is introduced into cells expressing the protein according to claim 17 or a salt thereof, activin receptors or activin intracellular information transmission molecules, with the case where the protein according to claim 1, the protein according to claim 2, the partial peptide according to claim 6 or a salt thereof and a test compound are introduced into cells expressing the protein according to claim 17 or a salt thereof, activin receptors or activin intracellular information transmission molecules, by measuring the amount of the protein according to claim 1, the protein according to claim 2, the partial peptide according to claim 6 or a salt thereof bound to the protein according to claim 17 or a salt thereof, activin receptors or activin intracellular information transmission molecules in the cells in both the cases.

**22.** A method for screening a compound or a salt thereof inhibiting or promoting a binding of the labeled protein according to claim 1, the labeled protein according to claim 2, the labeled partial peptide according to claim 6 or a labeled salt thereof to the protein according to claim 17 or a salt thereof, activin receptors or activin intracellular information transmission molecules, which comprises comparing the case where the labeled protein according to claim 1, the labeled protein according to claim 2, the labeled partial peptide according to claim 6 or a labeled salt thereof is contacted with a membrane fraction of cells expressing the protein according to claim 17 or a salt thereof, activin receptors or activin intracellular information transmission molecules, with the case where the labeled protein according to claim 1, the labeled protein according to claim. 2, the labeled partial peptide according to claim 6 or a labeled salt thereof and a test compound are contacted with a membrane fraction of cells expressing the protein according to claim 17 or a salt thereof, activin receptors or activin intracellular information transmission molecules, by measuring the amount of the protein according to claim 1, the protein according to claim 2, the partial peptide according to claim 6 or a salt thereof bound to the membrane fraction of the cells in both the cases.

**23.** A method for screening a compound or a salt thereof inhibiting or promoting a binding of the protein according to claim 1, the protein according to claim 2, the partial peptide according to claim 6 or a salt thereof to the protein according to claim 17 or a salt thereof, activin receptors or activin intracellular information transmission molecules, which comprises comparing the case where the protein according to claim 1, the protein according to claim 2, or a salt thereof is introduced into cells expressing the protein according to claim 17 or a salt thereof, activin receptors or activin intracellular information transmission molecules, with the case where the protein according to claim 1, the protein according to claim 2, the partial peptide according to claim 6 or a salt thereof and a test compound are introduced into cells expressing the protein according to claim 17 or a salt thereof, activin receptors or activin intracellular information transmission molecules, by measuring the cell-stimulating activity via the protein according to claim 17 or a salt thereof, activin receptors or activin intracellular information transmission molecules in both the cases.

**24.** The method for determining a protein according to claim 16 or the screening method according to any one of claims 18 to 23, which comprises using the two-hybrid method.

**25.** A kit for screening a compound or a salt thereof inhibiting or promoting a binding of the protein according to claim 1, the protein according to claim 2, the partial peptide according to claim 6 or a salt thereof to the protein according to claim 17 or a salt thereof, activin receptors or activin intracellular information transmission molecules, which comprises the protein according to claim 1, the protein according to claim 2, the partial peptide according to claim 6 or a salt thereof.

**26.** A compound or a salt thereof which inhibits or promotes a binding of the protein according to claim 1, the protein according to claim 2, the partial peptide according to claim 6 or a salt thereof to the protein according to claim 17 or a salt thereof, activin receptors or activin intracellular information transmission molecules, which is obtained by the screening method according to any one of claims 18 to 23 or by the screening kit according to claim 25.

**27.** A pharmaceutical composition comprising the protein according to claim 17, the compound according to claim 26 or a salt thereof.

**28.** The pharmaceutical composition according to claim 27, which is an agent for preventing or treating Alzheimer's disease, Parkinson's disease, epilepsy or Huntington's chorea.

**29.** A method for preventing and treating abnormalities in nerve cells or cerebral diseases correlated with the protein according to claim 17 or a salt thereof, activin receptors or activin intracellular information transmission molecules, which administering an effective amount of the protein according to claim 17, the compound according to claim 26 or a salt thereof to mammals.

**30.** Use of the protein according to claim 17, the compound according to claim 26 or a salt thereof for preparing an agent for preventing and treating abnormalities in nerve cells or cerebral diseases correlated with the protein according to claim 17 or a salt thereof, activin receptors or activin intracellular information transmission molecules.

Fig. 1

```
TCGCCGCCACGACGCGCCCAGCACCTCCGAGCGACTGACCGACCTCCACGCGCGTCCCGA     60
ACACACTGCCACCGCCGCCGCCGCCGCGCGCGCTCGCGCCGCACTCCCTCGCACGTCACC    120
ACGTGCGCTGCCGCCAACGCCTCCCGGCCGCTTCCGGCTCTGATGCCTGAGCGAATCACA    180
GGCGAGCTCCCGGGAAGATCCCGCTCTGAGGCTCCGCCCCCGGACAGGGCCCCGCCCACC    240
TCATAGCTCTTTTCCTCAGCCGCCCCCTCCTTCCTTCTCGGCTCAACTAGGTCAGCGCAA    300
GGTGATCCCGGAGAGCGGGGCGGCGGGGACCGCTCCTCCTGTTACTTATCGAGCGCGCGC    360
TCCCTCCCGAGCCTCACACCCTCGCTTCGCCCTTTTTTTTCCACTGTCCAGGAACTGGTT    420
CCCTCCTTCCTCTTCCACCTGCCCTACCTTCTCCAGAGATCCGACGTGGCGATTAGAGTT    480
CTCAGCGTCACACTGACTTCTAGGCAACTAGCCTAGACTGGAGCTGCGTGTTGTGGGAAC    540
CCCGCGGCAGTAGTTGAGCATCAGGCTCTTACCTTGGAGGTGGAGGGGTGAGAAGAATAG    600
AGGAAGAAGGGATAAGTCAGAGGAGGGCCTGAACAACTAGCCCCTCTATTGGCCTGCTTT    660
GGGTGAGCATTCAGTGAGTGTGTTTAAAAAAAAAAAGGGAGGGAAAACAAAAGACCTCAG    720
GAGCAGTTTTGTGTTGCTGTGTCTGGCTTCAAGAAGAAAATTCTAGACATTTATGCCGGC    780
AAGACCAAAGCTCAGCTAAGACTACTTCTCCCAAGAAGATAATTGTATCAGAGGATGGGT    840
TGGATCAGTACAGGTGGTTTGA GGA GAC GCT GAC AGA GGA CCA TGG AAA GGT GGG    895
                       Gly Asp Ala Asp Arg Gly Pro Trp Lys Gly Gly     11
```

Fig. 2

```
AGA GGA CGC GCG GCT CCT GGG CTT CCT CTG AGC TCA GCT CCA GGC ACC ACA  946
Arg Gly Arg Ala Ala Pro Gly Leu Pro Leu Ser Ser Ala Pro Gly Thr Thr   28
AGG CCA CAT AAG GAG GGT GAG GTC CCT GGA GTG GAC TAC ATT TTC ATA ACC  997
Arg Pro His Lys Glu Gly Glu Val Pro Gly Val Asp Tyr Ile Phe Ile Thr   45
GTT GAG GAG TTT ATG GAA TTG GAG AAA AGT GGT GCT CTC CTA GAA AGC GGG 1048
Val Glu Glu Phe Met Glu Leu Glu Lys Ser Gly Ala Leu Leu Glu Ser Gly   62
ACC TAT GAA GAC AAC TAC TAC GGT ACC CCG AAG CCT CCA GCT GAA CCA GCA 1099
Thr Tyr Glu Asp Asn Tyr Tyr Gly Thr Pro lys Pro Pro Ala Glu Pro Ala   79
CCA TTA TTA AAT GTA ACA GAC CAG ATA CTT CCG GGA GCT ACT CCA AGT GCT 1150
Pro Leu Leu Asn Val Thr Asp Gln Ile Leu Pro Gly Ala Thr Pro Ser Ala   96
GAG GGG AAG CGG AAA AGA AAT AAG TCA GTG ACC AAC ATG GAG AAA GCA AGT 1201
Glu Gly Lys Arg Lys Arg Asn Lys Ser Val Thr Asn Met Glu Lys Ala Ser  113
ATA GAG CCT CCA GAG GAG GAA GAA GAA GAA AGG CCT GTA GTC AAT GGA AAC 1252
Ile Glu Pro Pro Glu Glu Glu Glu Glu Glu Arg Pro Val Val Asn Gly Asn  130
GGC GTG GTC ATA ACC CCA GAA TCC AGT GAA CAT GAA GAC AAA AGT GCA GGT 1303
Gly Val Val Ile Thr Pro Glu Ser Ser Glu His Glu Asp Lys Ser Ala Gly  147
```

Fig. 3

```
GCC TCA GGG GAG ACA CCC TCC CAG CCT TAC CCT GCA CCC GTG TAC AGC CAG 1354
Ala Ser Gly Glu Thr Pro Ser Gln Pro Tyr Pro Ala Pro Val Tyr Ser Gln  164
CCC GAA GAG CTC AAG GAC CAG ATG GAC GAT ACA AAG CCA ACA AAG CCT GAG 1405
Pro Glu Glu Leu Lys Asp Gln Met Asp Asp Thr Lys Pro Thr Lys Pro Glu  181
GAG AAC GAG GAC TCT GAT CCA TTG CCT GAT AAC TGG GAA ATG GCC TAC ACA 1456
Glu Asn Glu Asp Ser Asp Pro Leu Pro Asp Asn Trp Glu Met Ala Tyr Thr  198
GAG AAG GGG GAA GTC TAC TTC ATT GAC CAT AAC ACA AAG ACA ACA TCA TGG 1507
Glu Lys Gly Glu Val Tyr Phe Ile Asp His Asn Thr Lys Thr Thr Ser Trp  215
CTG GAT CCG CGA CTT GCG AAA AAG GCT AAA CCT CCA GAA GAG TGC AAA GAA 1558
Leu Asp Pro Arg Leu Ala Lys Lys Ala Lys Pro Pro Glu Glu Cys Lys Glu  232
AAT GAG CTT CCA TAT GGC TGG GAA AAA ATC GAT GAT CCT ATA TAT GGC ACT 1609
Asn Glu Leu Pro Tyr Gly Trp Glu Lys Ile Asp Asp Pro Ile Tyr Gly Thr  249
TAC TAT GTT GAC CAC ATA AAT AGA AGA ACA CAG TTT GAA AAC CCT GTC CTG 1660
Tyr Tyr Val Asp His Ile Asn Arg Arg Thr Gln Phe Glu Asn Pro Val Leu  266
GAA GCA AAA AGG AAG CTA CAG CAA CAT AAC ATG CCC CAC ACA GAA CTT GGA 1711
Glu Ala Lys arg Lys Leu Gln Gln His Asn Met Pro His Thr Glu Leu Gly  283
```

Fig. 4

```
GCA AAG CCC CTG CAG GCC CCA GGT TTC CGA GAA AAG CCA CTC TTC ACC CGG 1762
Ala Lys Pro Leu Gln Ala Pro Gly Phe Arg Glu Lys Pro Leu Phe Thr Arg  300
GAT GCA TCC CAG TTG AAG GGA ACG TTC CTC AGC ACC ACC CTC AAA AAG AGC 1813
Asp Ala Ser Gln Leu Lys Gly Thr Phe Leu Ser Thr Thr Leu Lys Lys Ser  317
AAC ATG GGC TTT GGG TTT ACC ATA ATT GGT GGA GAC GAG CCG GAT GAG TTT 1864
Asn Met Gly Phe Gly Phe Thr Ile Ile Gly Gly Asp Glu Pro Asp Glu Phe  334
CTA CAG GTG AAA AGT GTG ATC CCG GAT GGG CCT GCC GCA CAG GAT GGG AAA 1915
Leu Gln Val Lys Ser Val Ile Pro Asp Gly Pro Ala Ala Gln Asp Gly Lys  351
ATG GAG ACA GGT GAT GTC ATT GTC TAT ATT AAT GAA GTT TGT GTC CTT GGA 1966
Met Glu Thr Gly Asp Val Ile Val Tyr Ile Asn Glu Val Cys Val Leu Gly  368
CAC ACT CAT GCA GAT GTT GTC AAA CTT TTC CAG TCT GTT CCT ATT GGT CAG 2017
His Thr His Ala Asp Val Val Lys Leu Phe Gln Ser Val Pro Ile Gly Gln  385
AGT GTC AAC TTG GTG TTG TGT CGT GGC TAC CCT TTG CCC TTT GAC CCT GAA 2068
Ser Val Asn Leu Val Leu Cys Arg Gly Tyr Pro Leu Pro Phe Asp Pro Glu  402
GAT CCT GCT AAC AGC ATG GTG CCA CCC CTT GCA ATA ATG GAG AGG CCA CCT 2119
Asp Pro Ala Asn Ser Met Val Pro Pro Leu Ala Ile Met Glu Arg Pro Pro  419
```

Fig. 5

```
CCG GTG ATG GTC AAT GGA AGA CAT AAC TAT GAA ACA TAC TTG GAA TAC ATT 2170
Pro Val Met Val Asn Gly Arg His Asn Tyr Glu Thr Tyr Leu Glu Tyr Ile  436
TCT CGG ACC TCA CAG TCG GTC CCA GAT ATT ACA GAC CGG CCA CCT CAT TCT 2221
Ser Arg Thr Ser Gln Ser Val Pro Asp Ile Thr Asp Arg Pro Pro His Ser  453
TTG CAC TCC ATG CCA GCT GAC GGC CAG CTA GAT GGC ACG TAT CCA CCA CCC 2272
Leu his Ser Met Pro Ala Asp Gly Gln Leu Asp Gly Thr Tyr Pro Pro Pro  470
GTC CAT GAC GAC AAT GTG TCT ATG GCT TCG TCT GGA GCC ACT CAA GCT GAA 2323
Val His Asp Asp Asn Val Ser Met Ala Ser Ser Gly Ala Thr Gln Ala Glu  487
CTT ATG ACC TTA ACC ATT GTG AAA GGT GCC CAG GGA TTT GGC TTT ACT ATT 2374
Leu Met Thr Leu Thr Ile Val Lys Gly Ala Gln Gly Phe Gly Phe Thr Ile  504
GCC GAC AGT CCC ACG GGA CAG CGG GTG AAA CAA ATC CTT GAC ATT CAG GGA 2425
Ala Asp Ser Pro Thr Gly Gln Arg Val Lys Gln Ile Leu Asp Ile Gln Gly  521
TGC CCT GGG CTG TGT GAA GGA GAC CTC ATT GTT GAG ATC AAC CAA CAG AAT 2476
Cys Pro Gly Leu Cys Glu Gly Asp Leu Ile Val Glu Ile Asn Gln Gln Asn  538
GTA CAG AAC CTG AGC CAT ACA GAA GTA GTG GAT ATA CTT AAG GAC TGC CCC 2527
Val Gln Asn Leu Ser His Thr Glu Val Val Asp Ile Leu Lys Asp Cys Pro  555
```

Fig. 6

```
GTT GGA AGT GAG ACT TCT TTA ATC ATC CAT CGA GGA GGT TTC TTT TCT CCA 2578
Val Gly Ser Glu Thr Ser Leu Ile Ile His Arg Gly Gly Phe Phe Ser Pro  572
TGG AAA ACT CCA AAG CCT ATG ATG GAC CGA TGG GAG AAC CAA GGC AGT CCA 2629
Trp Lys Thr Pro Lys Pro Met Met Asp Arg Trp Glu Asn Gln Gly Ser Pro  589
CAA ACA AGT TTA TCT GCT CCG GCC GTC CCA CAG AAC CTG CCC TTC CCA CCT 2680
Gln Thr Ser Leu Ser Ala Pro Ala Val Pro Gln Asn Leu Pro Phe Pro Pro  606
GCC CTT CAC AGG AGC TCC TTT CCT GAT TCA ACA GAG GCC TTT GAC CCA CGG 2731
Ala Leu His Arg Ser Ser Phe Pro Asp Ser Thr Glu Ala Phe Asp Pro Arg  623
AAG CCT GAC CCA TAT GAG CTC TAC GAG AAA TCG AGA GCC ATT TAT GAA AGT 2782
Lys Pro Asp Pro Tyr Glu Leu Tyr Glu Lys Ser Arg Ala Ile Tyr Glu Ser  640
AGG CAA CAA GTG CCA CCC AGG ACC AGT TTT CGA ATG GAT TCC TCT GGT CCA 2833
Arg Gln Gln Val Pro Pro Arg Thr Ser Phe Arg Met Asp Ser Ser Gly Pro  657
GAT TAT AAG GAA CTG GAT GTT CAC CTT CGG AGG ATG GAG TCT GGA TTT GGC 2884
Asp Tyr Lys Glu Leu Asp Val His Leu Arg Arg Met Glu Ser Gly Phe Gly  674
TTT AGA ATC CTT GGG GGA GAT GAA CCT GGA CAG CCT ATT TTG ATC GGA GCC 2935
Phe Arg Ile Leu Gly Gly Asp Glu Pro Gly Gln Pro Ile Leu Ile Gly Ala  691
```

Fig. 7

```
GTC ATT GCC ATG GGC TCA GCT GAC AGA GAC GGC CGT CTA CAC CCA GGA GAT 2986
Val Ile Ala Met Gly Ser Ala Asp Arg Asp Gly Arg Leu His Pro Gly Asp  708
GAG CTT GTC TAT GTC GAT GGG ATC CCA GTG GCT GGC AAG ACC CAC CGC TAT 3037
Glu Leu Val Tyr Val Asp Gly Ile Pro Val Ala Gly Lys Thr His Arg Tyr  725
GTC ATC GAC CTC ATG CAC CAC GCG GCC CGC AAT GGG CAG GTT AAC CTC ACT 3088
Val Ile Asp Leu Met His His Ala Ala Arg Asn Gly Gln Val Asn Leu Thr  742
GTG AGA AGA AAG GTG CTA TGT GGA GGG GAG CCC TGC CCA GAG AAT GGG AGG 3139
Val Arg Arg Lys Val Leu Cys Gly Gly Glu Pro Cys Pro Glu Asn Gly Arg  759
AGT CCA GGC TCT GTA TCA ACT CAC CAC AGC TCT CCG CGC AGT GAC TAT GCC 3190
Ser Pro Gly Ser Val Ser Thr His His Ser Ser Pro Arg Ser Asp Tyr Ala  776
ACC TAC TCC AAC AGC AAC CAC GCC GCC CCC AGC AGC AAT GCC TCA CCT CCT 3241
Thr Tyr Ser Asn Ser Asn His Ala Ala Pro Ser Ser Asn Ala Ser Pro Pro  793
GAA GGC TTT GCC TCA CAC AGC TTG CAG ACC AGT GAT GTG GTC ATT CAC CGC 3292
Glu Gly Phe Ala Ser His Ser Leu Gln Thr Ser Asp Val Val Ile His Arg  810
AAA GAA AAC GAA GGG TTT GGC TTC GTC ATC ATC AGC TCT CTG AAC AGG CCT 3343
Lys Glu Asn Glu Gly Phe Gly Phe Val Ile Ile Ser Ser Leu Asn Arg Pro  827
```

Fig. 8

```
GAG TCT GGA GCC ACC ATA ACT GTG CCC CAT AAA ATT GGA CGA ATC ATT GAT 3394
Glu Ser Gly Ala Thr Ile Thr Val Pro His Lys Ile Gly Arg Ile Ile Asp  844
GGG AGC CCT GCA GAT CGC TGT GCC AAA CTC AAA GTG GGC GAC CGT ATC TTA 3445
Gly Ser Pro Ala Asp Arg Cys Ala Lys Leu Lys Val Gly Asp Arg Ile Leu  861
GCA GTC AAC GGC CAG TCT ATC ATC AAC ATG CCT CAC GCT GAC ATT GTG AAG 3496
Ala Val Asn Gly Gln Ser Ile Ile Asn Met Pro His Ala Asp Ile Val Lys  878
CTC ATC AAG GAC GCC GGT CTC AGT GTC ACC CTT CGC ATC ATT CCT CAG GAG 3547
Leu Ile Lys Asp Ala Gly Leu Ser Val Thr Leu Arg Ile Ile Pro Gln Glu  895
GAG CTC AAC AGC CCA ACA TCA GCA CCC AGT TCA GAG AAA CAG AGC CCC ATG 3598
Glu Leu Asn Ser Pro Thr Ser Ala Pro Ser Ser Glu Lys Gln Ser Pro Met  912
GCC CAG CAG CAC AGC CCT CTG GCC CAG CAG AGT CCT CTG GCC CAG CCA AGC 3649
Ala Gln Gln His Ser Pro Leu Ala Gln Gln Ser Pro Leu Ala Gln Pro Ser  929
CCC GCC ACC CCC AAC AGC CCA GTC GCA CAG CCA GCT CCT CCC CAA CCT CTC 3700
Pro Ala Thr Pro Asn Ser Pro Val Ala Gln Pro Ala Pro Pro Gln Pro Leu  946
CAG CTG CAA GGA CAC GAA AAT AGT TAC AGG TCA GAA GTT AAA GCG AGG CAA 3751
Gln Leu Gln Gly His Glu Asn Ser Tyr Arg Ser Glu Val Lys Ala Arg Gln  963
```

Fig. 9

GAT GTG AAG CCA GAC ATC CGG CAG CCT CCC TTC ACA GAC TAC AGG CAG CCC 3802
Asp Val Lys Pro Asp Ile Arg Gln Pro Pro Phe Thr Asp Tyr Arg Gln Pro 980
CCG CTG GAC TAC AGG CAG CCC CCG GGA GGA GAC TAC TCA CAG CCC CCA CCC 3853
Pro Leu Asp Tyr Arg Gln Pro Pro Gly Gly Asp Tyr Ser Gln Pro Pro Pro 997
TTG GAC TAC AGG CAG CAC TCT CCA GAC ACC AGG CAG TAC CCT CTG TCA GAC 3904
Leu Asp Tyr Arg Gln His Ser Pro Asp Tyr Arg Gln Tyr Pro Leu Ser Asp 1014
TAC AGG CAG CCA CAG GAT TTT GAT TAT TTC ACT GTG GAC ATG GAG AAA GGA 3955
Tyr Arg Gln Pro Gln Asp Phe Asp Tyr Phe Thr Val Asp Met Glu Lys Gly 1031
GCC AAA GGA TTT GGA TTC AGC ATT CGT GGA GGA AGG GAA TAC AAG ATG GAT 4006
Ala Lys Gly Phe Gly Phe Ser Ile Arg Gly Gly Arg Glu Tyr Lys Met Asp 1048
CTG TAT GTG TTG AGA TTG GCA GAG GAT GGG CCA GCC ATA AGG AAC GGC AGG 4057
Leu Tyr Val Leu Arg Leu Ala Glu Asp Gly Pro Ala Ile Arg Asn Gly Arg 1065
ATG AGG GTA GGA GAT CAG ATC ATT GAA ATA AAT GGG GAA AGC ACA CGA GAC 4108
Met Arg Val Gly Asp Gln Ile Ile Glu Ile Asn Gly Glu Ser Thr Arg Asp 1082
ATG ACC CAC GCC AGA GCA ATA GAA CTC ATC AAG TCT GGA GGA AGA AGA GTG 4159
Met Thr His Ala Arg Ala Ile Glu Leu Ile Lys Ser Gly Gly Arg Arg Val 1099

Fig. 10

```
CGG CTG CTG CTG AAG AGA GGC ACG GGG CAG GTC CCG GAG TAT GGA ATG GTA 4210
Arg Leu Leu Leu Lys Arg Gly Thr Gly Gln Val Pro Glu Tyr Gly Met Val 1116
CCT TCC AGC CTC TCC ATG TGC ATG AAA AGT GAC AAG CAT GGG TCC CCA TAT 4261
Pro Ser Ser Leu Ser Met Cys Met Lys Ser Asp Lys His Gly Ser Pro Tyr 1133
TTC TAC TTA CTG GGC CAC CCT AAA GAC ACG ACG AAC CCC ACG CCT GGA GTG 4312
Phe Tyr Leu Leu Gly His Pro Lys Asp Thr Thr Asn Pro Thr Pro Gly Val 1150
CTG CCG CTG CCG CCG CCC CAG GCC TGC CGG AAG TAGGCGTCTCCCTCGAAGACATC 4368
Leu Pro Leu Pro Pro Pro Gln Ala Cys Arg Lys                         1161
CTCTCTCCATTCTCTCCATCACATCCAGCCCCACCCTCCGACCCTTCCCACCAGATAGGC        4428
CCAGACCCAACTTGGGATATCCAAAGGGAACACGACGTTAGGAAACCAAAGGAGCTTTCG        4488
GCCGGCGGCCAGAAGAAGCAGCGCCTGGGGAGCAGAGGGAGCGCTCGGCGAGCCCGCAG         4548
CGCAGTGCGCGGCCCAGGCTGGAGGAGGTGCCCGGCGGCCAGGGGCGGCCCGAGGCCGGC        4608
AGGCCCGCCTCGGAGGCGGCCGACGGGAAGGAGGCGCTGCGCGGCCGGCGTGAGGGCCTC        4668
GGGGCCGCGGGCGCGCGGGAGGCCGAGGCCAAGGTGGGTGTGCGCTCGGGGGCCCGACCC        4728
GCAGCGCGGCCCACGGGGGGCGGCCCAGCGCGCAAGGCGACGATGGCGCCGGGGCCCTGG        4788
AAGGTGCCGGGCTCCGACAAGCTGCCGGGCGCCCTGCAGCCTGGCGCCTCGGCCGCGGGC        4848
```

Fig. 11

```
AGATGAGCCCCAAGGCGAGGGGCCCCCGCCCGCCTCCACGCAGGCCGATCTTCCTGGGTT    4908
CCGTCTCACGGCGTTTTAATTTATTTCCACTGTCACACGCATAGATCTATACGAGGCGCC    4968
GAAGCCCGGGAGCGCCGGCGTGCGACGCGCGTAGGCGGCACGGCACGGTGTGCGCGCAGG    5028
CAGACCTAAACTGATCCTAAAGCCCCCGGTTCCATGGTGGGAGCTTTGGCAGCTACGGAA    5088
GAACCAAAATCACGCAAACATCACAGAGAGACAGTGCAGTGTAGCTTTAGATTCAAAAAA    5148
AAAAAAAA                                                        5156
```

Fig. 12

```
TCGCCGCCACGACGCGCCCAGCACCTCCGAGCGACTGACCGACCTCCACGCGCGTCCCGA    60
ACACACTGCCACCGCCGCCGCCGCCGCGCGCGCTCGCGCCGCACTCCCTCGCACGTCACC   120
ACGTGCGCTGCCGCCAACGCCTCCCGGCCGCTTCCGGCTCTGATGCCTGAGCGAATCACA   180
GGCGAGCTCCCGGGAAGATCCCGCTCTGAGGCTCCGCCCCCGGACAGGGCCCCGCCCACC   240
TCATAGCTCTTTTCCTCAGCCGCCCCCTCCTTCCTTCTCGGCTCAACTAGGTCAGCGCAA   300
GGTGATCCCGGAGAGCGGGGCGGCGGGGACCGCTCCTCCTGTTACTTATCGAGCGCGCGC   360
TCCCTCCCGAGCCTCACACCCTCGCTTCGCCCTTTTTTTTCCACTGTCCAGGAACTGGTT   420
CCCTCCTTCCTCTTCCACCTGCCCTACCTTCTCCAGAGATCCGACGTGGCGATTAGAGTT   480
CTCAGCGTCACACTGACTTCTAGGCAACTAGCCTAGACTGGAGCTGCGTGTTGTGGGAAC   540
CCCGCGGCAGTAGTTGAGCATCAGGCTCTTACCTTGGAGGTGGAGGGGTGAGAAGAATAG   600
AGGAAGAAGGGATAAGTCAGAGGAGGGCCTGAACAACTAGCCCCTCTATTGGCCTGCTTT   660
GGGTGAGCATTCAGTGAGTGTGTTTAAAAAAAAAAAGGGAGGGAAAACAAAAGACCTCAG   720
GAGCAGTTTTGTGTTGCTGTGTCTGGCTTCAAGAAGAAAATTCTAGACATTTATGCCGGC   780
AAGACCAAAGCTCAGCTAAGACTACTTCTCCCAAGAAGATAATTGTATCAGAGGATGGGT   840
TGGATCAGTACAGGTGGTTTGAGGAGACGCTGACAGAGGACCATGGAAAGGTGGGAGAGG   900
ACGCGCGGCTCCTGGGCTTCCTCTGAGCTCAGCTCCAGGCACCACAAGGCCACATAAGGA   960
```

Fig. 13

```
GGGTGAGGTCCCTGGAGTGGACTACATTTTCATAACCGTTGAGGAGTTT ATG GAA TTG GAG   1021
                                                  Met Glu Leu Glu      4
AAA AGT GGT GCT CTC CTA GAA AGC GGG ACC TAT GAA GAC AAC TAC TAC GGT 1072
Lys Ser Gly Ala Leu Leu Glu Ser Gly Thr Tyr Glu Asp Asn Tyr Tyr Gly   21
ACC CCG AAG CCT CCA GCT GAA CCA GCA CCA TTA TTA AAT GTA ACA GAC CAG 1123
Thr Pro lys Pro Pro Ala Glu Pro Ala Pro Leu Leu Asn Val Thr Asp Gln   38
ATA CTT CCG GGA GCT ACT CCA AGT GCT GAG GGG AAG CGG AAA AGA AAT AAG 1174
Ile Leu Pro Gly Ala Thr Pro Ser Ala Glu Gly Lys Arg Lys Arg Asn Lys   55
TCA GTG ACC AAC ATG GAG AAA GCA AGT ATA GAG CCT CCA GAG GAG GAA GAA 1225
Ser Val Thr Asn Met Glu Lys Ala Ser Ile Glu Pro Pro Glu Glu Glu Glu   72
GAA GAA AGG CCT GTA GTC AAT GGA AAC GGC GTG GTC ATA ACC CCA GAA TCC 1276
Glu Glu Arg Pro Val Val Asn Gly Asn Gly Val Val Ile Thr Pro Glu Ser   89
AGT GAA CAT GAA GAC AAA AGT GCA GGT GCC TCA GGG GAG ACA CCC TCC CAG 1327
Ser Glu His Glu Asp Lys Ser Ala Gly Ala Ser Gly Glu Thr Pro Ser Gln  106
CCT TAC CCT GCA CCC GTG TAC AGC CAG CCC GAA GAG CTC AAG GAC CAG ATG 1378
Pro Tyr Pro Ala Pro Val Tyr Ser Gln Pro Glu Glu Leu Lys Asp Gln Met  123
```

Fig. 14

GAC GAT ACA AAG CCA ACA AAG CCT GAG GAG AAC GAG GAC TCT GAT CCA TTG 1429
Asp Asp Thr Lys Pro Thr Lys Pro Glu Glu Asn Glu Asp Ser Asp Pro Leu 140
CCT GAT AAC TGG GAA ATG GCC TAC ACA GAG AAG GGG GAA GTC TAC TTC ATT 1480
Pro Asp Asn Trp Glu Met Ala Tyr Thr Glu Lys Gly Glu Val Tyr Phe Ile 157
GAC CAT AAC ACA AAG ACA ACA TCA TGG CTG GAT CCG CGA CTT GCG AAA AAG 1531
Asp His Asn Thr Lys Thr Thr Ser Trp Leu Asp Pro Arg Leu Ala Lys Lys 174
GCT AAA CCT CCA GAA GAG TGC AAA GAA AAT GAG CTT CCA TAT GGC TGG GAA 1582
Ala Lys Pro Pro Glu Glu Cys Lys Glu Asn Glu Leu Pro Tyr Gly Trp Glu 191
AAA ATC GAT GAT CCT ATA TAT GGC ACT TAC TAT GTT GAC CAC ATA AAT AGA 1633
Lys Ile Asp Asp Pro Ile Tyr Gly Thr Tyr Tyr Val Asp His Ile Asn Arg 208
AGA ACA CAG TTT GAA AAC CCT GTC CTG GAA GCA AAA AGG AAG CTA CAG CAA 1684
Arg Thr Gln Phe Glu Asn Pro Val Leu Glu Ala Lys arg Lys Leu Gln Gln 225
CAT AAC ATG CCC CAC ACA GAA CTT GGA GCA AAG CCC CTG CAG GCC CCA GGT 1735
His Asn Met Pro His Thr Glu Leu Gly Ala Lys Pro Leu Gln Ala Pro Gly 242
TTC CGA GAA AAG CCA CTC TTC ACC CGG GAT GCA TCC CAG TTG AAG GGA ACG 1786
Phe Arg Glu Lys Pro Leu Phe Thr Arg Asp Ala Ser Gln Leu Lys Gly Thr 259

Fig. 15

```
TTC CTC AGC ACC ACC CTC AAA AAG AGC AAC ATG GGC TTT GGG TTT ACC ATA 1837
Phe Leu Ser Thr Thr Leu Lys Lys Ser Asn Met Gly Phe Gly Phe Thr Ile  276
ATT GGT GGA GAC GAG CCG GAT GAG TTT CTA CAG GTG AAA AGT GTG ATC CCG 1888
Ile Gly Gly Asp Glu Pro Asp Glu Phe Leu Gln Val Lys Ser Val Ile Pro  293
GAT GGG CCT GCC GCA CAG GAT GGG AAA ATG GAG ACA GGT GAT GTC ATT GTC 1939
Asp Gly Pro Ala Ala Gln Asp Gly Lys Met Glu Thr Gly Asp Val Ile Val  310
TAT ATT AAT GAA GTT TGT GTC CTT GGA CAC ACT CAT GCA GAT GTT GTC AAA 1990
Tyr Ile Asn Glu Val Cys Val Leu Gly His Thr His Ala Asp Val Val Lys  327
CTT TTC CAG TCT GTT CCT ATT GGT CAG AGT GTC AAC TTG GTG TTG TGT CGT 2041
Leu Phe Gln Ser Val Pro Ile Gly Gln Ser Val Asn Leu Val Leu Cys Arg  344
GGC TAC CCT TTG CCC TTT GAC CCT GAA GAT CCT GCT AAC AGC ATG GTG CCA 2092
Gly Tyr Pro Leu Pro Phe Asp Pro Glu Asp Pro Ala Asn Ser Met Val Pro  361
CCC CTT GCA ATA ATG GAG AGG CCA CCT CCG GTG ATG GTC AAT GGA AGA CAT 2143
Pro Leu Ala Ile Met Glu Arg Pro Pro Pro Val Met Val Asn Gly Arg His  378
AAC TAT GAA ACA TAC TTG GAA TAC ATT TCT CGG ACC TCA CAG TCG GTC CCA 2194
Asn Tyr Glu Thr Tyr Leu Glu Tyr Ile Ser Arg Thr Ser Gln Ser Val Pro  395
```

Fig. 16

GAT ATT ACA GAC CGG CCA CCT CAT TCT TTG CAC TCC ATG CCA GCT GAC GGC 2245

Asp Ile Thr Asp Arg Pro Pro His Ser Leu his Ser Met Pro Ala Asp Gly 412

CAG CTA GAT GGC ACG TAT CCA CCA CCC GTC CAT GAC GAC AAT GTG TCT ATG 2296

Gln Leu Asp Gly Thr Tyr Pro Pro Pro Val His Asp Asp Asn Val Ser Met 429

GCT TCG TCT GGA GCC ACT CAA GCT GAA CTT ATG ACC TTA ACC ATT GTG AAA 2347

Ala Ser Ser Gly Ala Thr Gln Ala Glu Leu Met Thr Leu Thr Ile Val Lys 446

GGT GCC CAG GGA TTT GGC TTT ACT ATT GCC GAC AGT CCC ACG GGA CAG CGG 2398

Gly Ala Gln Gly Phe Gly Phe Thr Ile Ala Asp Ser Pro Thr Gly Gln Arg 463

GTG AAA CAA ATC CTT GAC ATT CAG GGA TGC CCT GGG CTG TGT GAA GGA GAC 2449

Val Lys Gln Ile Leu Asp Ile Gln Gly Cys Pro Gly Leu Cys Glu Gly Asp 480

CTC ATT GTT GAG ATC AAC CAA CAG AAT GTA CAG AAC CTG AGC CAT ACA GAA 2500

Leu Ile Val Glu Ile Asn Gln Gln Asn Val Gln Asn Leu Ser His Thr Glu 497

GTA GTG GAT ATA CTT AAG GAC TGC CCC GTT GGA AGT GAG ACT TCT TTA ATC 2551

Val Val Asp Ile Leu Lys Asp Cys Pro Val Gly Ser Glu Thr Ser Leu Ile 514

ATC CAT CGA GGA GGT TTC TTT TCT CCA TGG AAA ACT CCA AAG CCT ATG ATG 2602

Ile His Arg Gly Gly Phe Phe Ser Pro Trp Lys Thr Pro Lys Pro Met Met 531

Fig. 17

```
GAC CGA TGG GAG AAC CAA GGC AGT CCA CAA ACA AGT TTA TCT GCT CCG GCC 2653
Asp Arg Trp Glu Asn Gln Gly Ser Pro Gln Thr Ser Leu Ser Ala Pro Ala  548
GTC CCA CAG AAC CTG CCC TTC CCA CCT GCC CTT CAC AGG AGC TCC TTT CCT 2704
Val Pro Gln Asn Leu Pro Phe Pro Pro Ala Leu His Arg Ser Ser Phe Pro  565
GAT TCA ACA GAG GCC TTT GAC CCA CGG AAG CCT GAC CCA TAT GAG CTC TAC 2755
Asp Ser Thr Glu Ala Phe Asp Pro Arg Lys Pro Asp Pro Tyr Glu Leu Tyr  582
GAG AAA TCG AGA GCC ATT TAT GAA AGT AGG CAA CAA GTG CCA CCC AGG ACC 2806
Glu Lys Ser Arg Ala Ile Tyr Glu Ser Arg Gln Gln Val Pro Pro Arg Thr  599
AGT TTT CGA ATG GAT TCC TCT GGT CCA GAT TAT AAG GAA CTG GAT GTT CAC 2857
Ser Phe Arg Met Asp Ser Ser Gly Pro Asp Tyr Lys Glu Leu Asp Val His  616
CTT CGG AGG ATG GAG TCT GGA TTT GGC TTT AGA ATC CTT GGG GGA GAT GAA 2908
Leu Arg Arg Met Glu Ser Gly Phe Gly Phe Arg Ile Leu Gly Gly Asp Glu  633
CCT GGA CAG CCT ATT TTG ATC GGA GCC GTC ATT GCC ATG GGC TCA GCT GAC 2959
Pro Gly Gln Pro Ile Leu Ile Gly Ala Val Ile Ala Met Gly Ser Ala Asp  650
AGA GAC GGC CGT CTA CAC CCA GGA GAT GAG CTT GTC TAT GTC GAT GGG ATC 3010
Arg Asp Gly Arg Leu His Pro Gly Asp Glu Leu Val Tyr Val Asp Gly Ile  667
```

Fig. 18

```
CCA GTG GCT GGC AAG ACC CAC CGC TAT GTC ATC GAC CTC ATG CAC CAC GCG 3061
Pro Val Ala Gly Lys Thr His Arg Tyr Val Ile Asp Leu Met His His Ala  684
GCC CGC AAT GGG CAG GTT AAC CTC ACT GTG AGA AGA AAG GTG CTA TGT GGA 3112
Ala Arg Asn Gly Gln Val Asn Leu Thr Val Arg Arg Lys Val Leu Cys Gly  701
GGG GAG CCC TGC CCA GAG AAT GGG AGG AGT CCA GGC TCT GTA TCA ACT CAC 3163
Gly Glu Pro Cys Pro Glu Asn Gly Arg Ser Pro Gly Ser Val Ser Thr His  718
CAC AGC TCT CCG CGC AGT GAC TAT GCC ACC TAC TCC AAC AGC AAC CAC GCC 3214
His Ser Ser Pro Arg Ser Asp Tyr Ala Thr Tyr Ser Asn Ser Asn His Ala  735
GCC CCC AGC AGC AAT GCC TCA CCT CCT GAA GGC TTT GCC TCA CAC AGC TTG 3265
Ala Pro Ser Ser Asn Ala Ser Pro Pro Glu Gly Phe Ala Ser His Ser Leu  752
CAG ACC AGT GAT GTG GTC ATT CAC CGC AAA GAA AAC GAA GGG TTT GGC TTC 3316
Gln Thr Ser Asp Val Val Ile His Arg Lys Glu Asn Glu Gly Phe Gly Phe  769
GTC ATC ATC AGC TCT CTG AAC AGG CCT GAG TCT GGA GCC ACC ATA ACT GTG 3367
Val Ile Ile Ser Ser Leu Asn Arg Pro Glu Ser Gly Ala Thr Ile Thr Val  786
CCC CAT AAA ATT GGA CGA ATC ATT GAT GGG AGC CCT GCA GAT CGC TGT GCC 3418
Pro His Lys Ile Gly Arg Ile Ile Asp Gly Ser Pro Ala Asp Arg Cys Ala  803
```

Fig. 19

AAA CTC AAA GTG GGC GAC CGT ATC TTA GCA GTC AAC GGC CAG TCT ATC ATC 3469
Lys Leu Lys Val Gly Asp Arg Ile Leu Ala Val Asn Gly Gln Ser Ile Ile 820
AAC ATG CCT CAC GCT GAC ATT GTG AAG CTC ATC AAG GAC GCC GGT CTC AGT 3520
Asn Met Pro His Ala Asp Ile Val Lys Leu Ile Lys Asp Ala Gly Leu Ser 837
GTC ACC CTT CGC ATC ATT CCT CAG GAG GAG CTC AAC AGC CCA ACA TCA GCA 3571
Val Thr Leu Arg Ile Ile Pro Gln Glu Glu Leu Asn Ser Pro Thr Ser Ala 854
CCC AGT TCA GAG AAA CAG AGC CCC ATG GCC CAG CAG CAC AGC CCT CTG GCC 3622
Pro Ser Ser Glu Lys Gln Ser Pro Met Ala Gln Gln His Ser Pro Leu Ala 871
CAG CAG AGT CCT CTG GCC CAG CCA AGC CCC GCC ACC CCC AAC AGC CCA GTC 3673
Gln Gln Ser Pro Leu Ala Gln Pro Ser Pro Ala Thr Pro Asn Ser Pro Val 888
GCA CAG CCA GCT CCT CCC CAA CCT CTC CAG CTG CAA GGA CAC GAA AAT AGT 3724
Ala Gln Pro Ala Pro Pro Gln Pro Leu Gln Leu Gln Gly His Glu Asn Ser 905
TAC AGG TCA GAA GTT AAA GCG AGG CAA GAT GTG AAG CCA GAC ATC CGG CAG 3775
Tyr Arg Ser Glu Val Lys Ala Arg Gln Asp Val Lys Pro Asp Ile Arg Gln 922
CCT CCC TTC ACA GAC TAC AGG CAG CCC CCG CTG GAC TAC AGG CAG CCC CCG 3826
Pro Pro Phe Thr Asp Tyr Arg Gln Pro Pro Leu Asp Tyr Arg Gln Pro Pro 939

Fig. 20

```
GGA GGA GAC TAC TCA CAG CCC CCA CCC TTG GAC TAC AGG CAG CAC TCT CCA 3877
Gly Gly Asp Tyr Ser Gln Pro Pro Pro Leu Asp Tyr Arg Gln His Ser Pro  956
GAC ACC AGG CAG TAC CCT CTG TCA GAC TAC AGG CAG CCA CAG GAT TTT GAT 3928
Asp Tyr Arg Gln Tyr Pro Leu Ser Asp Tyr Arg Gln Pro Gln Asp Phe Asp  973
TAT TTC ACT GTG GAC ATG GAG AAA GGA GCC AAA GGA TTT GGA TTC AGC ATT 3979
Tyr Phe Thr Val Asp Met Glu Lys Gly Ala Lys Gly Phe Gly Phe Ser Ile  990
CGT GGA GGA AGG GAA TAC AAG ATG GAT CTG TAT GTG TTG AGA TTG GCA GAG 4030
Arg Gly Gly Arg Glu Tyr Lys Met Asp Leu Tyr Val Leu Arg Leu Ala Glu 1007
GAT GGG CCA GCC ATA AGG AAC GGC AGG ATG AGG GTA GGA GAT CAG ATC ATT 4081
Asp Gly Pro Ala Ile Arg Asn Gly Arg Met Arg Val Gly Asp Gln Ile Ile 1024
GAA ATA AAT GGG GAA AGC ACA CGA GAC ATG ACC CAC GCC AGA GCA ATA GAA 4132
Glu Ile Asn Gly Glu Ser Thr Arg Asp Met Thr His Ala Arg Ala Ile Glu 1041
CTC ATC AAG TCT GGA GGA AGA AGA GTG CGG CTG CTG CTG AAG AGA GGC ACG 4183
Leu Ile Lys Ser Gly Gly Arg Arg Val Arg Leu Leu Leu Lys Arg Gly Thr 1058
GGG CAG GTC CCG GAG TAT GGA ATG GTA CCT TCC AGC CTC TCC ATG TGC ATG 4234
Gly Gln Val Pro Glu Tyr Gly Met Val Pro Ser Ser Leu Ser Met Cys Met 1075
```

Fig. 21

```
AAA AGT GAC AAG CAT GGG TCC CCA TAT TTC TAC TTA CTG GGC CAC CCT AAA 4285
Lys Ser Asp Lys His Gly Ser Pro Tyr Phe Tyr Leu Leu Gly His Pro Lys 1092
GAC ACG ACG AAC CCC ACG CCT GGA GTG CTG CCG CTG CCG CCG CCC CAG GCC 4336
Asp Thr Thr Asn Pro Thr Pro Gly Val Leu Pro Leu Pro Pro Pro Gln Ala 1109
TGC CGG AAG TAGGCGTCTCCCTCGAAGACATCCTCTCTCCATTCTCTCCATCACATCCAGCCCC 4400
Cys Arg Lys                                                         1112
ACCCTCCGACCCTTCCCACCAGATAGGCCCAGACCCAACTTGGGATATCCAAAGGGAACA        4460
CGACGTTAGGAAACCAAAGGAGCTTTCGGCCGGCGGCCAGAAGAAGCAGCGCCTGGGGGA        4520
GCAGAGGGAGCGCTCGGCGAGCCCGCAGCGCAGTGCGCGGCCCAGGCTGGAGGAGGTGCC        4580
CGGCGGCCAGGGGCGGCCCGAGGCCGGCAGGCCCGCCTCGGAGGCGGCCGACGGGAAGGA        4640
GGCGCTGCGCGGCCGGCGTGAGGGCCTCGGGGCCGCGGGCGCGGGAGGCCGAGGCCAA          4700
GGTGGGTGTGCGCTCGGGGGCCCGACCCGCAGCGCGGCCCACGGGGGCGGCCCAGCGCG         4760
CAAGGCGACGATGGCGCCGGGGCCCTGGAAGGTGCCGGGCTCCGACAAGCTGCCGGGCGC        4820
CCTGCAGCCTGGCGCCTCGGCCGCGGGCAGATGAGCCCCAAGGCGAGGGGCCCCCGCCCG        4880
CCTCCACGCAGGCCGATCTTCCTGGGTTCCGTCTCACGGCGTTTTAATTTATTTCCACTG        4940
TCACACGCATAGATCTATACGAGGCGCCGAAGCCCGGGAGCGCCGGCGTGCGACGCGCGT        5000
```

Fig. 22

```
AGGCGGCACGGCACGGTGTGCGCGCAGGCAGACCTAAACTGATCCTAAAGCCCCCGGTTC    5060
CATGGTGGGAGCTTTGGCAGCTACGGAAGAACCAAAATCACGCAAACATCACAGAGAGAC    5120
AGTGCAGTGTAGCTTTAGATTCAAAAAAAAAAAAAA                            5156
```

Fig. 23

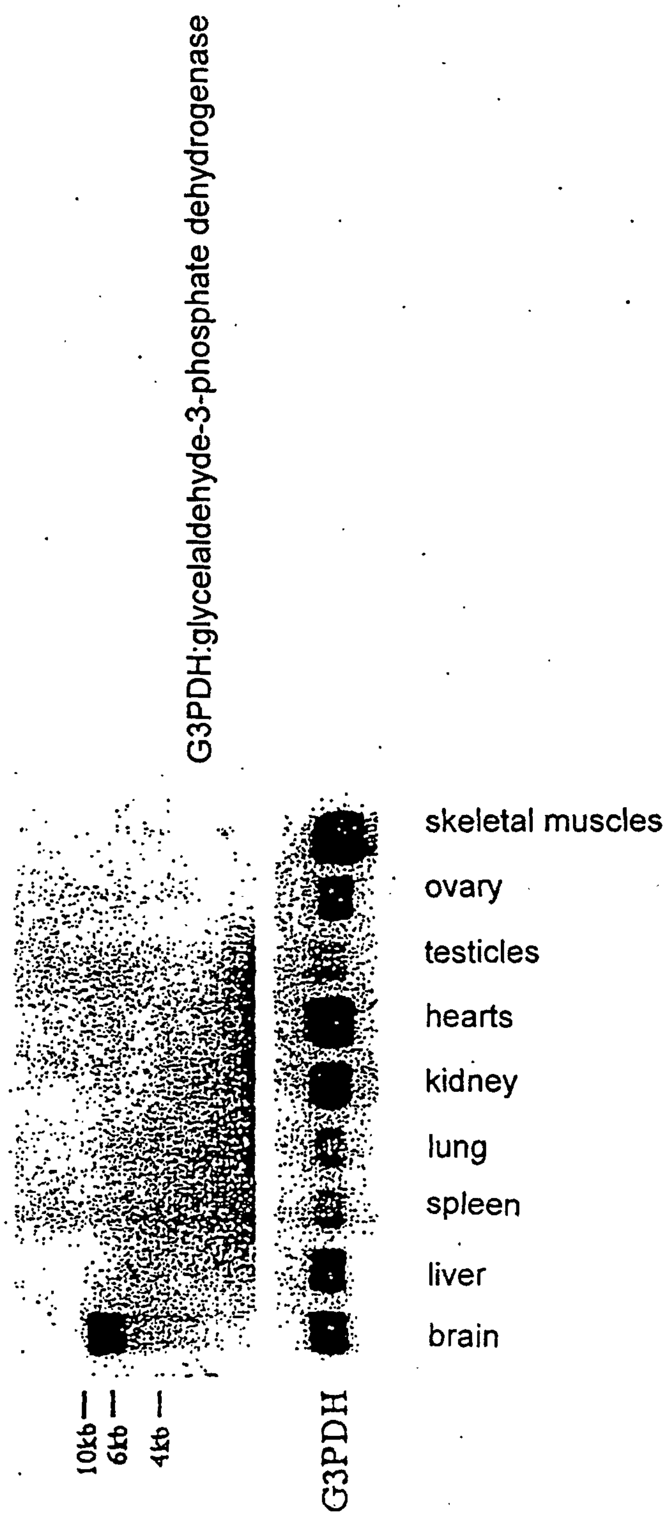
G3PDH:glycelaldehyde-3-phosphate dehydrogenase
skeletal muscles
ovary
testicles
hearts
kidney
lung
spleen
liver
brain
10kb
6kb
4kb
G3PDH

Fig. 24

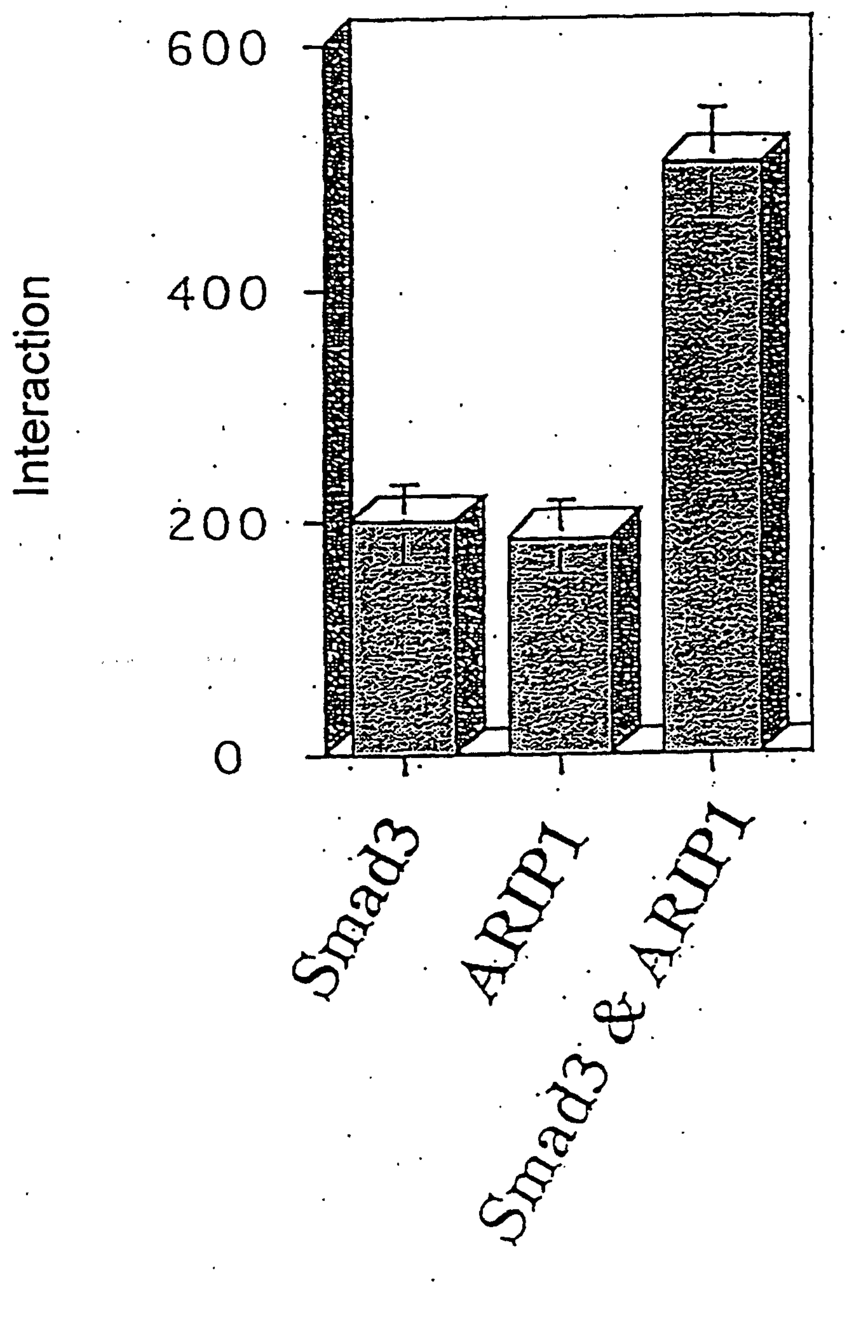

## INTERNATIONAL SEARCH REPORT

International application No.
PCT/JP99/06275

A. CLASSIFICATION OF SUBJECT MATTER
Int.Cl[7] C12N15/12, C07K14/47, C07K16/18, C12N1/21, A01K 67/027, A61K31/7088, A61K35/76, A61K38/02,A61K45/00, A61P25/16, A61P25/28, A61P25/08, A61P25/14, A61P43/00, A61P25/00, A61K48/00, A61K48/00, A61K39/395,

According to International Patent Classification (IPC) or to both national classification and IPC

B. FIELDS SEARCHED

Minimum documentation searched (classification system followed by classification symbols)
Int.Cl[7] C12N15/12, C07K14/47, C07K16/18, C12N1/21, A01K 67/027, A61K31/7088, A61K35/76, A61K38/02, A61K45/00, A61P25/16, A61P25/28, A61P25/08, A61P25/14, A61P43/00, A61P25/00, A61K48/00, A61K48/00, A61K39/395,

Documentation searched other than minimum documentation to the extent that such documents are included in the fields searched

Electronic data base consulted during the international search (name of data base and, where practicable, search terms used)
BIOSIS(DIALOG),WPI(DIALOG), GeneSeq/EMBL/DDBJ/Genbank

C. DOCUMENTS CONSIDERED TO BE RELEVANT

| Category* | Citation of document, with indication, where appropriate, of the relevant passages | Relevant to claim No. |
|---|---|---|
| X<br>A | Shoji H., et al."Identification of a novel type II activin receptor, type IIA-N, induced during the neural differentiation of murine P19 emboryonal carcinoma cells.", Biochemical Biophysical Research Communications (1998, May), Vol.246, No.2, p.320-324 | 17<br>1-16,18-28,30 |
| X<br>A | Sugino H.,et al."Activin: diversity of functions and signal transduction", Seikagaku(1996), Vol68, No.8, p.1405-1428 | 17<br>1-16,18-28,30 |
| X<br>A | Funaba M.,et al."Immunolocalization of type I or type II activin receptors in the rat brain", Journal of Neuroendocrinology(1997), Vol.9, No.2, p.105-111 | 26<br>1-25,27,28,30 |
| X<br>A | WO, 94/6456, A (Genentech INC),<br>31 March, 1994 (31.03.94),<br>Claim 23<br>& JP, 8-501314, A<br>Claim 23<br>& EP, 661993, A1 & US, 5654404, A<br>& US, 5703048, A | 26,27<br>1-25,28,30 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 14 January, 2000 (14.01.00) | 25 January, 2000 (25.01.00) |

| Name and mailing address of the ISA/ | Authorized officer |
|---|---|
| Japanese Patent Office | |
| Facsimile No. | Telephone No. |

Form PCT/ISA/210 (second sheet) (July 1992)

## INTERNATIONAL SEARCH REPORT

International application No.
PCT/JP99/06275

C (Continuation). DOCUMENTS CONSIDERED TO BE RELEVANT

| Category* | Citation of document, with indication, where appropriate, of the relevant passages | Relevant to claim No. |
|---|---|---|
| X<br>A | EP, 771873, A2 (Takeda Chem. Ind. Ltd.),<br>07 May, 1997 (07.05.97),<br>Claim 1, 20<br>& JP, 10-72497, A<br>Claim 1, 21 | 17,26<br>1-16,18-25,<br>27,28,30 |

Form PCT/ISA/210 (continuation of second sheet) (July 1992)

**INTERNATIONAL SEARCH REPORT**

International application No.
PCT/JP99/06275

**Box I Observations where certain claims were found unsearchable (Continuation of item 1 of first sheet)**

This international search report has not been established in respect of certain claims under Article 17(2)(a) for the following reasons:

1. ☒ Claims Nos.: 29
because they relate to subject matter not required to be searched by this Authority, namely:

The subject matter of claim 29 relates to a method for treatment of the human body by therapy, which does not require an international search report by this International Search Authority in accordance with PCT Article 17(2) (a)(i) and Rule 39.1(iv).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1992)